# EUROPEAN PATENT APPLICATION

(11) **EP 4 353 731 A1**
(43) Date of publication of application: **17.04.2024**
(21) Application number: 22820314.7
(22) Date of filing: 09.06.2022
(51) Int. Cl.: C07F 9/38, C12Q 1/06, C12Q 1/34, C12Q 1/42

(54) **METHOD FOR PRODUCING FLUORESCENT PROBE LIBRARY USING SOLID-PHASE EXTRACTION AND METHOD OF MEASURING ENZYME ACTIVITY USING SAME**

(30) Priority: 09.06.2021 JP 2021096839
(71) Applicant: The University of Tokyo, Bunkyo-ku, Tokyo 113-8654 (JP); RIKEN, Wako-shi, Saitama 351-0198 (JP)
(72) Inventor: KOMATSU Toru, Tokyo 113-8654 (JP); URANO Yasuteru, Tokyo 113-8654 (JP); SAKAMOTO Shingo, Tokyo 113-8654 (JP); WATANABE Rikiya, Wako-shi, Saitama 351-0198 (JP); KAGAMI Yu, Tokyo 113-8654 (JP)
(74) Representative: ABG Intellectual Property Law, S.L.
(86) International application number: PCT/JP2022/023319
(87) International publication number: WO 2022/260135

(57) **Abstract**

To provide a synthesis scheme capable of easily synthesizing various kinds of fluorescent probes. A method for preparing a compound represented by Formula (III) below, the method including steps (1) to (5).

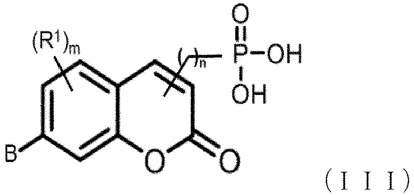

## Description

### TECHNICAL FIELD

The present invention relates to a method for synthesizing a novel fluorescent probe by which various kinds of fluorescent probes can be easily synthesized, a fluorescent mother nucleus compound that can be used in the synthesis method, a fluorescent probe and a fluorescent probe library for detection of enzyme activity which are obtained by the synthesis method, an enzyme activity measurement method using the same, and use of the fluorescent probe for biomarker detection.

### BACKGROUND ART

The research group of the present inventors has hitherto performed profiling based on detection of activity of an enzyme in blood at a single molecule level using a group of microdevice-compatible enzyme activity detection fluorescent probes, and found new relevance to a disease; however, it is considered that many enzyme activities not reported to be related to a disease still exist in body fluids such as blood and urine. Therefore, if it is possible to construct a fluorescent probe library capable of detecting various kinds of enzyme activities in an ultra-sensitive and exhaustive manner in a microdevice, it is considered to contribute to the search for enzyme activity as a new biomarker.

On the other hand, since a fluorescent probe used for enzyme activity detection is usually synthesized and purified over a period of several days to several weeks and then used for a fluorescence assay, it has been conventionally difficult to prepare a fluorescent probe group that detects activities of various enzymes present in blood or the like as described above in an exhaustive manner.

### SUMMARY OF THE INVENTION

An object of the present invention is to provide a synthesis scheme of a fluorescent probe utilizing advantages of liquid-phase synthesis and solid-phase synthesis. Another object of the present invention is to provide a synthesis scheme of a fluorescent probe that may enable solid-phase synthesis and easy desorption from a solid phase. Still another object of the present invention is to provide a synthesis scheme capable of easily synthesizing various kinds of fluorescent probes.

### Solution to Problem

The present inventors have conducted intensive studies to solve the above problems, and as a result, have found that it is possible to provide a synthesis scheme in which a highly pure target product can be obtained only by an operation of mixing and solution removal and, various kinds of fluorescent probes can also be easily synthesized by parallel processing, on the basis of a SAS (Synthesis-based on affinity separation) method which has a combination of advantages of a liquid-phase synthesis method, which has high reaction efficiency but has a complicated purification operation, and advantages of a solid-phase synthesis method, which has a simple purification operation but has low reaction efficiency.

Specifically, there is provided a synthesis scheme using a procedure in which a fluorescent probe is synthesized in a liquid phase using a fluorescent dye having phosphonic acid, phosphoric acid ester, or phosphoric amide in a molecular skeleton as a mother nucleus, and then solid-phase extraction is performed using a carrier to which phos-tag that specifically captures a phosphate group is bonded.

In this scheme, since a fluorescent probe to be finally obtained has phosphonic acid, it is also possible to impart high water solubility required for an assay using a microdevice, and this scheme is considered to be optimal for the purpose of the present invention.

According to the present invention, the following inventions are provided.
[1] A compound represented by General Formula (I) below: 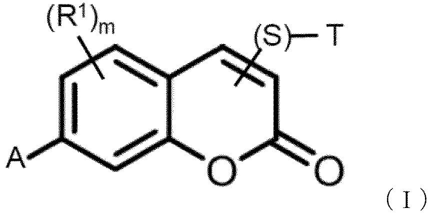 wherein
   A is an amino group (-NR²H) or a hydroxyl group (-OH),
   where R² is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO);
   R¹, when present, is the same or different monovalent substituent present on a benzene ring;
   S, when present, is a linker;
   T is selected from a phosphonic acid group (-P(=O)(OH)₂), a phosphoric acid ester group (-O-P(=O)(OH)₂), or a phosphoric amide group (-NH-P(=O)(OH)₂); and
   m is an integer of 0 to 3.
[2] The compound according to [1], wherein A is -NR²H.
[3] The compound according to [1], wherein A is -OH.
[4] A method for preparing a compound represented by Formula (III) below: 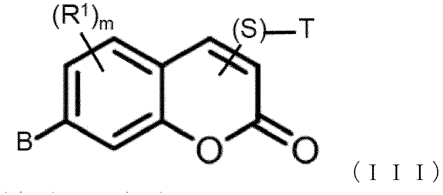 wherein
   B is selected from an amide group (-NR²C(=O)R, R is a hydrogen atom or an alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), an ester group, a phosphoric acid ester group, a sulfuric acid ester group, an ether group, or -O-L' (L' represents a saccharide or a partial structure of a saccharide); and
   S, T, R¹, and m are as defined in Formula (I),
   the method comprising the steps of:
      (1) protecting a T group of a compound represented by Formula (I) below: 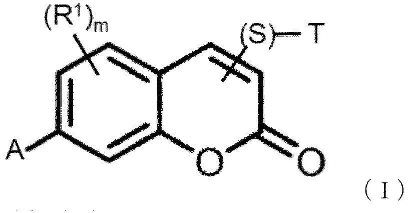 wherein,
         A is an amino group (-NR²H) or a hydroxyl group (-OH),
         where, R² is a hydrogen atom or a substituted or unsubstituted alkyl group having 1 to 8 carbon atoms;
         R¹, when present, is the same or different monovalent substituent present on a benzene ring;
         S, when present, is a linker;
         T is selected from a phosphonic acid group (-P(=O)(OH)₂), a phosphoric acid ester group (-O-P(=O)(OH)₂), or a phosphoric amide group (-NH-P(=O)(OH)₂); and
         m is an integer of 0 to 3;
      (2) (i) when A is an amino group (-NR²H), converting the amino group of a product obtained in the step (1) into an amide group (-NR²C(=O)R, R is a hydrogen atom or an alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or
         (ii) when A is a hydroxyl group, converting the hydroxyl group of a product obtained in the step (1) into an ester group, a phosphoric acid ester group, a sulfuric acid ester group, an ether group, or -O-L' (L' represents a saccharide or a partial structure of a saccharide);
      (3) removing a protective group of T of a product obtained in the step (2), which may optionally include a crude purification step after the removal of the protective group;
      (4) adding a compound represented by Formula (II) below to a product obtained in the step (3): 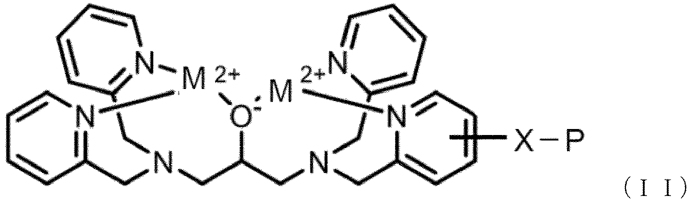 wherein,
         M is Zn or Cu;
         X is a linker group; and
         P is a carrier; and
      (5) purifying a product obtained in the step (4) and then liquating or eluting a compound of Formula (III).
[5] A method for preparing one kind of compound represented by Formula (III) below for each vessel of a plurality of reaction vessels by performing the following steps (1) to (5) in parallel in the plurality of reaction vessels:
   (1) protecting a T group of a compound represented by Formula (I) below: 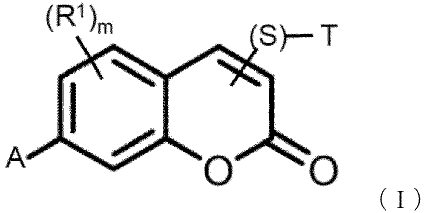 wherein,
      A is an amino group (-NR²H) or a hydroxyl group (-OH),
      where, R² is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO);
      R¹, when present, is the same or different monovalent substituent present on a benzene ring;
      S, when present, is a linker;
      T is selected from a phosphonic acid group (-P(=O)(OH)₂), a phosphoric acid ester group (-O-P(=O)(OH)₂), or a phosphoric amide group (-NH-P(=O)(OH)₂); and
      m is an integer of 0 to 3;
   (2) (i) when A is an amino group (-NR²H), converting the amino group of a product obtained in the step (1) into an amide group (-NR²C(=O)R, R is a hydrogen atom or an alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or
      (ii) when A is a hydroxyl group, converting the hydroxyl group of a product obtained in the step (1) into an ester group, a phosphoric acid ester group, a sulfuric acid ester group, an ether group, or -O-L' (L' represents a saccharide or a partial structure of a saccharide);
   (3) removing a protective group of T of a product obtained in the step (2), which may optionally include a crude purification step after the removal of the protective group;
   (4) adding a compound represented by Formula (II) below to a product obtained in the step (3): 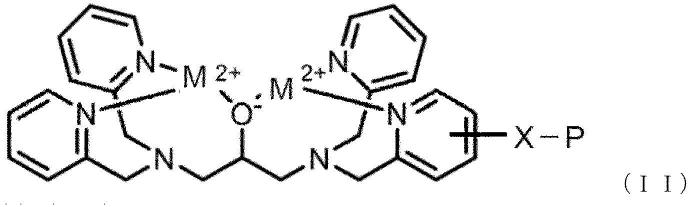 wherein,
      M is Zn or Cu;
      X is a linker group; and
      P is a carrier; and
   (5) purifying a product obtained in the step (4) and then liquating or eluting a compound of Formula (III): 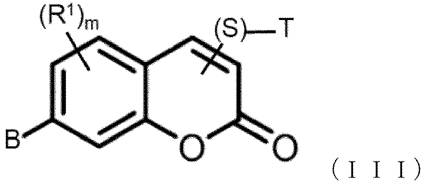 wherein,
      B is selected from an amide group (-NR²C(=O)R, R is a hydrogen atom or an alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), an ester group, a phosphoric acid ester group, a sulfuric acid ester group, an ether group, or -O-L' (L' represents a saccharide or a partial structure of a saccharide); and
      S, T, R¹, and m are as defined in Formula (I).
[6] A compound represented by General Formula (III) below or a salt thereof: 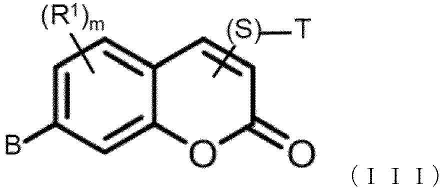 wherein,
   B is selected from an amide group (-NR²C(=O)R, R is a hydrogen atom or an alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), an ester group, a phosphoric acid ester group, a sulfuric acid ester group, an ether group, or -O-L' (L' represents a saccharide or a partial structure of a saccharide);
   R¹, when present, is the same or different monovalent substituent present on a benzene ring;
   S, when present, is a linker;
   T is selected from a phosphonic acid group (-P(=O)(OH)₂), a phosphoric acid ester group (-O-P(=O)(OH)₂), or a phosphoric amide group (-NH-P(=O)(OH)₂); and
   m is an integer of 0 to 3.
[7] A fluorescent probe for detecting enzyme activity, comprising the compound represented by General Formula (III) or the salt thereof according to [6].
[8] A method for detecting activity of a plurality of enzymes in a biological sample, the method comprising bringing the biological sample into contact with the compound of General Formula (III) according to [6].
[9] The method according to [8], wherein a library of the compound of General Formula (III) according to [6] is used.
[10] The method according to [8] or [9], wherein a microdevice is used.
[11] A method for testing an enzyme assay of a composition containing the compound of General Formula (III) according to [6], the method comprising bringing the composition into contact with a biological sample containing or suspected of containing an enzyme which cleaves the compound.
[12] A method for screening a fluorescent probe capable of detecting a biomarker of a specific disease, the method comprising the steps of:
   (1) adding a library of the compound of Formula (III) according to [6] to a microdevice so that at least one well of the microdevice includes one kind of compound of Formula (III);
   (2) adding a solution containing a biological sample to the microdevice so that at least one well containing one molecule of enzyme is generated in the microdevice, the biological sample being a biological sample obtained from a patient with a specific disease or a biological sample obtained from a healthy subject;
   (3) bringing the compound of Formula (III) into contact with an enzyme to detect fluorescence in a well of the microdevice, the step including
      bringing the compound of Formula (III) into contact with the biological sample obtained from a patient with a specific disease to measure a fluorescence intensity (first fluorescence intensity) from the compound of Formula (III), and
      bringing the compound of Formula (III) into contact with the biological sample obtained from a healthy subject to measure a fluorescence intensity (second fluorescence intensity) from the compound of Formula (III); and
   (4) determining that the compound is a biomarker activity detection fluorescent probe by showing that the compound is a candidate for the fluorescent probe when there is a difference between the first fluorescence intensity and the second fluorescence intensity.
[13] A method for detecting activity of ENPP in a biological sample, the method comprising bringing a compound of Formula (IV) or a salt thereof into contact with a biological sample in an aqueous solution, an increase in fluorescence intensity in the aqueous solution indicating the presence of activity of ENPP: 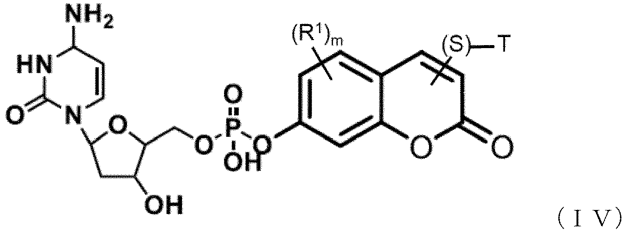 wherein R¹, S, T, and m are as described in detail in General Formula (I).
[14] The method according to [13], wherein the biological sample is a biological sample of a patient with pancreatic cancer, a patient suspected of having pancreatic cancer, or a healthy subject.
[15] A method for diagnosing pancreatic cancer or a method for predicting a possibility that a subject from which a biological sample is derived has pancreatic cancer, the method comprising the steps of: (a) applying a fluorescent probe containing a compound of Formula (IV) or a salt thereof to a clinical specimen of a test subject; and (b) measuring a fluorescent image of the clinical specimen to which the fluorescent probe is applied.
[16] A method for diagnosing pancreatic cancer or a method for predicting a possibility that a subject from which a biological sample is derived has pancreatic cancer, the method comprising the steps of: (a) bringing a biological sample obtained from a subject into contact with a fluorescent probe containing a compound of Formula (IV) or a salt thereof; and (b) measuring a fluorescence intensity of the biological sample brought into contact with the fluorescent probe.
[17] The diagnostic method or prediction method according to [15] or [16], wherein the compound of Formula (VI) is a compound below: 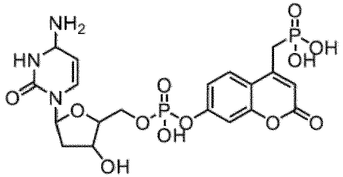
[18] A fluorescent probe for detection of pancreatic cancer for use in the method according to [15] or [16], comprising a compound of Formula (IV) or a salt thereof.
[19] A kit for detection of pancreatic cancer cells or tissues, comprising a compound of Formula (IV) or a salt thereof.
[20] The fluorescent probe according to [18], wherein the compound of Formula (VI) is a compound below: 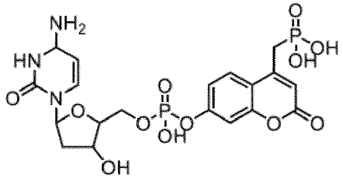
[21] A method for diagnosing pancreatic cancer or a method for predicting a possibility that a subject from which a biological sample is derived has pancreatic cancer, the method comprising detecting, by a microdevice, single-molecule enzyme activity of CD13 in a biological sample obtained from a subject, using a fluorescent probe containing the compound or the salt thereof according to [6], in which B is -NR²-CO-L, and a moiety of -CO-L is an alanine, lysine, arginine, or methionine residue in General Formula (III).
[22] A method for diagnosing pancreatic cancer or a method for predicting a possibility that a subject from which a biological sample is derived has pancreatic cancer, the method comprising detecting, by a microdevice, single-molecule enzyme activity of DPP4 in a biological sample obtained from a subject, using a fluorescent probe containing the compound or the salt thereof according to [6], in which B is -NR²-CO-L, and a moiety of -CO-L is -Pro-Xaa (Pro represents a proline residue and Xaa represents an amino acid residue such as glycine, serine, or glutamic acid) peptide in General Formula (III).
[23] A method for diagnosing pancreatic cancer or a method for predicting a possibility that a subject from which a biological sample is derived has pancreatic cancer, the method comprising:
   detecting, by a microdevice, single-molecule enzyme activity of CD13 in a biological sample obtained from a subject, using a fluorescent probe containing the compound or the salt thereof according to [6], in which B is -NR²-CO-L, and a moiety of -CO-L is an alanine, lysine, arginine, or methionine residue in General Formula (III); and
   detecting, by a microdevice, single-molecule enzyme activity of DPP4 in a biological sample obtained from a subject, using a fluorescent probe containing the compound or the salt thereof according to [6], in which B is -NR²-CO-L, and a moiety of -CO-L is -Pro-Xaa (Pro represents a proline residue and Xaa represents an amino acid residue such as glycine, serine, or glutamic acid) peptide in General Formula (III).
[24] The method according to any one of [21] to [23], wherein the biological sample is a biological sample of a patient with pancreatic cancer, a patient suspected of having pancreatic cancer, or a healthy subject.

### Effects of the Invention

According to the present invention, it is possible to provide a synthesis scheme of a fluorescent probe utilizing advantages of liquid-phase synthesis and solid-phase synthesis. According to the present invention, it is possible to provide a synthesis of a fluorescent probe that may enable solid-phase synthesis and easy desorption from a solid phase. According to the present invention, it is possible to provide a synthesis scheme capable of easily synthesizing various kinds of fluorescent probes. Specifically, in the synthesis scheme based on the present invention, it is possible to perform probe synthesis and purification by a very simple scheme of only mixing and solution removal, and parallel processing is also possible, so that about ten kinds of probes can also be prepared in a few days, and thus the efficiency of probe development can be greatly improved.

A library of the compound (fluorescent probe) of the present invention can be constructed by performing the reactions according to the synthesis scheme based on the present invention in parallel in a plurality of reaction vessels.

It is possible to provide a method for detecting activity of a plurality of enzymes in a biological sample using the compound of the present invention or a library thereof.

Since the library of the compound of the present invention includes several hundred kinds of compounds capable of detecting a plurality of enzymes, it is possible to search (screen) biomarker candidate activities from a biological sample using the library in an ultra-sensitive and exhaustive manner, and it is also possible to screen a biomarker activity detection fluorescent probe using the library.

A biomarker activity detection fluorescent probe obtained by the screening method of the present invention can be used for detection of a biomarker specific to a predetermined disease to diagnose the disease.

### BRIEF DESCRIPTION OF THE DRAWINGS

Fig. 1 is a schematic view of comparing a preparation method of the present invention with a conventional liquid-phase synthesis method and a conventional solid-phase synthesis method.
Fig. 2 shows an example in which a fluorescent probe of the present invention is synthesized.
Fig. 3 is a schematic view of a method for preparing a compound group of the invention.
Fig. 4 shows measurement results of HPLC in synthesis of an amidase probe (Synthesis Example 3).
Fig. 5 is a perspective view schematically illustrating a microdevice according to an embodiment of the present invention.
Fig. 6 is a conceptual diagram of disease diagnosis using the present invention.
Fig. 7A shows a fluorescence microscopic image in which enzyme activity in blood for each probe was detected using a microdevice in Example 1.
Fig. 7B shows a fluorescence microscopic image in which enzyme activity in blood for each probe was detected using a microdevice in Example 1.
Fig. 8 shows a fluorescence microscopic image obtained using Arg-PMAC in Example 2.
Fig. 9 is an ROC curve obtained in a test performed using Arg-PMAC in Example 2.
Fig. 10 is an ROC curve obtained in a test performed using Met-PMAC in Example 2.
Fig. 11 shows a representative fluorescence microscopic image obtained in Example 3.
Fig. 12 is an ROC curve obtained in Example 3.
Fig. 13 shows results of activity measurement of PMUM-dCMP using a purified enzyme of ENPP3 on a plate reader.
Fig. 14 shows a fluorescence microscopic image obtained in Example 6.
Fig. 15 shows results of comparing the numbers of wells having a fluorescence intensity, which is detected in Example 6, of 2500 AU or more between a healthy subject and a patient with pancreatic cancer.
Fig. 16 shows an ROC curve created by making a determination based on results of ENPP activity measurement in Example 6.

### DETAILED DESCRIPTION OF THE INVENTION

In the present specification, "halogen atom" means a fluorine atom, a chlorine atom, a bromine atom, or an iodine atom.

In the present specification, "alkyl" may be any aliphatic hydrocarbon group that is linear, branched, cyclic, or in a combination thereof. The number of carbon atoms in the alkyl group is not particularly limited, but is, for example, 1 to 6 (C₁₋₆), 1 to 10 (C₁₋₁₀), 1 to 15 (C₁₋₁₅), or 1 to 20 (C₁₋₂₀). When the number of carbon atoms is specified, it means an "alkyl" having the number of carbon atoms within that numerical range. For example, C₁₋₈ alkyls include methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl, and the like. In the present specification, an alkyl group may have one or more optional substituents. Examples of such substituents include, but are not limited to, an alkoxy group, a halogen atom, an amino group, a mono- or di-substituted amino group, a substituted silyl group, and acyl. When an alkyl group has two or more substituents, they may be the same as or different from each other. The same is also true for the alkyl moiety of other substituents (for example, an alkoxy group, an arylalkyl group, or the like) including an alkyl moiety.

In the present specification, when certain functional groups are defined as "optionally substituted", the type of substituent, substitution position, and the number of substituents are not particularly limited, and when there are two or more substituents, they may be the same as or different from each other. Examples of the substituents include, but are not limited to, an alkyl group, an alkoxy group, a hydroxyl group, a carboxyl group, a halogen atom, a sulfo group, an amino group, an alkoxycarbonyl group, and an oxo group. Other substituents may be present in these substituents. Examples of such cases include, but are not limited to, an alkyl halide group and a dialkylamino group.

In the present specification, "alkoxy group" is a structure in which the above alkyl group is bonded to an oxygen atom, and examples thereof include saturated alkoxy group that is linear, branched, cyclic, or in a combination thereof. Preferred examples thereof include a methoxy group, an ethoxy group, a n-propoxy group, an isopropoxy group, a cyclopropoxy group, a n-butoxy group, an isobutoxy group, a s-butoxy group, a t-butoxy group, a cyclobutoxy group, a cyclopropylmethoxy group, a n-pentyloxy group, a cyclopentyloxy group, a cyclopropylethyloxy group, a cyclobutylmethyloxy group, a n-hexyloxy group, a cyclohexyloxy group, a cyclopropylpropyloxy group, a cyclobutylethyloxy group, and a cyclopentylmethyloxy group.

### 1. Fluorescent Dye Mother Nucleus

One implementation of the present invention is a compound represented by General Formula (I) below (hereinafter, also referred to as "Compound 1 of the present invention").

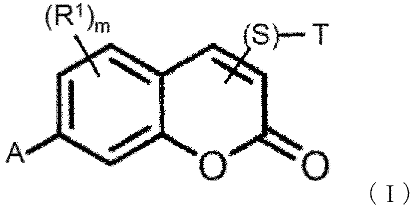

Compound 1 of the present invention plays a role as a mother nucleus molecule in a method for preparing a novel fluorescent probe of the present invention described below.

Since Compound 1 of the present invention has phosphonic acid, a phosphoric acid ester group, or phosphoric amide group, a fluorescent probe to be finally obtained using the compound also has a phosphonic acid, phosphoric acid ester, or phosphoric amide, so that it becomes possible to impart high water solubility required for an assay using a microdevice.

In Formula (I), A is an amino group (-NR²H) or a hydroxyl group (-OH).

R² is selected from the group consisting of a hydrogen atom and a branched, linear or cyclic, substituted or unsubstituted alkyl group having 1 to 8 carbon atoms. The alkyl group having 1 to 8 carbon atoms includes methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl, and the like.

One or more non-adjacent and non-terminal C atoms of the alkyl group may be replaced with O, S, CO, or COO. Therefore, the alkyl group defined as R² includes, for example, a case where a part of the alkyl group contains a polyethylene glycol chain and a case where the entire R² is a polyethylene glycol chain.

Examples of the substituent that the alkyl group of R² can have include, but are not limited to, an alkoxy group, a hydroxyl group, a carboxyl group, a halogen atom, a sulfo group, an amino group, an alkoxycarbonyl group, and an oxo group.

In one preferred aspect of Compound 1 of the present invention, R² is a hydrogen atom.

In Formula (I), R¹, when present, is the same or different monovalent substituent present on a benzene ring. Examples of the monovalent substituent of R¹ include halogen, a substituted or unsubstituted alkyl group having 1 to 8 carbon atoms, an alkoxy group, a carboxyl group, a sulfonyl group, and an aryl group.

One or more non-adjacent and non-terminal C atoms of the alkyl group of R¹ may be replaced with O, S, CO, or COO. Therefore, the alkyl group of R¹ includes, for example, a case where a part of the alkyl group contains a polyethylene glycol chain and a case where the entire R¹ is a polyethylene glycol chain.

m is an integer of 0 to 3, preferably 0 to 1.

The monovalent substituent of R¹ can be introduced at any position on the benzene ring of the coumarin skeleton.

In Formula (I), T is selected from a phosphonic acid group (-P(=O)(OH)₂), a phosphoric acid ester group (-O-P(=O)(OH)₂), or a phosphoric amide group (-NH-P(=O)(OH)₂). Preferably, T is a phosphonic acid group (-P(=O)(OH)₂).

In Formula (I), S, when present, is a linker which bonds a phosphonic acid group, a phosphoric acid ester group, or a phosphoric amide group of T to coumarin.

Examples of the linker include, but are not limited to, a substituted or unsubstituted hydrocarbon group, polyethylene glycol, a heterocyclic group, an amide group (including any group represented by -NHCO- or -CONH-), and an aromatic ring having an amide group. The linker that can be used for Compound 1 of the present invention also includes a linker in which two or more kinds of the groups listed above are bonded.

Examples of the hydrocarbon group include an alkylene group having 1 to 10 carbon atoms, an alkynylene group having 1 to 10 carbon atoms, a cycloalkylene group, and an aromatic hydrocarbon.

Polyethylene glycol is represented by -(C₂H₄-O)ₜ- (t is an integer of 1 to 10), and either an ethylene group or an oxo group may be a side bonded to coumarin.

Examples of the heterocyclic ring include a triazole group.

The aromatic ring having an amide group can be represented by *-NHCO-Ar- or *-Ar-CONH- (Ar represents an aromatic ring, and * represents a side bonded to coumarin).

A substituted or unsubstituted hydrocarbon group, preferably an alkylene group having 1 to 10 carbon atoms may be present at one or both ends of polyethylene glycol, a heterocyclic group, an amide group, or an aromatic ring having an amide group.

In one preferred aspect of the present invention, the phosphonic acid group, the phosphoric acid ester group, or the phosphoric amide group is directly bonded to coumarin.

In another preferred aspect of the present invention, the phosphonic acid group, the phosphoric acid ester group, or the phosphoric amide group is bonded to coumarin via an alkylene group having 1 to 10 carbon atoms (for example, a methylene group or an ethylene group), an amide group, or a triazole group.

The phosphonic acid group, the phosphoric acid ester group, or the phosphoric amide group can be introduced at any position of the 3-position or 4-position of the coumarin skeleton.

One preferred mode of the present invention is a compound having the following structure, in which A is -NR²H in Formula (I).

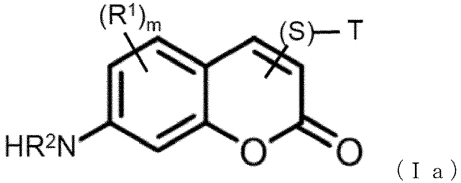

In Formula (Ia), S, T, R¹, R², and m are as defined in Formula (I).

A preferred mode of the present invention is a compound in which A is -NH₂ in Formula (I).

One preferred aspect of the compound represented by Formula (Ia) is a compound having the following structure.

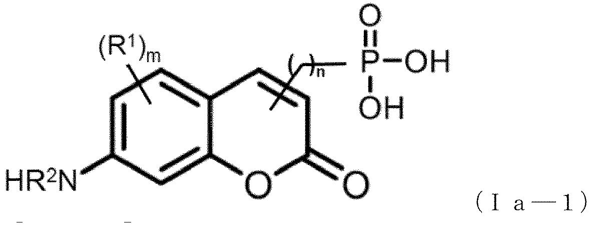

In Formula (Ia-1), R¹, R², and m are as defined in Formula (I), and n is an integer of 0 to 10.

One preferred example of the compound represented by Formula (Ia-1) is the following compound. 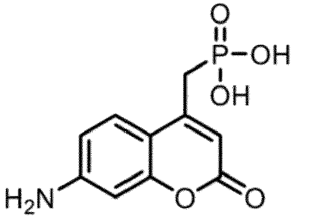 4-Phosphonomethyl-7-aminocoumarin (PMAC)

Another preferred mode of the present invention is a compound having the following structure, in which A is -OH in Formula (I).

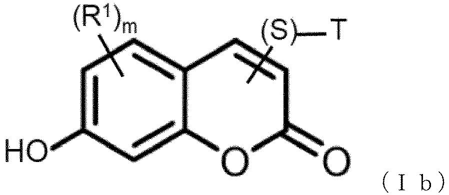

In Formula (Ib), S, T, R¹, and m are as defined in Formula (I).

One preferred aspect of the compound represented by Formula (Ib) is a compound having the following structure.

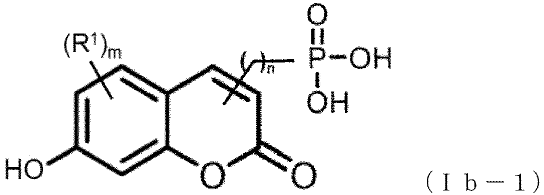

In Formula (Ib-1), R¹ and m are as defined in Formula (I), and n is an integer of 0 to 10.

One preferred example of the compound represented by Formula (Ib-1) is the following compound. 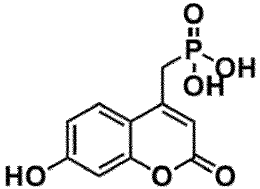 4-Phosphonomethyl-7-hydroxycoumarin

Methods for producing a representative compound of Compound 1 of the present invention were specifically shown in Examples of the present specification. Therefore, in view of those explanations, those skilled in the art can suitably select reaction raw materials, reaction conditions, reaction reagents, and the like and prepare Compound 1 of the present invention by optionally modifying or altering those methods.

### 2. Method for Preparing Fluorescent Probe

### (1) Method for Preparing Compound Represented by General Formula (III)

Another implementation of the present invention is a method for preparing a compound represented by Formula (III) below, the method including the following steps (1) to (5) (hereinafter, also referred to as the "preparation method of the present invention"): 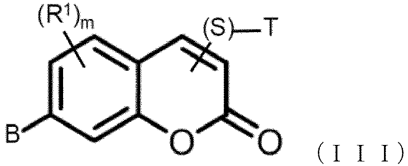
(1) protecting a T group of a compound represented by Formula (I) below: 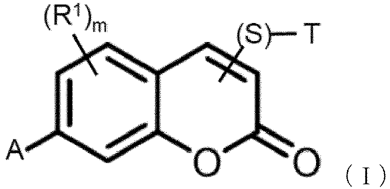 wherein
   A is an amino group (-NR²H) or a hydroxyl group (-OH),
   where R² is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO);
   R¹, when present, is the same or different monovalent substituent present on a benzene ring;
   S, when present, is a linker;
   T is selected from a phosphonic acid group (-P(=O)(OH)₂), a phosphoric acid ester group (-O-P(=O)(OH)₂), or a phosphoric amide group (-NH-P(=O)(OH)₂); and
   m is an integer of 0 to 3.
(2) (i) when A is an amino group (-NR²H), converting the amino group of a product obtained in the step (1) into an amide group (-NR²C(=O)R, R is a hydrogen atom or an alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or
   (ii) when A is a hydroxyl group, converting the hydroxyl group of a product obtained in the step (1) into an ester group, a phosphoric acid ester group, a sulfuric acid ester group, an ether group, or -O-L' (L' represents a saccharide or a partial structure of a saccharide);
(3) removing a protective group of T of a product obtained in the step (2), which may optionally include a crude purification step after the removal of the protective group;
(4) adding a compound represented by Formula (II) below to a product obtained in the step (3): 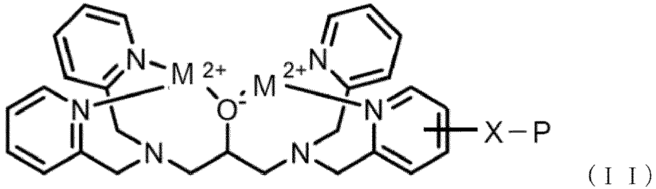 wherein,
   M is Zn or Cu;
   X is a linker group; and
   P is a carrier; and
(5) purifying a product obtained in the step (4) and then liquating or eluting a compound of Formula (III).

The preparation method of the present invention can adopt a procedure in which a fluorescent probe is synthesized in a liquid phase using a fluorescent dye having phosphonic acid, phosphoric acid ester, or phosphoric amide in a molecular skeleton as a mother nucleus, and then solid-phase extraction is performed using a carrier to which phos-tag that specifically captures a phosphate group is bonded.

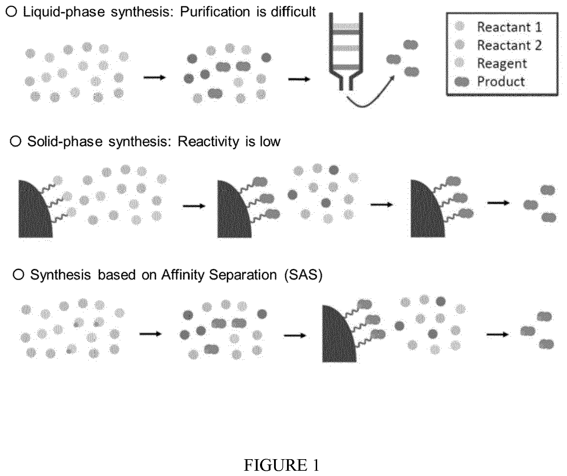

A schematic view of comparing a preparation method of the present invention with a conventional liquid-phase synthesis method and a conventional solid-phase synthesis method is shown in Fig. 1.

A liquid-phase synthesis method has high reaction efficiency, but is complicated because column chromatography or preparative HPLC is used for purification operation, and purification requires several hours/compound. A solid-phase synthesis method has a problem in that the purification operation is simple but the reaction efficiency is low. The preparation method of the present invention is based on the SAS (Synthesis-based on affinity separation) method having a combination of both advantages, and it is possible to obtain a target product (preferably with high purity) only by the operation of mixing and solution removal. In the preparation method of the present invention, it is also possible to easily synthesize various kinds of fluorescent probes by parallel processing.

Each step of the preparation method of the present invention will be described in detail below.

### Step (1)

In the step (1), an operation of protecting a T group of the following Compound 1 of the present invention described above is performed. 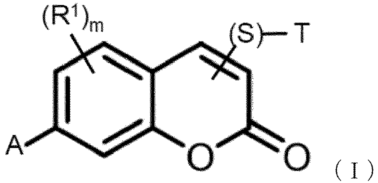 (A, S, T, R¹, and m are as defined above.)

The phosphonic acid group, the phosphoric acid ester group, or the phosphoric amide group, which is the T group, can be protected by a method usually used for protecting these protective groups, and for example, can be suitably protected by a silyl-based protective group (for example, a tert-butyldiphenylsilyl group) or a tert-butyl group. As a reagent for protecting the phosphonic acid group, the phosphoric acid ester group, or the phosphoric amide group, TBDPS-Cl (tert-butyl(chloro)diphenylsilane), TBDMS-Cl (tert-butyldimethylsilyl chloride), isobutene, tert-butanol, and the like are suitably used. For example, phosphonic acid, phosphoric acid ester, and phosphoric amide can be protected by dissolving Compound 1 of the present invention, for example, in a solution containing imidazole (such as a DMSO solution) and adding TBDPS-Cl.

### Step (2)

In the step (2), the portion A of a product obtained in the step (1) is converted into a functional group that is cleaved by contact with a substance to be measured such as an enzyme.

The functional group that is cleaved by contact with a substance to be measured is appropriately determined depending on the type of biomolecule to be targeted in the measurement of enzyme activity or the like performed using the compound (III) as a final product, and depending on whether A is an amino group or a hydroxyl group, the step (2) is divided into the following (i) and (ii).

(i) When A is an amino group (-NR²H), the amino group is converted into an amide group (-NR²C(=O)R, R is a hydrogen atom or a substituted or unsubstituted alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)).

The partial structure of an amino acid means to form an amino acid, an amino acid residue, a peptide, or a part of an amino acid together with C=O to which the partial structure is bonded.

In the present specification, as for the "amino acid", any compound can be used as long as it has both an amino group and a carboxyl group, and the amino acid includes natural and non-natural compounds. The amino acid may be any of a neutral amino acid, a basic amino acid, and an acidic amino acid, it is possible to use amino acids which themselves function as a transmitter such as a neurotransmitter, and amino acids which are constituents of polypeptide compounds such as physiologically active peptides (including dipeptides, tripeptides, tetrapeptides and oligopeptides) or proteins, and the amino acid may be, for example, an α amino acid, a β amino acid, a γ amino acid, or the like. It is preferable to use an optically active amino acid as the amino acid. For example, as the α amino acid, either a D- or a L-amino acid may be used, and it may be preferable to select an optically active amino acid that functions in a living body.

The N terminal of the amino acid may be subjected to N-acylation (for example, N-acetylation or the like) or N-carbamoylation.

In the present specification, the "amino acid residue" refers to a structure corresponding to the remaining partial structure specified by removing a hydroxyl group from a carboxyl group of an amino acid.

The amino acid residue includes an α-amino acid residue, a β-amino acid residue, or a γ-amino acid residue.

In the present specification, the "peptide" refers to a structure in which two or more amino acids are linked by a peptide bond.

Examples of a case where L forms a part of an amino acid together with C=O to which L is bonded include a structure in which a carboxyl group of a side chain of an amino acid is bonded to -NR²H to form a carbonyl group, thereby forming a part of an amino acid, such as a γ-glutamyl group.

In one mode of the present invention, the partial structure of the amino acid of L is represented by Formula (3) below. 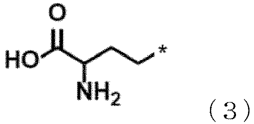
* represents a site that is bonded to C=O.

In one preferred mode of the present invention, -NR²-CO-L is represented by the following formula.

-NH-CO-CHR₃-NHR₀ (1)

In Formula (1), the moiety of -CO-CHR₃-NHR₀ constitutes an amino acid residue.

In Formula (1), R₃ represents a group constituting a side chain of a natural amino acid (glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, or proline), such as hydrogen or a methyl group. R₃ can also form a ring with a nitrogen atom of NHR₀.

R₃ also includes a group constituting a side chain of a non-natural amino acid (such as citrulline or norvaline). R₃ also includes a group in which a part of a group constituting a side chain of a natural amino acid or a non-natural amino acid is substituted or modified with another substituent. R₃ also includes a group other than a group constituting a side chain of a natural amino acid or a non-natural amino acid, for example, an alkyl group having various substituents.

In Formula (1), R₀ represents an N-terminal protective group (such as an acetyl group (-COCH₃), succinamide (-CO-C₂H₄-COOH), or Cbz (benzyloxycarbonyl group)) or a saturated or unsaturated alkyl group having 1 to 20 carbon atoms.

Examples of the amino acid residue represented by -CO-CHR₃-NHR₀ of Formula (1) include, but are not limited to, residues of amino acid selected from glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, proline, pyroglutamic acid, Lys(Cbz) (a group in which an amino group of a side chain of lysine is substituted with a benzyloxycarbonyl group), citrulline, Met(O2) (a group in which sulfur (S) of a side chain of methionine is substituted with sulfur dioxide (SO₂)), sarcosine, 2-aminobutanoic acid, thioproline, azetidinecarbonyl, Tyr(4-NO₂) (a group in which a hydroxyl group of a side chain of tyrosine is substituted with a nitro group), Ac-Met (a group in which the N terminal of methionine is protected with an acetyl group), Cbz-Ala (a group in which the N terminal of alanine is protected with Cbz (benzyloxycarbonyl group)), and the like.

The amino acid residue represented by -CO-CHR₃-NHR₀ of Formula (1) may be either L-form or D-form.

In one preferred mode of the present invention, -NR²-CO-L is represented by the following formula.

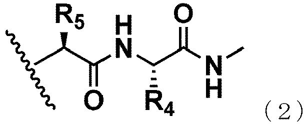

In Formula (2), R₄ represents a group constituting a side chain of a natural amino acid (glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, or proline), such as hydrogen or a methyl group. R₄ can also form a ring with a nitrogen atom of NH adjacent to the carbon to which R₄ is bonded. R₄ also includes a group constituting a side chain of a non-natural amino acid (such as citrulline or norvaline). R₄ also includes a group in which a part of a group constituting a side chain of a natural amino acid or a non-natural amino acid is substituted or modified with another substituent. R₄ also includes a group other than a group constituting a side chain of a natural amino acid or a non-natural amino acid, for example, an alkyl group having various substituents.

In Formula (2), R₅ represents a group constituting a side chain of a natural amino acid (glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, or proline), such as hydrogen or a methyl group. R₅ can also form a ring with a nitrogen atom of an amino group or the like adjacent to the carbon to which R₅ is bonded. R₅ also includes a group constituting a side chain of a non-natural amino acid (such as citrulline or norvaline). R₅ also includes a group in which a part of a group constituting a side chain of a natural amino acid or a non-natural amino acid is substituted or modified with another substituent. R₅ also includes a group other than a group constituting a side chain of a natural amino acid or a non-natural amino acid, for example, an alkyl group having various substituents.

In Formula (2), 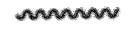 represents an amino group, an amino group subjected to N-acetylation or the like, a structure in which an amino group and an amino acid are bonded to each other(N terminal may be subjected to acetylation or the like), or a structure in which an amino group and a plurality of amino acids are bonded to each other by a peptide bond (N terminal may be subjected to acetylation).

The moiety of Formula (2a) below in Formula (2) constitutes a peptide.

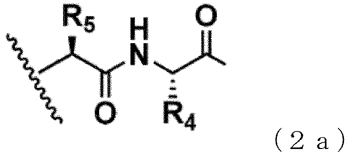

Examples of the peptide represented by Formula (2a) include, but are not limited to, Glu-Pro-, Suc-Ala-Ala-Pro-Abu-, Gly-Pro-, Lys-Ala, Phe-Met-, Cbz-Arg-Arg-, D-Ala-Leu-Lys-, Ac-Leu-Leu-Arg-, Lys-His-Leu-Tyr-, Phe-Thr-Thr-Tyr-, Suc-Leu-Leu-Val-Tyr-, Ac-Asp-Glu-Val-Asp-, Ac-Ile-Glu-Thr-Asp-, Ac-Ala-Ala-Pro-Val-, MeOSuc-Ala-Ala-Pro-Val-, Cbz-Gly-Val-Val-, Cbz-Gly-Pro-, Cbz-Ser-Lys-Leu-Gln-, and Ac-Leu-Arg-Gly-Gly-.

When the amino group (-NR²H) is converted into an amide group, the conversion is performed, for example, by condensing an organic acid with a dehydration-condensation agent (such as COMU or HATU), and preparing an acid anhydride or an acid chloride and reacting the acid anhydride or the acid chloride.

When the amino group (-NR²H) is converted into the group represented by Formula (1), the conversion is performed in such a manner that an amide bond is formed, for example, by condensing an appropriately protected amino acid with a dehydration-condensation agent (such as COMU or HATU), and preparing an acid anhydride or an acid chloride and reacting the acid anhydride or the acid chloride, and the protective group of the amino acid side chain is deprotected.

When the amino group (-NR²H) is converted into the group represented by Formula (2), the conversion is performed by condensing a side-chain protecting peptide separately prepared with a dehydration-condensation agent (such as COMU or HATU) and deprotecting a side-chain protective group.

When the amino group (-NR²H) is converted into a phosphoric amide group, the conversion is performed by a method using a reagent such as phosphoramidite.

When the amino group (-NR²H) is converted into a sulfonamide group, the conversion is performed by a reaction with sulfonic acid chloride.

(ii) When A is a hydroxyl group, the hydroxyl group is converted into an ester group, a phosphoric acid ester group, a sulfuric acid ester group, an ether group, or -O-L' (L' represents a saccharide or a partial structure of a saccharide).

The ester group is represented by -COOR³, and R³ is selected from a substituted or unsubstituted alkyl group having 1 to 10 carbon atoms or -(C₂H₄)ₛ-CH₃ (s is an integer of 1 to 10).

The phosphoric acid ester group includes phosphoric acid monoester and phosphoric acid diester.

In the case of phosphoric acid diester (-O-P(=O)(OH)(OR'), R' is represented by, for example, W-U-, W is an organic base, and U is a partial structure of a sugar or a derivative thereof, or a single bond.

The sugar or the derivative thereof of U is ribose, deoxyribose, or derivatives thereof. The ribose, deoxyribose, or derivatives thereof are bonded to the organic base at the 1'-position and to phosphate at the 5'-position.

The organic base of W is preferably selected from the group consisting of a nucleobase and a derivative thereof; a partial structure of choline ((CH₃)₃N⁺-C₂H₄-), a diethylamino group ((CH₃CH₂)2N-), and amino group represented by an amino group (R"₂N-) or ammonium group. R" is a hydrogen atom or an alkyl group having 1 to 10 carbon atoms, and may be the same or different.

The nucleobase is selected from the group consisting of adenine, thymine, cytosine, guanine, and uracil.

The phosphoric acid monoester or phosphoric acid diester may be bonded to a benzene ring via a linker (Y), and in this case, a site having phosphoric acid monoester is represented by the formula -Y-O-P(=O)(OH)₂, and a site having phosphoric acid diester is represented by the formula -Y-O-P(=O)(OH)(OR').

Y is -O-(CH₂)ₙ₁-, -O-(CH₂)ₙ₂-Ar₁-, -NH-(CH₂)ₙ₃-, or -NH-(CH₂)ₙ₄-Ar₂-.

In the linker, a bond in either the right or left direction may be bonded to the benzene ring, but O or NH is preferably bonded to the benzene ring.

n1, n2, n3, and n4 are each independently an integer of 1 to 10.

Ar₁ and Ar₂ are each independently a substituted or unsubstituted arylene group.

The unsubstituted arylene group in Ar₁ and Ar₂ is preferably one having 6 to 14 carbon atoms, and specific examples thereof include a phenylene group and a naphthylene group. Among them, the unsubstituted arylene group in Ar₁ and Ar₂ is preferably a phenylene group.

Examples of the substituent of the arylene group include a halogen atom and an alkyl group having 1 to 10 carbon atoms.

The halogen atom is preferably a chlorine atom, a bromine atom, or an iodine atom.

The alkyl group having 1 to 10 carbon atoms is preferably a linear alkyl group and more preferably a methyl group or an ethyl group.

The sulfuric acid ester is represented by -O-S(=O)₂(OH).

The ether group is represented by -OR⁴, and R⁴ is a substituted or unsubstituted alkyl group having 1 to 8 carbon atoms.

The partial structure of the saccharide of L' in -O-L' constitutes a saccharide or a part of a saccharide, together with O to which L' is bonded.

Examples of the saccharide include D-glucose, D-galactose, L-galactose, D-glucopyranose, D-xylose, D-mannose, D-fucose, L-fucose, D-arabinose, L-arabinose, D-N-acetylglucosamine, D-N-acetylgalactosamine, and sialic acid, and D-glucopyranose is preferable.

When the hydroxyl group is converted into an ester group, the conversion is performed, for example, by condensing an organic acid with a dehydration-condensation agent (such as COMU or HATU), and preparing an acid anhydride or an acid chloride and reacting the acid anhydride or the acid chloride.

When the hydroxyl group is converted into a phosphate group, the conversion is performed by reacting the product obtained in the step (1) using a phosphate chloride such as phosphoryl chloride and an analog thereof.

When the hydroxyl group is converted into a sulfate group, the conversion is performed by a methodology such as use of a base complex.

When the hydroxyl group is converted into an ether group, the conversion is performed by reaction with a brominated product, an iodinated product or the like which gives an intended alkylation under basic conditions.

When the hydroxyl group is converted into -O-L' (L' represents a saccharide or a partial structure of a saccharide), the conversion is performed by reaction with a brominated product, an iodinated product or the like which gives an intended glycosidic bond under basic conditions.

### Step (3)

In the step (3), a protective group of the T group of a product obtained in the step (2) is removed.

For removal of the protective group, for example, the reaction solution is subjected to acidic conditions such as trifluoroacetic acid, or deprotection is performed using a reagent that gives a fluorine ion such as TBAF.

The step (3) may include a step of crude purification after removal of the protective group of the T group.

Examples of the step of crude purification include, but are not limited to, ether precipitation and elution by HPLC.

### Step (4)

In the step (4), a compound represented by the following Formula (II) below is added to a product obtained in the step (3).

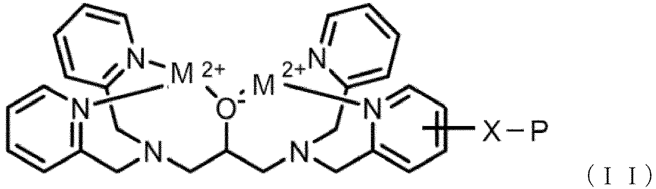

The step (4) is characterized in that solid-phase extraction is performed using a carrier to which phos-tag that specifically captures a phosphate group is bonded (the compound represented by Formula (II)). That is, as shown in Fig. 1, various reactants, reagents, and products are contained in the liquid phase at the stage where the step (3) is completed, but when the compound represented by Formula (II) is added, the compound having a phosphonic acid group can be specifically captured.

In Formula (II), M is Zn or Cu and is preferably Zn.

In Formula (II), X is a linker group.

Examples of the linker group include a C1-C6 alkylene group, an amino group (-NH-), an ether group (-O-), a thioether group (-S-), a carbonyl group (-C(=O)-), a thionyl group (-C(=S)-), an ester group, an amide group, a urea group (-NHC(=O)NH-), a thiourea group (-NHC(=S)NH-), and polyethylene glycol; a C1-C6 alkylene group having, at one end, a group selected from the group consisting of an amino group, an ether group, a thioether group, a carbonyl group, a thionyl group, an ester group, an amide group, a urea group, a thiourea group, and polyethylene glycol; a C1-C6 alkylene group having, at both ends, the same or different groups selected from the group consisting of an amino group, an ether group, a thioether group, a carbonyl group, a thionyl group, an ester group, an amide group, a urea group, and a thiourea group; and a group in which two or more groups selected from the group consisting of these groups are linearly bonded.

The linker group is preferably a C1-C6 alkylene group having an amide group at one end, polyethylene glycol, and a group in which these structures are connected by an amide bond or a triazole structure.

In Formula (II), P is a carrier.

As the carrier of P, any carrier can be used as long as it is a carrier used in solid-phase synthesis and solid-phase extraction, and for example, agarose gel, a resin (such as polystyrene beads), magnetic beads, and metal nanoparticles can be suitably used.

### Step (5)

In the step (5), a product obtained in the step (4) is purified and then the compound of Formula (III) is liquated or eluted.

Purification of the product is performed by washing the product with a wash buffer solution (for example, a solution of 50% H₂O and 50% MeCN, containing Bis Tris-AcOH and NaCl) and then washing the product with water.

Liquating or eluting the compound of Formula (III) can be performed by adjusting pH, and/or adding phosphoric acid or a phosphonic acid-containing compound (phosphonic acid as a representative example) to elute the compound.

The pH can be adjusted using an appropriate acid or base, and for example, the pH can be adjusted by adding an NH₃ aqueous solution, an ethylenediamine aqueous solution, or the like to elute the target compound.

### (2) Compound Represented by General Formula (III)

A compound represented by General Formula (III) below is obtained by the preparation method of the present invention.

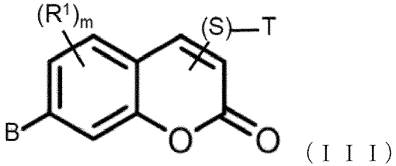

Still another implementation of the present invention is a compound represented by General Formula (III) (hereinafter, also referred to as the "compound of the present invention"). The compound represented by Formula (III) is also referred to as the "fluorescent probe of the present invention". The compound of the present invention can be used as a fluorescent probe for detection of one or more kinds of enzyme activity depending on the kind of the functional group introduced as B.

In Formula (III), S, T, R¹, and m are as defined in Formula (I).

In Formula (III), B is selected from an amide group (-NR²C(=O)R, R is a hydrogen atom or a substituted or unsubstituted alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently a hydrogen atom or an alkyl group having 1 to 8 carbon atoms), a sulfonamide group (-NR²-SO₂-R_{c}, R_{c} is a hydrogen atom or an alkyl group having 1 to 8 carbon atoms), an ester group, a phosphoric acid ester group, a sulfuric acid ester group, an ether group, or -O-L' (L' represents a saccharide or a partial structure of a saccharide).

When B is an amide group (-NR²C(=O)R, R is a hydrogen atom or a substituted or unsubstituted alkyl group having 1 to 8 carbon atoms), the compound represented by Formula (III) can be used as a fluorescent probe for activity detection of an amidase.

Examples of the substituent include a carboxyl group.

Examples of the substituted or unsubstituted alkyl group having 1 to 8 carbon atoms of R include, but are not limited to, a hexyl group and an ethyl group having a carboxyl group.

The partial structure of an amino acid of L in -NR²-CO-L means to form an amino acid, an amino acid residue, a peptide, or a part of an amino acid together with C=O to which the partial structure is bonded.

The amino acid, the amino acid residue, and the peptide are as described in detail in step (2).

Examples of a case where L forms a part of an amino acid together with C=O to which L is bonded include a structure in which a carboxyl group of a side chain of an amino acid is bonded to -NR²H to form a carbonyl group, thereby forming a part of an amino acid, such as the γ-glutamyl group described above.

In one mode of the present invention, the partial structure of the amino acid of L is represented by Formula (3) below. 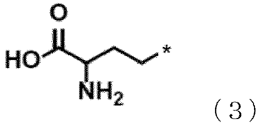
* represents a site that is bonded to C=O.

In one preferred mode of the present invention, -NR²-CO-L is represented by the following formula.

-NH-CO-CHR₃-NHR₀ (1)

In Formula (1), the moiety of -CO-CHR₃-NHR₀ constitutes an amino acid residue.

In Formula (1), R₃ represents a group constituting a side chain of a natural amino acid (glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, or proline), such as hydrogen or a methyl group. R₃ can also form a ring with a nitrogen atom of -NHR₀.

R₃ also includes a group constituting a side chain of a non-natural amino acid (such as citrulline or norvaline). R₃ also includes a group in which a part of a group constituting a side chain of a natural amino acid or a non-natural amino acid is substituted or modified with another substituent. R₃ also includes a group other than a group constituting a side chain of a natural amino acid or a non-natural amino acid, for example, an alkyl group having various substituents.

In Formula (1), R₀ represents an N-terminal protective group (such as an acetyl group (-COCH₃), succinamide (-CO-C₂H₄-COOH), or Cbz (benzyloxycarbonyl group)) or a saturated or unsaturated alkyl group having 1 to 20 carbon atoms.

Examples of the amino acid residue represented by -CO-CHR₃-NHR₀ of Formula (1) include, but are not limited to, residues of amino acid selected from glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, proline, pyroglutamic acid, Lys(Cbz) (a group in which an amino group of a side chain of lysine is substituted with a benzyloxycarbonyl group), citrulline, Met(O2) (a group in which sulfur (S) of a side chain of methionine is substituted with sulfur dioxide (SO₂)), sarcosine, 2-aminobutanoic acid, thioproline, azetidinecarbonyl, Tyr(4-NO₂) (a group in which a hydroxyl group of a side chain of tyrosine is substituted with a nitro group), Ac-Met (a group in which the N terminal of methionine is protected with an acetyl group), Cbz-Ala (a group in which the N terminal of alanine is protected with Cbz (benzyloxycarbonyl group)), and the like.

The amino acid residue represented by -CO-CH₂R₃-NHR₀ of Formula (1) may be either L-form or D-form.

When B is a substituent represented by Formula (1), the compound represented by Formula (III) can be used as a fluorescent probe for activity detection of an aminopeptidase or a protease.

In one preferred mode of the present invention, -NR²-CO-L is represented by the following formula.

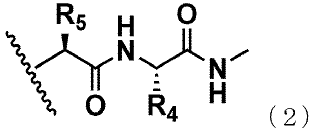

In Formula (2), R₄ represents a group constituting a side chain of a natural amino acid (glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, or proline), such as hydrogen or a methyl group. R₄ can also form a ring with a nitrogen atom of NH adjacent to the carbon to which R₄ is bonded. R₄ also includes a group constituting a side chain of a non-natural amino acid (such as citrulline or norvaline). R₄ also includes a group in which a part of a group constituting a side chain of a natural amino acid or a non-natural amino acid is substituted or modified with another substituent. R₄ also includes a group other than a group constituting a side chain of a natural amino acid or a non-natural amino acid, for example, an alkyl group having various substituents.

In Formula (2), R₅ represents a group constituting a side chain of a natural amino acid (glycine, alanine, leucine, isoleucine, valine, lysine, cysteine, threonine, arginine, asparagine, aspartic acid, glutamine, glutamic acid, serine, histidine, phenylalanine, methionine, tryptophan, tyrosine, or proline), such as hydrogen or a methyl group. R₅ can also form a ring with a nitrogen atom of an amino group or the like adjacent to the carbon to which R₅ is bonded. R₅ also includes a group constituting a side chain of a non-natural amino acid (such as citrulline or norvaline). R₅ also includes a group in which a part of a group constituting a side chain of a natural amino acid or a non-natural amino acid is substituted or modified with another substituent. R₅ also includes a group other than a group constituting a side chain of a natural amino acid or a non-natural amino acid, for example, an alkyl group having various substituents.

In Formula (2), 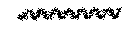 represents an amino group, an amino group subjected to N-acetylation or the like, a structure in which an amino group and an amino acid are bonded to each other (N terminal may be subjected to acetylation or the like), or a structure in which an amino group and a plurality of amino acids are bonded to each other by a peptide bond (N terminal may be subjected to acetylation).

The moiety of Formula (2a) below in Formula (2) constitutes a peptide.

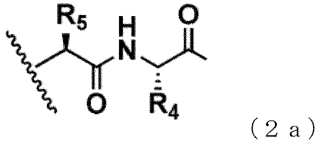

Examples of the peptide represented by Formula (2a) include, but are not limited to, Glu-Pro-, Suc-Ala-Ala-Pro-Abu-, Gly-Pro-, Lys-Ala, Phe-Met-, Cbz-Arg-Arg-, D-Ala-Leu-Lys-, Ac-Leu-Leu-Arg-, Lys-His-Leu-Tyr-, Phe-Thr-Thr-Tyr-, Suc-Leu-Leu-Val-Tyr-, Ac-Asp-Glu-Val-Asp-, Ac-Ile-Glu-Thr-Asp-, Ac-Ala-Ala-Pro-Val-, MeOSuc-Ala-Ala-Pro-Val-, Cbz-Gly-Val-Val-, Cbz-Gly-Pro-, Cbz-Ser-Lys-Leu-Gln-, and Ac-Leu-Arg-Gly-Gly-.

When B is a substituent represented by Formula (2), the compound represented by Formula (III) can be used as a fluorescent probe for activity detection of a peptidase or a protease.

When B is a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), the compound represented by Formula (III) can be used as a fluorescent probe for activity detection of a serine hydrolase.

When B is a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), the compound represented by Formula (III) can be used as a fluorescent probe for activity detection of a sulfur metabolism-related enzyme such as glutathione S-transferase.

The ester group is represented by -COOR³, and R³ is selected from a substituted or unsubstituted alkyl group having 1 to 10 carbon atoms or -(C₂H₄)ₛ-CH₃ (s is an integer of 1 to 10).

When B is an ester group in which R³ is a substituted or unsubstituted alkyl group having 1 to 10 carbon atoms, the compound represented by Formula (III) can be used as a fluorescent probe for activity detection of an esterase.

When B is an ester group in which R³ is -(C₂H₄)ₛ-CH₃ (s is an integer of 1 to 10), the compound represented by Formula (III) can be used as a fluorescent probe for activity detection of a lipase.

The phosphoric acid ester group includes phosphoric acid monoester (-OP(=O)(OH)₂) and phosphoric acid diester (-O-P(=O)(OH)(OR')).

When B is phosphoric acid monoester, the compound represented by Formula (III) can be used as a fluorescent probe for activity detection of a phosphatase.

When B is phosphoric acid diester (-O-P(=O)(OH)(OR'), R' is represented by, for example, W-U-, W is an organic base, and U is a partial structure of a sugar or a derivative thereof, or a single bond.

The sugar or the derivative thereof of U is ribose, deoxyribose, or derivatives thereof. The ribose, deoxyribose, or derivatives thereof are bonded to the organic base at the 1'-position and to phosphate at the 5'-position.

The organic base of W is preferably selected from the group consisting of a nucleobase and a derivative thereof; a partial structure of choline ((CH₃)₃N⁺-C₂H₄-), a diethylamino group ((CH₃CH₂)₂N-), and amino group represented by an amino group (R"₂N-) or ammonium group. R" is a hydrogen atom or an alkyl group having 1 to 10 carbon atoms, and may be the same or different.

The nucleobase is selected from the group consisting of adenine, thymine, cytosine, guanine, and uracil.

The phosphoric acid monoester or phosphoric acid diester may be bonded to a benzene ring via a linker (Y), and in this case, a site having phosphoric acid monoester is represented by the formula -Y-O-P(=O)(OH)₂, and a site having phosphoric acid diester is represented by the formula -Y-O-P(=O)(OH)(OR').

Y is -O-(CH₂)ₙ₁-, -O-(CH₂)ₙ₂-Ar₁-, -NH-(CH₂)ₙ₃-, or -NH-(CH₂)ₙ₄-Ar₂-.

In the linker, a bond in either the right or left direction may be bonded to the benzene ring, but O or NH is preferably bonded to the benzene ring.

n1, n2, n3, and n4 are each independently an integer of 1 to 10.

Ar₁ and Ar₂ are each independently a substituted or unsubstituted arylene group.

The unsubstituted arylene group in Ar₁ and Ar₂ is preferably one having 6 to 14 carbon atoms, and specific examples thereof include a phenylene group and a naphthylene group. Among them, the unsubstituted arylene group in Ar₁ and Ar₂ is preferably a phenylene group.

Examples of the substituent of the arylene group include a halogen atom and an alkyl group having 1 to 10 carbon atoms.

When B is phosphoric acid diester, the compound represented by Formula (III) can be used as a fluorescent probe for activity detection of ENPPs.

The sulfuric acid ester is represented by -O-S(=O)₂(OH).

When B is sulfuric acid ester, the compound represented by Formula (III) can be used as a fluorescent probe for activity detection of a sulfatase.

The ether group is represented by -OR⁴, and R⁴ is a substituted or unsubstituted alkyl group having 1 to 10 carbon atoms.

When B is an ether group, the compound represented by Formula (III) can be used as a fluorescent probe for activity detection of an oxidoreductase such as Cytochrome P450.

The partial structure of the saccharide of L' in -O-L' forms a saccharide or a part of a saccharide, together with O to which L' is bonded.

Examples of the saccharide include D-glucose, D-galactose, L-galactose, D-glucopyranose, D-xylose, D-mannose, D-fucose, L-fucose, D-arabinose, L-arabinose, D-N-acetylglucosamine, D-N-acetylgalactosamine, and sialic acid, and D-glucopyranose is preferable.

When B constitutes a part of β-D-glucopyranose, the compound represented by Formula (III) can be used as a fluorescent probe for activity detection of a β-glycosidase.

Non-limiting examples of the fluorescent probe of the present invention are shown below.

### (1) Fluorescent probe for detection of peptidase, amidase, and protease

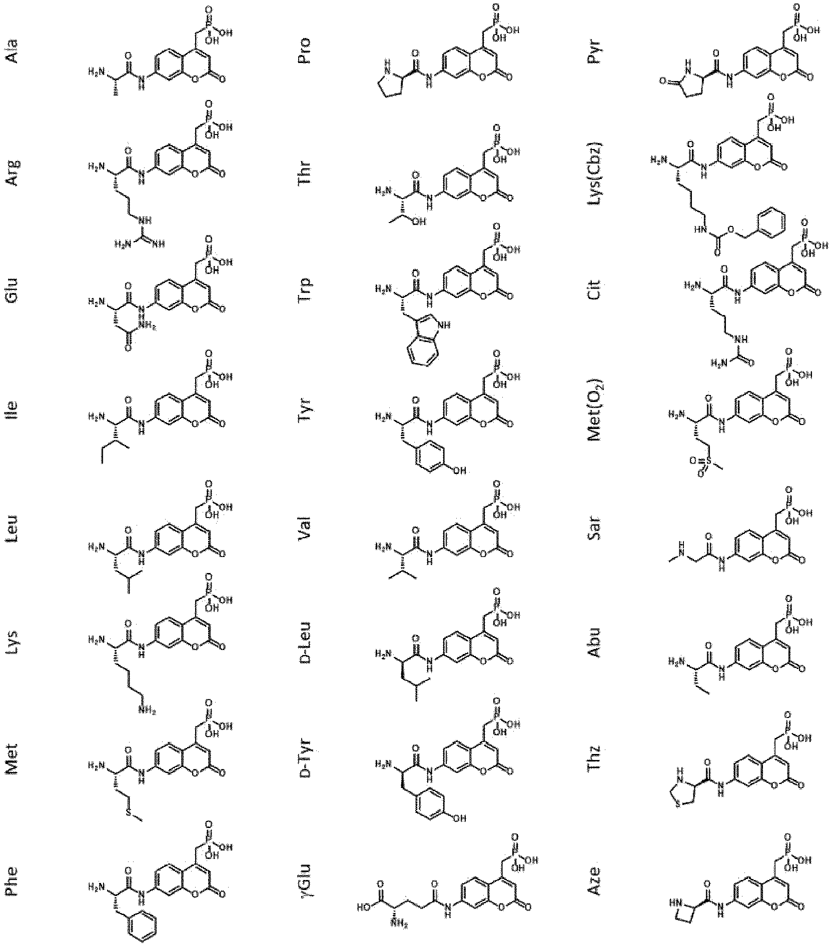

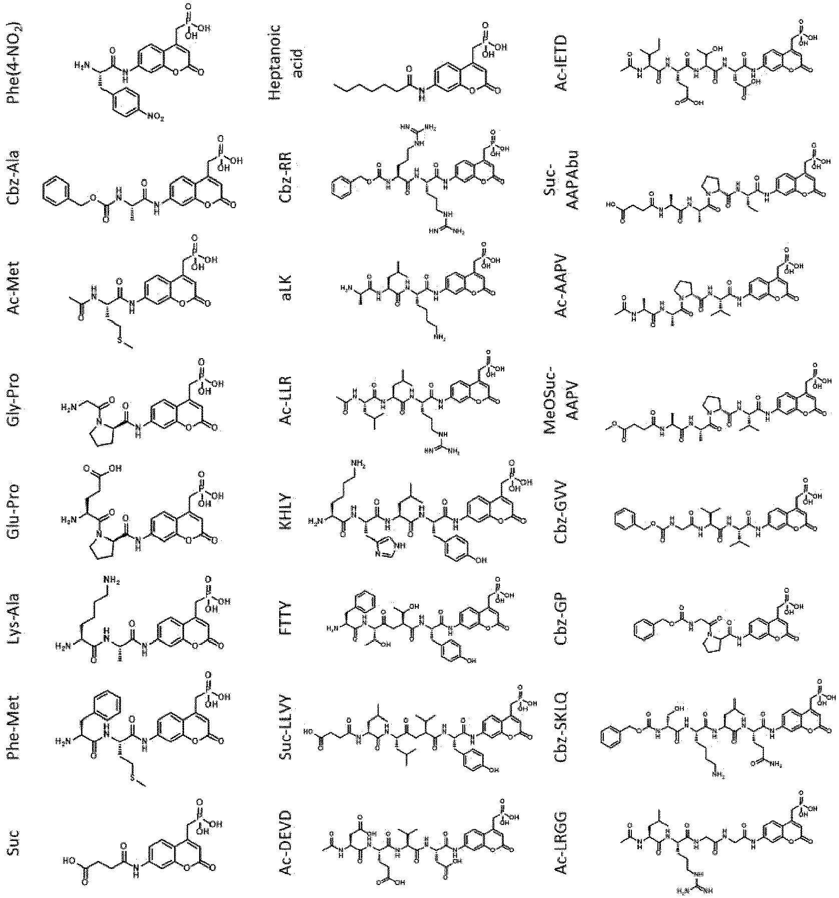

### (2) Fluorescent probe for detection of glycosidase

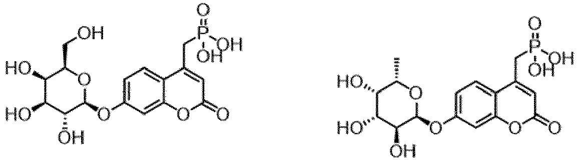

The compound represented by General Formula (III) may have one or two or more asymmetric carbons depending on the type of the substituent, and stereoisomers such as optical isomers or diastereoisomers may exist. Stereoisomers in pure form, any mixture of stereoisomers, racemates, and the like are all encompassed within the scope of the present invention. The compound represented by General Formula (III) or a salt thereof may exist as a hydrate or a solvate, but any of these substances is encompassed within the scope of the present invention. The type of solvent forming a solvate is not particularly limited, and examples thereof include solvents such as ethanol, acetone, and isopropanol.

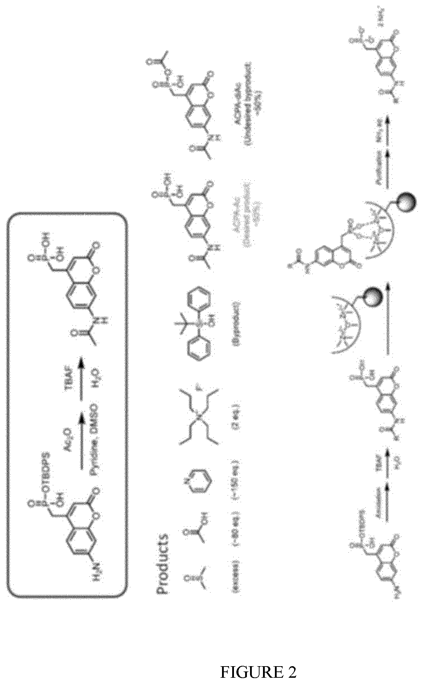

As a non-limiting example of the preparation method of the present invention, an example in which a fluorescent probe of the present invention was synthesized using PMAC in such a manner that a phosphonic acid group was first protected with TBDPS-Cl and an amino group was amidated, then deprotected, and reacted with phos-tag to perform purification and pH adjustment, is shown in Fig. 2.

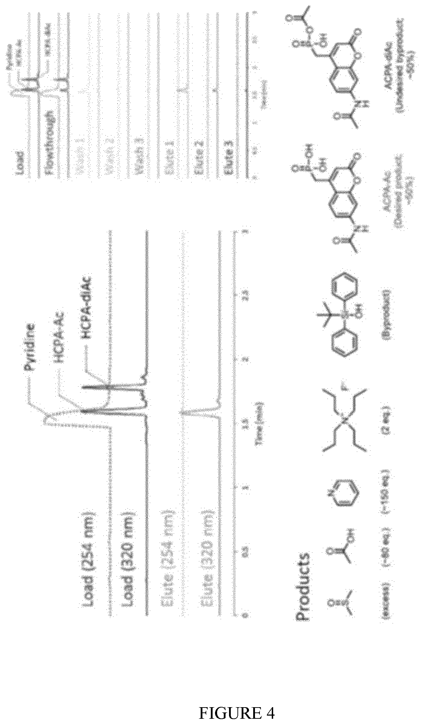

When the amidation of PMAC shown in the upper part of Fig. 2 is completed, various reactants, reagents, and products are contained in the liquid phase as shown in the middle part of Fig. 2. When the compound of Formula (II) (a carrier bonded to phos-tag) is added, the compound specifically captures PMAC-AC having a phosphonic acid group, and thus the target fluorescent probe of the present invention can be obtained only by purification and pH adjustment thereafter (see Fig. 4).

The preparation method of the present invention enables probe synthesis by simple experimental operation, and can be performed, for example, with a small plastic tube (for example, a 1.5 mL plastic tube).

### 3. Fluorescent probe of Present Invention

As described above, the fluorescent probe of the present invention that is the compound represented by Formula (III) can be used as a fluorescent probe for detection of one or more kinds of enzyme activity depending on the kind of the functional group introduced as B.

Therefore, still another implementation of the present invention is a fluorescent probe for detection of enzyme activity containing a compound represented by General Formula (III) or a salt thereof.

Still another mode of the present invention is a method for detecting activity of a target enzyme in a cell, the method including the steps of: (a) introducing the fluorescent probe of the present invention into a cell; and (b) measuring fluorescence emitted by reaction of the fluorescent probe with the target enzyme in the cell.

The fluorescent probe can be introduced into a cell by using a cell lysate, a cultured cell, or the like.

The method of the present invention can further include observing a fluorescence response using a fluorescence imaging means. As a means for observing a fluorescence response, a fluorometer having a wide measurement wavelength can be used, but the fluorescence response can also be visualized using a fluorescence imaging means capable of displaying the fluorescence response as a two-dimensional image. By using the fluorescence imaging means, the fluorescence response can be two-dimensionally visualized, so that the target enzyme can be instantly visually recognized. As a fluorescence imaging device, a device known in the art can be used. In some cases, it is also possible to detect a reaction between the sample to be measured and the fluorescent probe by a change in ultraviolet-visible absorption spectrum (for example, a change in absorbance at a specific absorption wavelength).

A method of using the fluorescent probe of the present invention is not particularly limited, and the fluorescent probe can be used in the same manner as a conventionally known fluorescent probe. Usually, the compound of the present invention or a salt thereof may be dissolved in an aqueous medium such as physiological saline or a buffer solution, a mixture of an aqueous medium and a water-miscible organic solvent such as ethanol, acetone, ethylene glycol, dimethyl sulfoxide, or dimethylformamide, or the like, this solution may be added to an appropriate buffer solution containing cells and tissues, and a fluorescence spectrum is measured. The fluorescent probe of the present invention may be used in the form of a composition in combination with an appropriate additive. For example, the fluorescent probe of the present invention can be combined with an additive such as a buffering agent, a solubilizing agent, or a pH adjusting agent.

The sample of cells to be measured in the step (a) can be cells expressing a target enzyme, but when such cells are cancer cells or cancer tissues expressing a target enzyme, the cancer cells or cancer tissues can be detected or visualized by the detection method of the present invention. That is, the fluorescent probe of the present invention, the composition containing the fluorescent probe, and the detection method of the present invention can also be used for prediction or diagnosis of cancer.

In the present specification, the term "cancer tissue" means any tissue including cancer cells.

The term "tissue" should be construed in the broadest sense, including any part or all of body parts or organs, and should not be construed in any limiting sense. The cancer tissue is preferably a tissue expressing a target enzyme. In the present specification, the term "diagnosis" needs to be interpreted in the broadest sense including confirmation of the presence of a disease at any biological site, for example, confirmation of the presence of cancer tissue macroscopically or under a microscope in the case of cancer.

In the detection method of the present invention, it is preferable to use a kit for detection of a target enzyme containing the fluorescent probe described above. In the kit, the fluorescent probe of the present invention is usually prepared as a solution, but for example, the fluorescent probe of the present invention is provided as a composition in an appropriate form such as a mixture in a powder form, a lyophilizate, a granule, a tablet, or a liquid, and can also be dissolved in distilled water for injection or an appropriate buffer solution at the time of use and applied.

The kit may appropriately contain other reagents and the like as necessary. For example, additives such as a solubilizing agent, a pH adjusting agent, a buffering agent, and an isotonizing agent can be used as the additive, and the blending amount thereof can be appropriately selected by those skilled in the art.

By providing a fluorescent probe for target enzyme detection of the present invention in a well of a microdevice described below, the fluorescent probe can be used for a method for detecting enzyme activity of a target enzyme in a biological sample.

That is, one preferred mode of the present invention is a fluorescent probe for target enzyme detection including the fluorescent probe of the present invention for a microdevice.

### 4. Microdevice

A microdevice according to an embodiment of the present invention includes the fluorescent probe for enzyme detection of the present invention described above.

In the present specification, the "microdevice" includes a microchamber type device, a liposome, a droplet, and the like.

According to the microdevice of the present embodiment, the activity of the target enzyme in the biological sample can be detected with high quantitativity and sensitivity.

The material for the microdevice is not particularly limited, and examples thereof include glass materials, silicon, and plastics containing a dendritic polymer or copolymer. Examples of the glass materials include soda lime glass, Pyrex^{®} glass, Vycor^{®} glass, and quartz glass. Examples of the resin polymer include poly(vinyl chloride), poly(vinyl alcohol), poly(methyl methacrylate), poly(vinyl acetate-co-maleic anhydride), poly(dimethylsiloxane) monomethacrylate, a cyclic olefin polymer, a fluorocarbon polymer, polystyrene, polypropylene, and polyethyleneimine. Examples of the copolymer include poly(vinyl acetate-co-maleic anhydride), poly(styrene-co-maleic anhydride), and poly(ethylene-co-acrylic acid) or derivatives thereof.

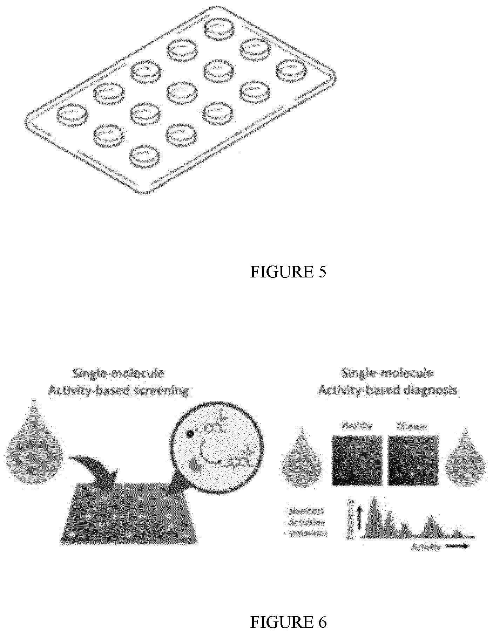

Examples of the shape of the microdevice include a multi-well plate in which an arbitrary number of wells (for example, microwells) are arranged as illustrated in Fig. 5. The number of wells is, for example, 1 or more and 10,000,000 or less, for example, 10 or more and 500,000 or less, for example, about 100,000, or the like, per plate.

The pore diameter of the well of the microdevice may be, for example, 10 nm or more and 10 µm or less, may be, for example, 100 nm or more and 10 µm or less, and may be, for example, 1 µm or more and 10 µm or less.

The depth of the well of the microdevice may be, for example, 10 nm or more and 100 µm or less, may be, for example, 100 nm or more and 80 µm or less, and may be, for example, 200 nm or more and 70 µm or less.

When the pore diameter and depth are within the above ranges, one molecule of the target enzyme can be captured in the well, and the enzyme activity of each molecule of the enzyme in the biological sample can be detected.

The microdevice may include one kind of the above-described fluorescent probe for enzyme detection in one well.

This makes it possible to detect the fluorescence intensity of one kind of the fluorescent probe of the present invention with respect to one molecule of the target enzyme in the biological sample and to compare the enzyme activities of one molecule of the target enzyme with each other.

The amount of the fluorescent probe of the present invention contained in one well of the microdevice may be, for example, 100 nM or more and 1000 µM or less, may be, for example, 1 µM or more and 1000 µM or less, and may be, for example, 10 µM or more and 1000 µM or less.

As a method of using the microdevice, first, a solution containing a biological sample is added to the microdevice. Sealing oil is then added dropwise to enclose the target enzyme in the biological sample in the wells of the microdevice.

The fluorescent probe of the present invention may be added to the solution containing a biological sample.

### 5. Construction of Library of Fluorescent Probe

Studies by the inventors have shown that an organic small molecule fluorescent probe is an excellent molecular tool capable of detecting activity of an enzyme by fluorescence increase, and in particular, it is possible to detect a plurality of enzyme activities in a biological sample with ultra-high sensitivity by using the organic small molecule fluorescent probe for single-molecule enzyme activity detection using the above-described microdevice, and to diagnose a disease by detecting an abnormality thereof (for example, PCT/JP2020/22546, Science Advances 2020).

Since a fluorescent probe used for such activity detection is usually prepared by synthesizing and purifying one compound over a period of several days to several months, it has been difficult to prepare a fluorescent probe group that detects activities of various enzymes present in blood or the like as described above in an exhaustive manner.

On the other hand, in the method for preparing a fluorescent probe of the present invention described above, it is possible to perform probe synthesis and purification by a very simple scheme of only mixing and solution removal, and parallel processing is also possible, so that about ten kinds of probes can also be prepared in a few days, and thus the efficiency of development such as probe improvement can be greatly improved.

That is, still another implementation of the present invention is

A method for preparing one kind of compound represented by Formula (III) below for each vessel of a plurality of reaction vessels by performing the following steps (1) to (5) in parallel in the plurality of reaction vessels:
(1) protecting a T group of a compound represented by Formula (I) below: 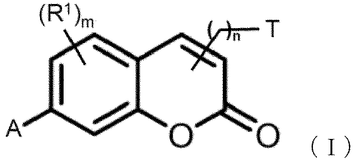 wherein,
   A is an amino group (-NR²H) or a hydroxyl group (-OH),
   where, R² is a hydrogen atom or a substituted or unsubstituted alkyl group having 1 to 8 carbon atoms;
   R¹, when present, is the same or different monovalent substituent present on a benzene ring;
   T is selected from a phosphonic acid group (-P(=O)(OH)₂), a phosphoric acid ester group (-O-P(=O)(OH)₂), or a phosphoric amide group (-NH-P(=O)(OH)₂);
   m is an integer of 0 to 3; and
   n is an integer of 0 to 1;
(2) (i) when A is an amino group (-NR²H), converting the amino group of a product obtained in the step (1) into an amide group (-NR²C(=O)R, R is a hydrogen atom or an alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently a hydrogen atom or an alkyl group having 1 to 8 carbon atoms ), or a sulfonamide group (-NR²-SO₂-R_{c}, R_{c} is a hydrogen atom or an alkyl group having 1 to 8 carbon atoms), or
   (ii) when A is a hydroxyl group, converting the hydroxyl group of a product obtained in the step (1) into an ester group, a phosphoric acid ester group, a sulfuric acid ester group, an ether group, or -O-L' (L' represents a saccharide or a partial structure of a saccharide);
(3) removing a protective group of T of a product obtained in the step (2), which may optionally include a crude purification step after the removal of the protective group;
(4) adding a compound represented by Formula (II) below to a product obtained in the step (3): 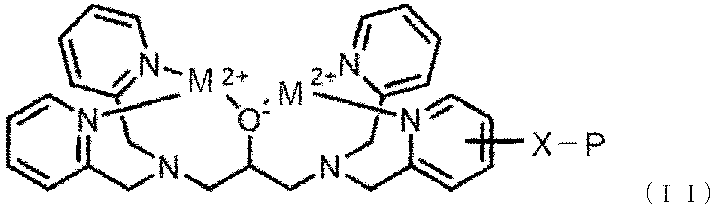 wherein,
   M is Zn or Cu;
   X is a linker group; and
   P is a carrier; and
(5) purifying a product obtained in the step (4) and then liquating or eluting a compound of Formula (III): 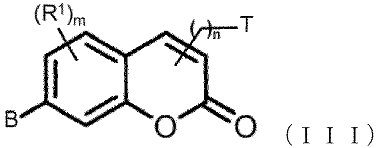 wherein,
   B is selected from an amide group (-NR²C(=O)R, R is a hydrogen atom or an alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently hydrogen atom or an alkyl group having 1 to 8 carbon atoms), a sulfonamide group (-NR²-SO₂-R_{c}, R_{c} is hydrogen atom or an alkyl group having 1 to 8 carbon atoms), an ester group, a phosphoric acid ester group, a sulfuric acid ester group, an ether group, or -O-L' (L' represents a saccharide or a partial structure of a saccharide); and
   R¹, m, and n are as defined in Formula (I).

As described above, by preparing one kind of compound represented by Formula (III) below for each vessel of a plurality of reaction vessels by performing the following steps (1) to (5) in parallel in the plurality of reaction vessels, a group of compounds represented by Formula (III) can be constructed. The method for preparing a compound represented by Formula (III) is also referred to as the "method 2 for preparing a compound of the present invention" or the "method for preparing a compound group of the present invention".

The steps (1) to (5) used in the method 2 for preparing a compound of the present invention are the same as those detailed in detail in the preparation method of the present invention.

When the method 2 for preparing a compound of the present invention is performed, the compound of Formula (I) used in the step (1) may be the same or different in each reaction vessel.

When the compound of Formula (I) used in the step (1) is the same in each reaction vessel, it is preferable to use different reagents in each reaction vessel in order to convert the portion A into a functional group cleaved by contact with a substance to be measured such as an enzyme in the step (2).

As a plurality of reaction vessels used in the method 2 for preparing a compound of the present invention, a plurality (for example, 1 to 30) of small plastic tubes described in the method for preparing a fluorescent probe of the present invention can be used in parallel.

In the method 2 for preparing a compound of the present invention, all the compounds constituting the obtained compound group may be different, or the same compound may be partially contained and most of the compounds may be different.

It is preferable that 80% or more of the compounds constituting the compound group are different, it is more preferable that 90% or more of the compounds constituting the compound group are different, it is further preferable that 95% or more of the compounds constituting the compound group are different, and it is still more preferable that all the compounds constituting the compound group are different.

By performing the method 2 for preparing a compound of the present invention, it is possible to synthesize several tens of probes/day.

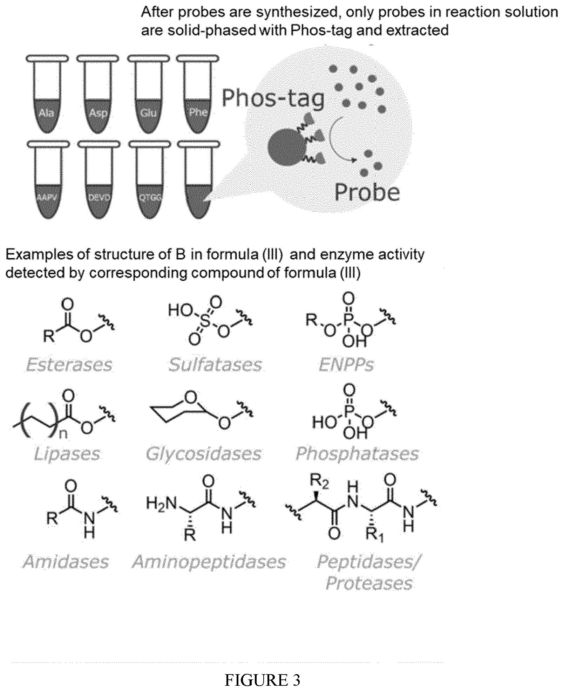

By the method 2 for preparing a compound of the invention, as schematically shown in Fig. 3, it is possible to prepare, at a time, a fluorescent probe group capable of detecting various hydrolytic enzymes (such as an esterase, a sulfatase, ENPPs, a phosphatase, a lipase, a glycosidase, an amidase, an aminopeptidase, a peptidase, and a protease).

It is possible to construct a library of several hundred kinds of compounds capable of detecting a plurality of enzymes by repeating the preparation method 2 of the compound of the present invention several times.

That is, still another mode of the present invention is a method for constructing a library of a compound of Formula (III) (hereinafter, also referred to as the "library construction method of the present invention"), including repeating preparation of a group of compounds of Formula (III) multiple times.

### 6. Method for Detecting Enzyme Activity in Biological Sample

Still another implementation of the present invention is a method for detecting activity of a plurality of enzymes in a biological sample using a compound of Formula (III) (that is, the compound of the present invention) or a library thereof (hereinafter, also referred to as the "detection method of the present invention").

In the detection method of the present invention, a microdevice can be suitably used.

The microdevice used in the detection method of the present invention is a microdevice including the compound of Formula (III) or the library thereof described above.

According to the microdevice of the present embodiment, activity of a plurality of enzymes in a biological sample can be detected with high quantitativity and sensitivity.

The material and shape of the microdevice that can be used in the detection method of the present invention are as described in detail for the microdevice used in the fluorescent probe for enzyme detection of the present invention.

The pore diameter of the well of the microdevice may be, for example, 10 nm or more and 10 µm or less, may be, for example, 100 nm or more and 10 µm or less, and may be, for example, 1 µm or more and 10 µm or less.

The depth of the well of the microdevice may be, for example, 10 nm or more and 100 µm or less, may be, for example, 100 nm or more and 80 µm or less, and may be, for example, 200 nm or more and 70 µm or less.

When the pore diameter and depth are within the above ranges, one molecule of the enzyme can be captured in the well, and the enzyme activity of each molecule of the enzyme in the biological sample can be detected.

The microdevice may include one kind of the compound of Formula (III) (the fluorescent probe of the present invention) in one well.

This makes it possible to detect the fluorescence intensity of one type of the fluorescent probe of the present invention with respect to one molecule of the enzyme in the biological sample and to compare the enzyme activities of one molecule of each of the plurality of enzymes and the compound of the present invention.

The amount of the fluorescent probe of the present invention contained in one well of the microdevice may be, for example, 100 nM or more and 1000 µM or less, may be, for example, 1 µM or more and 1000 µM or less, and may be, for example, 10 µM or more and 1000 µM or less.

As a method of using the microdevice, first, a solution containing a biological sample is added to the microdevice. Sealing oil is then added dropwise to enclose the enzyme in the biological sample in the wells of the microdevice. The fluorescent probe of the present invention may be added to the solution containing a biological sample.

According to the present invention, there is provided a method for detecting activity of a plurality of enzymes in a biological sample, the method including bringing the biological sample (for example, a biological sample, a biopsy sample, a body fluid sample, and an aqueous solution which are isolated from a subject) into contact with the compound of Formula (III). In a certain mode, the biological sample may be a blood sample (for example, a serum sample or a plasma sample). This method may further include measuring the fluorescence of the compound after contacting the compound and the aqueous solution. When the compound emits fluorescence, the presence or absence of the fluorescence indicates the presence of the enzyme in the aqueous solution, and the intensity of the fluorescence indicates the intensity of the activity of the enzyme in the aqueous solution.

One mode of the present invention is a method for detecting enzyme activity in a biological sample, the method including bringing a compound of Formula (III) into contact with a plurality of enzymes.

In one preferred aspect of the method for detecting enzyme activity of the present invention, contacting is performed in the presence of serum.

According to the detection method of the present invention, enzyme activity of a plurality of enzymes in a biological sample can be detected.

The detection method of the present invention will be described in detail below.

### [Step 1]

First, a solution containing a biological sample is added to the microdevice including the compound of Formula (III) or the library thereof described above. Examples of the biological sample include a biological sample, a biopsy sample, a body fluid sample, and an aqueous solution which are isolated from a subject. The biological sample may be a blood sample (for example, a serum sample or a plasma sample).

As the pH of the solution containing a biological sample, any value may be selected. For example, the pH of the solution can be set to a value close to that in the living body, and in this case, the pH may be, for example, 6.0 or more and 8.0 or less. In order to narrow down the target to a specific enzyme group, the pH of the solution can also be made acidic so as to reach an optimum pH.

A solution containing a biological sample and a solution containing the fluorescent probe of the present invention may be separately prepared, the ratio of the biological sample and the fluorescent probe may be appropriately adjusted and mixed, and the mixed solution may be added to the microdevice.

The protein concentration of the biological sample may be usually, for example, 1 pM or more and 100 pM or less, and may be, for example, 10 pM or more and 100 pM or less, as the concentration of a "target protein".

The upper limit of the total protein concentration in the sample may be, for example, about 10 µM.

As a method for measuring the protein concentration of a biological sample, for example, a method using an antibody antigen reaction (for example, ELISA method or the like) can be used as a method for measuring the concentration of a "target protein". Examples of a method for measuring the total protein concentration in a sample include colorimetric methods using a reaction between a protein and a reagent (such as a bicinchoninic acid (BCA) method, Bradford method, Lowry method, and a biuret method).

The biological sample may be diluted using various aqueous solvents or the like so as to have the above concentration. Examples of the aqueous solvent include water, physiological saline, phosphate buffered saline (PBS), tris buffered saline (TBS), and HEPES buffered saline, but are not limited thereto.

### [Step 2]

Sealing oil is then added dropwise to enclose the enzyme in the biological sample in the wells of the microdevice. The sealing oil may be any known sealing oil that is usually used for enclosing a sample in a microdevice, and examples thereof include fluorine-based oil (such as FC-40)

### [Step 3]

Next, fluorescence in the well of the microdevice is detected using a fluorescence scanner, a fluorescence microscope, or the like. The enzyme activity can be evaluated from the detected fluorescence intensity.

What enzyme is contained in the well of the microdevice in which the enzyme activity is detected can be determined, for example, by comparison with an enzyme activity pattern with a separately prepared target protein.

In one mode of the detection method of the present invention, by bringing one kind of the fluorescent probe of the present invention into contact with a plurality of enzymes in a biological sample, the fluorescence intensity of one kind of the fluorescent probe of the present invention can be detected with respect to one molecule of the plurality of enzymes in the biological sample, and the enzyme activities of one molecule of the plurality of enzymes can be compared. In the step 1, the enzyme activity of one molecule of enzyme can be measured by preparing a well (for example, a plurality of wells) containing one molecule of enzyme and a plurality of fluorescent probes.

In another mode of the detection method of the present invention, by bringing each fluorescent probe included in the library of fluorescent probes of the present invention into contact with a plurality of enzymes in a biological sample, the fluorescence intensity of each fluorescent probe of the present invention can be detected with respect to one molecule of the plurality of enzymes in the biological sample, and the enzyme activity of one molecule of the plurality of enzymes can be compared, and it is also possible to screen a more useful fluorescent probe in activity detection of the plurality of enzymes in the biological sample.

By the detection method of the present invention, it is possible to detect one molecular level of various pathology-related enzymes in blood.

Still another mode of the present invention is a method for testing an enzyme assay of a composition containing the compound of Formula (III), the method including bringing the composition into contact with a biological sample containing or suspected of containing an enzyme which cleaves the compound.

In the present test method, a technique similar to the content described in the detection method of the present invention can be used.

### 7. Screening Method

Since the library of the compound of Formula (III) obtained by the library construction method of the present invention includes a plurality of, for example, several hundred kinds of compounds capable of detecting a plurality of enzymes, it is possible to search (screen) biomarker candidate activities from a biological sample using the library in an exhaustive manner. More specifically, it is possible to screen a fluorescent probe useful for diagnosis of a disease and to identify enzyme activity as a biomarker from the screening results.

That is, still another implementation of the present invention is a method for screening a fluorescent probe capable of detecting a biomarker of a specific disease using a library of the compound of Formula (III). In the screening method of the present invention, it is preferable to use a microdevice.

One mode of the present invention is
a method for screening a fluorescent probe capable of detecting a biomarker of a specific disease (hereinafter, also referred to as the "screening method of the present invention"), the method including the steps of:
(1) adding a library of the compound of Formula (III) to a microdevice so that at least one well of the microdevice includes one kind of compound of Formula (III);
(2) adding a solution containing a biological sample to the microdevice so that at least one well containing one molecule of enzyme is generated in the microdevice, the biological sample being a biological sample obtained from a patient with a specific disease or a biological sample obtained from a healthy subject;
(3) bringing the compound of Formula (III) into contact with an enzyme to detect fluorescence in a well of the microdevice, the step including
   bringing the compound of Formula (III) into contact with the biological sample obtained from a patient with a specific disease to measure a fluorescence intensity (first fluorescence intensity) from the compound of Formula (III), and
   bringing the compound of Formula (III) into contact with the biological sample obtained from a healthy subject to measure a fluorescence intensity (second fluorescence intensity) from the compound of Formula (III); and
(4) determining that the compound is a biomarker activity detection fluorescent probe by showing that the compound is a candidate for the fluorescent probe when there is a difference between the first fluorescence intensity and the second fluorescence intensity.

By the screening method of the present invention, it is possible to search (screen) candidate compounds for fluorescent probes capable of detecting biomarker activity from the library of the compound of Formula (III) in an ultra-sensitive and exhaustive manner.

Examples of the biological sample include a biological sample, a biopsy sample, a body fluid sample, and an aqueous solution which are isolated from a subject. The biological sample may be a blood sample (for example, a serum sample or a plasma sample). In the screening method of the present invention, the biological sample is preferably a clinical specimen sample.

The steps (1) and (2) and the procedure of measuring fluorescence in (3) of the screening method of the present invention are performed in the same manner as the procedures described in the detection method of the present invention.

As a specific condition for adding at least one well containing one molecule of enzyme in the step (2), at least one well in one lane is stochastically added at a concentration equal to or higher than the concentration at which one molecule of enzyme is contained.

In the screening method of the present invention, for the steps (1) and (2), a solution containing a biological sample and a solution containing a compound of Formula (III) may be separately prepared, the ratio of the biological sample and the fluorescent probe may be appropriately adjusted and both of them are mixed, and the mixed solution (that is, the solution containing the biological sample and the compound of Formula (III)) may be added so that about one molecule of enzyme in the biological sample is distributed in each one well in one lane of the microdevice.

In one mode of the present invention, as the biological sample in the steps (1) to (3), body fluid samples (including blood, urine, saliva, and the like) derived from a healthy subject and a patient with a disease are used, and fluorescence in a well of the microdevice is detected. As a result of determining the enzyme activity in the biological sample based on the result of detecting fluorescence, when the activity is significantly different between both (that is, body fluid samples derived from a healthy subject and a patient with a disease), the compound of Formula (III) added to the microdevice in the step (1) is determined as a biomarker activity detection fluorescent probe.

In the assay result of the compound of Formula (III) determined as the biomarker activity detection fluorescent probe, when the enzyme activity is significantly different between the body fluid samples derived from the healthy subject and the patient with a disease, it is considered that the difference is caused by the activity of the biomarker candidate in the body fluid sample derived from the patient with a disease, and thus it is highly likely that the biomarker candidate is contained in the body fluid sample. By identifying enzymes contained in wells that are significantly different in the assay results of both body fluid samples, the type of biomarker candidate can be determined.

Depending on the type of disease, a plurality of biomarker candidates may be contained in the body fluid sample derived from a patient with a disease, and in this case, in the screening method of the present invention, it is also possible to determine two or more compounds of Formula (III) as biomarker activity detection fluorescent probes.

### 8. Detection of Biomarker

As described above, the fluorescent probe of the present invention, which is the compound represented by Formula (III), can be used as a fluorescent probe for detection of one or more kinds of enzyme activity depending on the kind of the functional group introduced as B, and the fluorescent probe of the present invention can be further used for detection of a biomarker specific to a predetermined disease to diagnose the disease.

That is, still another implementation of the present invention is a method for diagnosing a disease state by detecting single-molecule enzyme activity using the fluorescent probe of the present invention (hereinafter, also referred to as the "diagnostic method of the present invention").

A conceptual diagram of disease diagnosis using the present invention is shown in Fig. 6. The left figure relates to the screening method of the present invention described above, and the right figure shows an outline of a method for diagnosing a disease state by detecting a single-molecule enzyme activity. By using the present invention, it is expected that disease diagnosis based on information such as the number of enzymes, activity, and fluctuation in activity can be performed.

An example of the fluorescent probe of the present invention that can be used for such biomarker detection, and a non-limiting example of the diagnostic method of the present invention are shown below.

### (1) Detection of Activity of ENPP

That is, still another implementation of the present invention is a method for detecting activity of ENPP in a biological sample, the method including bringing a compound of Formula (IV) or a salt thereof into contact with a biological sample in an aqueous solution, an increase in fluorescence intensity in the aqueous solution indicating the presence of activity of ENPP:

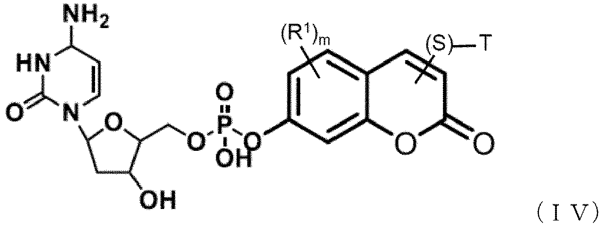

In Formula (IV), R¹, S, T, and m are as described in detail in General Formula (I).

In the method for detecting activity of ENPP, one preferred example of the biological sample is a biological sample of a patient with pancreatic cancer, a patient suspected of having pancreatic cancer, or a healthy subject.

Still another mode of the present invention is a method for diagnosing pancreatic cancer or a method for predicting a possibility that a subject from which a biological sample is derived has pancreatic cancer (hereinafter, also referred to as the "diagnostic or prediction method 1 of the present invention"), the method including the steps of: (a) applying a fluorescent probe containing a compound of Formula (IV) or a salt thereof to a clinical specimen of a test subject; and (b) measuring a fluorescent image of the clinical specimen to which the fluorescent probe is applied.

The application of the fluorescent probe to a clinical specimen in the step (a) can be performed, for example, by locally spraying a solution of the fluorescent probe to the clinical specimen.

The diagnostic or prediction method 1 of the present invention can be performed during the surgical treatment of pancreatic cancer or can be performed ex vivo. The diagnostic or prediction method 1 of the present invention also includes a mode in which medical practice is not included.

Still another mode of the present invention is a method for diagnosing pancreatic cancer or a method for predicting a possibility that a subject from which a biological sample is derived has pancreatic cancer (hereinafter, also referred to as the "diagnostic or prediction method 2 of the present invention"), the method including the steps of: (a) bringing a biological sample obtained from a subject into contact with a fluorescent probe containing a compound of General Formula (IV) or a salt thereof; and (b) measuring a fluorescence intensity of the biological sample brought into contact with the fluorescent probe.

The diagnostic or prediction methods 1 and 2 of the present invention are based on the detection of the enzyme activity of ENPP by the compound of Formula (IV) or a salt thereof. As ENPP, it is preferable to detect the enzyme activity of ENPP3 specifically found in pancreatic cancer.

The diagnostic or prediction method 2 of the present invention can preferably be performed using a microdevice.

The diagnostic or prediction method 2 of the present invention can be performed by preparing a solution containing a biological sample and a compound of General Formula (IV) or a salt thereof, adding the solution to a microdevice, enclosing the added solution in each well, incubating the microdevice in which the solution is enclosed, and then measuring the fluorescence intensity of each well with a fluorescence microscope.

The diagnostic method of the present invention can include measuring the number of wells in which the detected fluorescence intensity is equal to or higher than a predetermined intensity, that is, the number of wells in which ENPP is considered to be enclosed.

Specifically, the diagnostic or prediction method 2 of the present invention is performed, for example, as follows, but is not limited thereto.

The compound of General Formula (IV) is diluted with an assay buffer to a predetermined concentration (for example, 200 µM). The composition of the assay buffer can be appropriately determined, and can be, for example, 100 mM Tris-HCl (pH 9.3), 1 mM MgCl₂, or 0.5% (w/v) CHAPS.

Next, a biological sample, preferably a blood sample (for example, a serum sample or a plasma sample) collected from a subject (including both of a patient with pancreatic cancer and a healthy subject) is diluted with the above assay buffer (for example, about 250 times), and mixed with a diluted solution of the compound of General Formula (IV) at a predetermined ratio (for example, equal amount).

The mixed solution of both was added to a microdevice, followed by the addition of sealing oil to enclose the solution in each well.

The microdevice in which the solution is enclosed is incubated under predetermined conditions (for example, 25°C for 40 minutes), and then the fluorescence intensity of each well is measured with a fluorescence microscope.

The number of wells in which the detected fluorescence intensity is equal to or higher than a predetermined intensity (for example, 2500 AU or more), that is, the number of wells in which ENPP is considered to be enclosed is measured.

In order to diagnose that the subject from which the biological sample is derived is pancreatic cancer and predict a possibility of pancreatic cancer from the number of wells in which ENPP is considered to be enclosed as measured above, an ROC curve for determination based on the result of ENPP activity measurement using a compound of General Formula (IV) or a salt thereof is created, and the possibility of pancreatic cancer is determined by the ROC curve.

In preferred aspects of the detection method of the present invention and the diagnostic method of the present invention, the compound of General Formula (VI) is preferably the following compound.

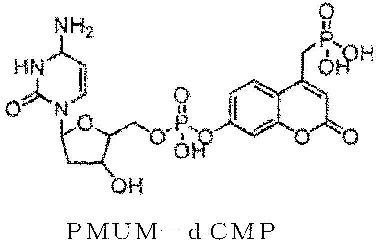

Still another implementation of the present invention is a fluorescent probe for detection of pancreatic cancer for use in the diagnostic or prediction methods 1 and 2 of the present invention, containing a compound of General Formula (IV) or a salt thereof.

The method of using the fluorescent probe for detection of pancreatic cancer is the same as that described for the fluorescent probe of the present invention.

Still another implementation of the present invention is a kit for detection of pancreatic cancer cells or tissues, containing a compound of General Formula (IV) or a salt thereof.

In the kit, the compound of General Formula (IV) or a salt thereof is usually prepared as a solution, but for example, the fluorescent probe of the present invention is provided as a composition in an appropriate form such as a mixture in a powder form, a lyophilizate, a granule, a tablet, or a liquid, and can also be dissolved in distilled water for injection or an appropriate buffer solution at the time of use and applied.

The kit may appropriately contain other reagents and the like as necessary. For example, additives such as a solubilizing agent, a pH adjusting agent, a buffering agent, and an isotonizing agent can be used as the additive, and the blending amount thereof can be appropriately selected by those skilled in the art.

In the fluorescent probe for detection of pancreatic cancer and the kit for detection of pancreatic cancer cells or tissues, the compound of General Formula (VI) is preferably PMUM-dCMP described above.

### (2) Detection of Activity of CD 13 and Detection of Activity of DPP4

Still another implementation of the present invention is a method for detecting activity of CD13 in a biological sample, the method including bringing the compound of the present invention (hereinafter, also referred to as "Compound A of the present invention"), in which B is -NR²-CO-L, a moiety of -CO-L is an alanine, lysine, arginine, or methionine residue in General Formula (III), into contact with a biological sample in an aqueous solution, an increase in fluorescence intensity in the aqueous solution indicating the presence of activity of CD13.

Still another implementation of the present invention is a method for diagnosing pancreatic cancer or a method for predicting a possibility that a subject from which a biological sample is derived has pancreatic cancer, the method comprising detecting, by a microdevice, single-molecule enzyme activity of CD 13 in a biological sample obtained from a subject, using a fluorescent probe containing Compound A of the present invention (hereinafter, also referred to as the "diagnostic or prediction method A1 of the present invention"), in which when B is -NR²-CO-L, a moiety of - CO-L is an alanine, lysine, arginine, or methionine residue in General Formula (III).

Still another mode of the present invention is a method for diagnosing pancreatic cancer or a method for predicting a possibility that a subject from which a biological sample is derived has pancreatic cancer (hereinafter, also referred to as the "diagnostic or prediction method A2 of the present invention"), the method including the steps of: (a) bringing, by a microdevice, a biological sample obtained from a subject into contact with a fluorescent probe containing Compound A of the present invention; and (b) measuring a fluorescence intensity of the biological sample brought into contact with the fluorescent probe.

The diagnostic or prediction methods A1 and A2 of the present invention are based on the detection of the enzyme activity of one molecule of CD13 by Compound A of the present invention.

Still another implementation of the present invention is a method for detecting activity of DPP4 in a biological sample, the method including bringing the compound of the present invention (hereinafter, also referred to as "Compound B of the present invention"), in which B is -NR²-CO-L, a moiety of -CO-L is -Pro-Xaa (Pro represents a proline residue and Xaa represents an amino acid residue such as glycine, serine, or glutamic acid) peptide in General Formula (III), into contact with a biological sample in an aqueous solution, an increase in fluorescence intensity in the aqueous solution indicating the presence of activity of DPP4.

Still another implementation of the present invention is a method for diagnosing pancreatic cancer or a method for predicting a possibility that a subject from which a biological sample is derived has pancreatic cancer, the method comprising detecting, by a microdevice, single-molecule enzyme activity of DPP4 in a biological sample obtained from a subject, using a fluorescent probe containing Compound B of the present invention (hereinafter, also referred to as the "diagnostic or prediction method B 1 of the present invention"), when B is -NR²-CO-L, and a moiety of -CO-L is -Pro-Xaa (Pro represents a proline residue and Xaa represents an amino acid residue such as glycine, serine, or glutamic acid) peptide in General Formula (III).

Still another mode of the present invention is a method for diagnosing pancreatic cancer or a method for predicting a possibility that a subject from which a biological sample is derived has pancreatic cancer (hereinafter, also referred to as the "diagnostic or prediction method B2 of the present invention"), the method including the steps of: (a) bringing, by a microdevice, a biological sample obtained from a subject into contact with a fluorescent probe containing Compound B of the present invention; and (b) measuring a fluorescence intensity of the biological sample brought into contact with the fluorescent probe.

The diagnostic or prediction methods B1 and B2 of the present invention are based on the detection of the enzyme activity of one molecule of DPP4 by Compound B of the present invention.

Still another mode of the present invention is a method for diagnosing pancreatic cancer or a method for predicting a possibility that a subject from which a biological sample is derived has pancreatic cancer, the method including detecting, by a microdevice, single-molecule enzyme activity of CD13 in a biological sample obtained from a subject, using a fluorescent probe containing the compound of the present invention, in which when B is -NR²-CO-L, a moiety of -CO-L is an alanine, lysine, arginine, or methionine residue in General Formula (III), and
detecting, by a microdevice, single-molecule enzyme activity of DPP4 in a biological sample obtained from a subject, using a fluorescent probe containing the compound of the present invention, in which when B is -NR²-CO-L, a moiety of -CO-L is -Pro-Xaa (Pro represents a proline residue and Xaa represents an amino acid residue such as glycine, serine, or glutamic acid) peptide in General Formula (III).

The diagnostic or prediction methods A1 and A2 (or B1 and B2) of the present invention can be performed by preparing a solution containing a biological sample and Compound A (or B) of the present invention, adding the solution to a microdevice, enclosing the added solution in each well, incubating the microdevice in which the solution is enclosed, and then measuring the fluorescence intensity of each well with a fluorescence microscope.

The diagnostic or prediction method of the present invention can include measuring the number of wells in which the detected fluorescence intensity is equal to or higher than a predetermined intensity, that is, the number of wells in which CD13 (or DPP4) is considered to be enclosed.

The subjects in the diagnostic or prediction methods A1, A2, B1, and B2 of the present invention, the combination of the diagnostic or prediction methods A1 and B1 of the present invention, the combination of the diagnostic or prediction methods A2 and B2 of the present invention, and the like (these are also collectively referred to as the "diagnostic or prediction method of the present invention") include patients with pancreatic cancer, patients suspected of having pancreatic cancer (such as diabetic patients with a high risk of pancreatic cancer, humans with pancreatic cancer patients in family, and smokers), and healthy subjects.

That is, the diagnostic or prediction method of the present invention is performed not only for a patient with pancreatic cancer or a patient suspected of having pancreatic cancer but also for the purpose of confirming the possibility of pancreatic cancer with respect to a healthy middle aged person (healthy subject), and can also contribute to finding early pancreatic cancer by performing an image examination (detailed examination) such as MRI on a subject having a high possibility of pancreatic cancer.

The diagnostic or prediction method of the present invention can be performed during the surgical treatment of pancreatic cancer or can be performed ex vivo. The diagnostic or prediction method of the present invention also includes a mode in which medical practice is not included.

Specifically, the diagnostic or prediction methods A1 and A2 of the present invention are performed, for example, as follows, but is not limited thereto.

Compound A of the present invention is diluted with an assay buffer to a predetermined concentration (for example, 100 µM). The composition of the assay buffer can be appropriately determined, and can be, for example, 100 mM HEPES-NaOH (pH 7.4), 1 mM MgCl₂, 1 mM CaCl₂, or 3 mM Triton X-100.

Next, a biological sample, preferably a blood sample (for example, a serum sample or a plasma sample) collected from a subject (including both of a patient with pancreatic cancer and a healthy subject) is diluted with the above assay buffer (for example, about 10,000 times), and mixed with a diluted solution of Compound A of the present invention at a predetermined ratio (for example, equal amount).

The mixed solution of both was added to a microdevice, followed by the addition of sealing oil to enclose the solution in each well.

The microdevice in which the solution is enclosed is incubated under predetermined conditions (for example, 37°C for 2 hours), and then the fluorescence intensity of each well is measured with a fluorescence microscope.

The number of wells in which the detected fluorescence intensity is equal to or higher than a predetermined intensity (for example, 2500 AU or more), that is, the number of wells in which CD13 is considered to be enclosed is measured.

In order to diagnose that the subject from which the biological sample is derived is pancreatic cancer and predict a possibility of pancreatic cancer from the number of wells in which CD13 is considered to be enclosed as measured above, an ROC curve for determination based on the result of CD 13 activity measurement using Compound A of the present invention is created, and the possibility of pancreatic cancer is determined by the ROC curve.

Specifically, the diagnostic or prediction methods B1 and B2 of the present invention are performed, for example, as follows, but is not limited thereto.

Compound B of the present invention is diluted with an assay buffer to a predetermined concentration (for example, 100 µM). The composition of the assay buffer can be appropriately determined, and can be 100 mM HEPES-NaOH (pH 7.4), 1 mM MgCl₂, 1 mM CaCl₂, or 3 mM Triton X-100.

Next, a biological sample, preferably a blood sample (for example, a serum sample or a plasma sample) collected from a subject (including both of a patient with pancreatic cancer and a healthy subject) is diluted with the above assay buffer (for example, about 2,500 times), and mixed with a diluted solution of Compound B of the present invention at a predetermined ratio (for example, equal amount).

The mixed solution of both was added to a microdevice, followed by the addition of sealing oil to enclose the solution in each well.

The microdevice in which the solution is enclosed is incubated under predetermined conditions (for example, 25°C for 2 hours), and then the fluorescence intensity of each well is measured with a fluorescence microscope.

The histogram created based on the fluorescence intensity of each well is approximated to a curve including two normal distributions. A well having a fluorescence intensity higher than the minimum value of the approximate curve is defined as a high activity group, a well having a fluorescence intensity lower than the minimum value of the approximate curve is defined as a low activity group, and the ratio of the high activity group to the total of these groups is measured.

In order to diagnose that the subject from which the biological sample is derived is pancreatic cancer and predict a possibility of pancreatic cancer from the proportion of the highly active group measured above, an ROC curve for determination based on the result of DPP4 activity measurement using Compound B of the present invention is created, and the possibility of pancreatic cancer is determined by the ROC curve.

Non-limiting examples of Compound A of the present invention include the following compounds.

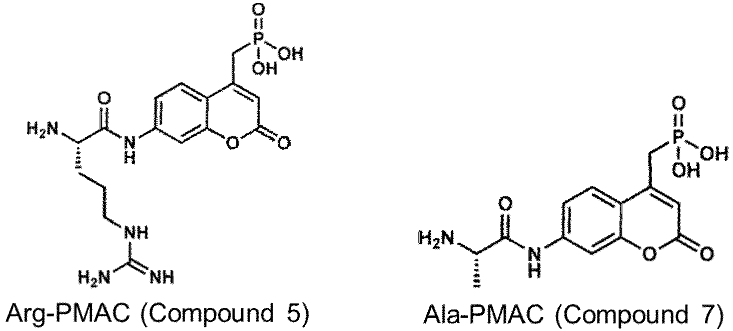

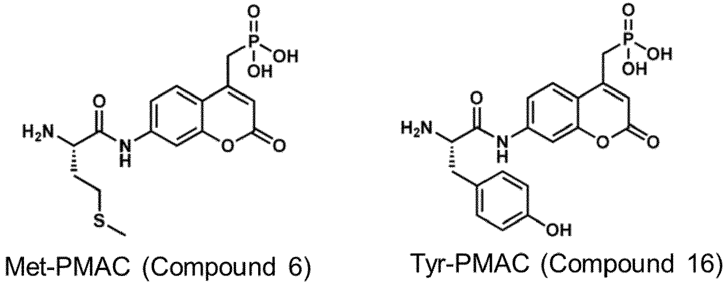

Non-limiting examples of Compound B of the present invention include the following compounds.

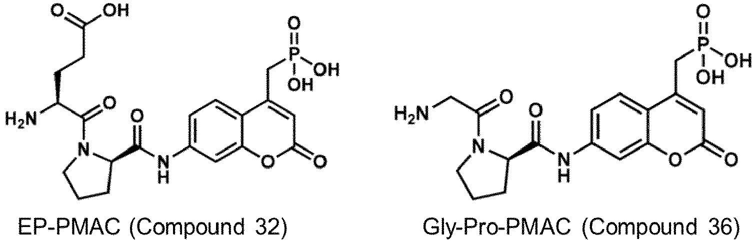

### EXAMPLES

Hereinafter, the present invention will be described with reference to Examples; however, the present invention is not limited thereto.

### [Sample and Measurement Method]

Reagents and solvents for organic synthesis were supplied from Tokyo Chemical Industry Co., Ltd. (TCI), Wako Pure Chemical Industries, Ltd., or Aldrich Chemical Company, and were used without further purification.

Proton nuclear magnetic resonance (¹H NMR) spectra were recorded with JEOL JMN-LA400 instrument.

Mass spectra were measured with JEOL JMS-T100LP AccuTOF^{™} LC-plus4G.

### 1. Synthesis of Fluorescent Mother Nucleus Having Phosphonic Acid

### [Synthesis Example 1]

### Synthesis of Compound 2

A compound (Compound 2) in which phosphonic acid of NH₂ type fluorescent mother nucleus was protected was synthesized by the following procedures.

### (1) Synthesis of Compound 1 (TFA-PMAC (4-phosphonomethyl-7-aminocoumarin): ((2-oxo-7-(2,2,2-trifluoroacetamide)-2H-chromene-4-yl)methyl)phosphonic acid))

Compound 1 was synthesized according to the following scheme.

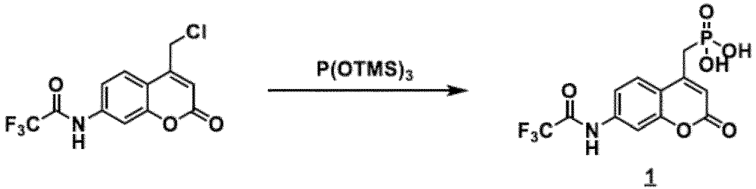

N-(4-(chloromethyl)-2-oxo-2H-chromene-7-yl)-2,2,2-trifluoroacetamide (182 mg, 0.60 mmol) was dissolved in P(OTMS)₃ (2 mL, 6.0 mmol), refluxed entirely at 170°C for 5 minutes, and then extracted with THF and brine, and the organic phase was evaporated. The residue was purified by MPLC (eluent: A/B = 95/5 > 5/95, A: 0.1% TFA-containing 100% H₂O, B: 0.1% TFA-containing 100% CH₃CN). Toluene was added to the eluent, and the eluent was evaporated until colorless solid Compound 1 (80 mg, 0.23 mmol, yield: 38%) was obtained.

Analysis results of the obtained compound by ¹H-NMR, ¹³C-NMR, and a high-resolution mass spectrometry (HR-MS) are shown below.

¹H-NMR (400 MHz, CD₃OD) δ 7.84 (d, J = 9.1 Hz, 1H), 7.74 (d, J = 2.3 Hz, 1H), 7.57 (dd, J = 8.7, 2.3 Hz, 1H), 6.35 (d, J = 4.1 Hz, 1H), 3.37 (d, J = 22.9 Hz, 2H)
¹³C-NMR (100 MHz, CDCl₃) δ 161.0, 154.0, 149.4, 140.0, 126.8, 117.2, 116.3, 115.2, 115.1, 114.4, 107.9,30.9.
HRMS (ESI-): Calcd. for [M-H]⁻, 350.00413, Found, 350.00261 (-1.52 mmu).

### (2) Synthesis of Compound 2 (PMAC-TBDPS: tert-butyldiphenylsilylhydrogen ((7-amino-2-oxo-2H-chromene-4-yl)methyl)phosphonate)

Compound 2 was synthesized according to the following scheme.

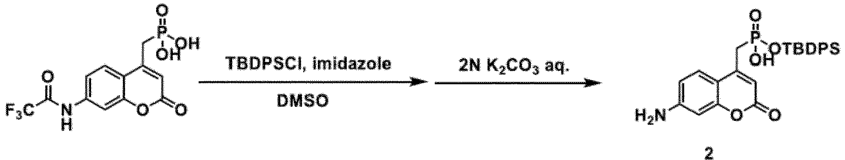

TBDPS-Cl (219 µL, 0.86 mmol) was added dropwise to a DMSO (1.5 mL) solution of Compound 1 (150 mg, 0.43 mmol) and imidazole (117 mg, 1.72 mmol). The reaction mixture was stirred in air at room temperature for 1 hour. Thereafter, a 2 N K₂CO₃ aqueous solution (4.5 mL) was added, the whole was heated at 60°C for 30 minutes, and then the mixture was purified by MPLC (eluent: A/B = 70/30 > 0/100, A: 0.1 M TEAA-containing 100% H₂O, B: 0.1 M TEAA-containing 100% CH₃CN). The eluent was evaporated to distill off the organic solvent and the aqueous phase was extracted with AcOEt while acidifying. The organic solution was washed with brine, dried over Na₂SO₄, and evaporated until yellow solid Compound 2 (65 mg, 0.13 mmol, yield: 31%) was obtained.

Analysis results of the obtained compound by ¹H-NMR, ¹³C-NMR, and a high-resolution mass spectrometer (HR-MS) are shown below.

¹H-NMR (400 MHz, CDCl₃) δ 7.72-7.53 (m, 4H), 7.46-7.27 (m, 6H), 7.20-7.01 (m, 1H), 6.41 (s, 1H), 6.27 (d, J = 8.7 Hz, 1H), 5.82 (d, J = 3.7 Hz, 2H), 2.91 (d, J = 23.3 Hz, 2H), 1.04 (s, 9H)
¹³C-NMR (100 MHz, CDCl3) δ 161.2, 155.5, 147.9, 135.3, 131.7, 130.5, 129.1, 128.3, 128.0, 127.0, 112.5, 112.1, 101.8, 32.2, 26.4, 19.4.
HRMS (ESI-): Calcd. for [M-H]⁻, 492.13961, Found, 492.13778 (-1.83 mmu).

### [Synthesis Example 2]

### Synthesis of Compound 4

A compound (Compound 4) in which phosphonic acid of OH type fluorescent mother nucleus was protected was synthesized by the following procedures.

### (1) Synthesis of Compound 3 (PMUM (4-phosphonomethylumbelliferone):((7-hydroxy-2-oxo-2H-chromene-4-yl)methyl)phosphonic acid)

Compound 3 was synthesized according to the following scheme.

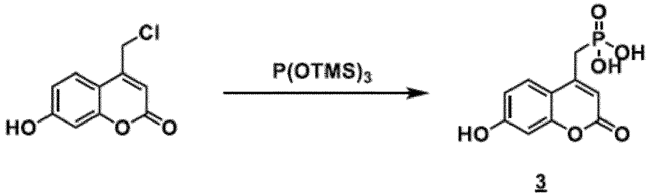

4-(Chloromethyl)-7-hydroxy-2H-chromene-2-one (209 mg, 0.99 mmol) was dissolved in P(OTMS)₃ (5 mL, 14.9 mmol), refluxed entirely at 170°C for 1 hour, and then extracted with THF and brine to be acidic with a 2 N HCl aqueous solution, and the organic phase was evaporated. The residue was purified by MPLC (eluent: A/B = 95/5 > 5/95, A: 0.1% TFA-containing 100% H₂O, B: 0.1% TFA-containing 100% CH₃CN). Toluene was added to the eluent, and the eluent was evaporated. The product was freeze-dried to obtain colorless solid Compound 3 (105 mg, 0.41 mmol, yield: 41%).

Analysis results of the obtained compound by ¹H-NMR, ¹³C-NMR, and a high-resolution mass spectrometer (HR-MS) are shown below.

¹H-NMR (400 MHz, CD₃OD) δ 7.70 (d, J = 8.7 Hz, 1H), 6.79 (dd, J = 8.7, 2.3 Hz, 1H), 6.68 (d, J = 2.3 Hz, 1H), 6.19 (d, J = 4.6 Hz, 1H), 3.32 (d, J = 23.3 Hz, 2H).

¹³C-NMR (100 MHz, CD3OD) δ 162.1, 161.8, 155.6, 150.5, 127.3, 112.9, 111.8, 111.6, 102.1, 31.1.

HRMS (ESI⁺): Calcd. for [M+H]⁺, 257.02150, Found, 257.02155 (+0.05 mmu).

### (2) Synthesis of Compound 4 (PMUM-TBDPS: tert-butyldiphenylsilylhydrogen ((7-hydroxy-2-oxo-2H-chromene-4-yl)methyl)phosphonate)

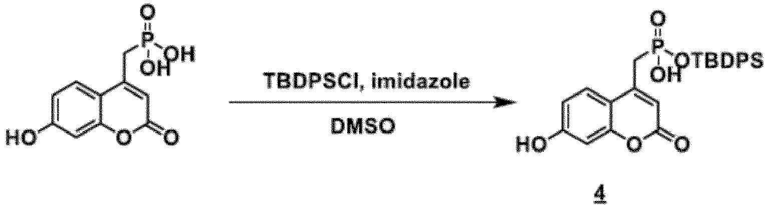

TBDPS-Cl (298 µL, 1.17 mmol) was added dropwise to a DMSO (2 mL) solution of Compound 3 (200 mg, 0.78 mmol) and imidazole (212 mg, 3.12 mmol). The reaction mixture was stirred in air at room temperature for 15 minutes. To the mixture, 0.1 M TEAA-containing 100% H₂O and 0.1 M TEAA-containing 100% CH₃CN were added, and the mixture was purified by MPLC (eluent: A/B = 70/30 > 0/100, A: 0.1 M TEAA-containing 100% H₂O, B: 0.1 M TEAA-containing 100% CH₃CN). The eluent was evaporated to distill off the organic solvent, and filtration was performed. The residue was dried to obtain colorless solid Compound 4 (182 mg, 0.37 mmol, yield: 47%).

Analysis results of the obtained compound by ¹H-NMR, ¹³C-NMR, and a high-resolution mass spectrometer (HR-MS) are shown below.

1H-NMR (400 MHz, CDCl₃) δ 7.91-7.81 (dd, J = 8.0, 1.6 Hz, 4H), 7.48 (d, J = 8.7 Hz, 1H), 7.44-7.33 (m, 6H), 6.56 (dd, J = 8.7, 2.3 Hz, 1H), 6.52 (d, J = 2.3 Hz, 1H), 5.95 (d, J = 4.1 Hz, ¹H), 3.12 (d, J = 22.4 Hz, 2H), 1.13 (s, 9H)
¹³C-NMR (100 MHz, CDCl3) δ 162.7, 162.2, 155.5, 153.2, 153.1, 135.7, 134.1, 129.8, 127.7, 114.0, 111.6, 110.7, 102.8, 37.1, 26.8, 19.5.
HRMS (ESI⁻): Calcd. for [M-H]⁻, 493.12363, Found, 493.12158 (-2.05 mmu).

### 2. Synthesis of Fluorescent Probe by Solid-Phase Extraction Using Phos-tag

### [Synthesis Example 3]

### Synthesis Example of Amidase Probe

An amidase probe was synthesized by the following procedures according to the following reaction scheme using the compound (Compound 2) in which phosphonic acid of NH₂ type fluorescent mother nucleus was protected.

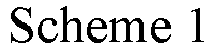

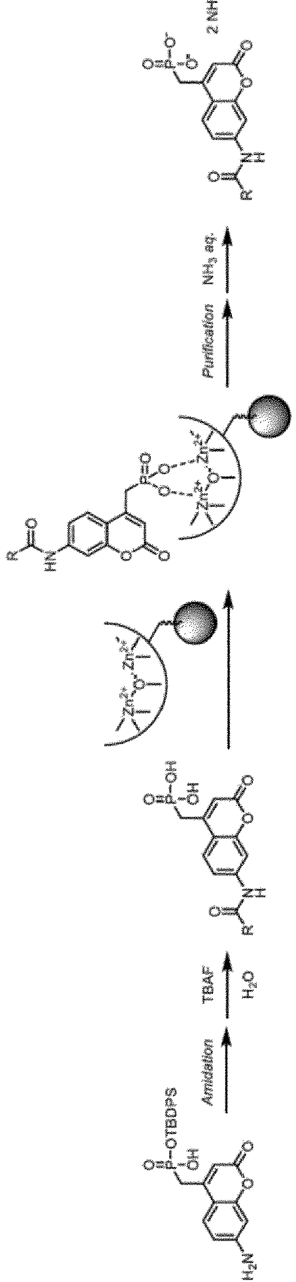

1. PMAC-TBDPS (5 µmol), COMU (30 µmol), and acetic acid (30 µmol) were mixed in a 1.5 mL test tube and dissolved in 30 µL of NMP.
2. The reaction mixture was stirred at 60°C.
3. 300 µL of TFA, 5 µL of triethylsilane, and 5 µL of H₂O were added and the reaction mixture was stirred.
4. 1 mL of Et₂O was added and the mixture was centrifuged (15,000 rpm × 10 min, 4°C) to remove the supernatant.
5. The residue was dissolved in a 1 mL load buffer solution (80% Bis Tris buffer solution (1 M, pH 6.8) and 20% MeCN) and supported on 500 µL phos-tag beads.
6. The beads were washed five times with 1 mL of wash buffer solution (80% H₂O and 20% MeCN, pH 6.8, containing 100 mM Bis Tris-AcOH).
7. The beads were washed three times with 1 mL of washing solution (80% H₂O and 20% MeCN).
8. The compound was eluted three times with 300 µL of an eluent (80% H₂O and 20% MeCN, containing 1% ethylenediamine), neutralized by adding 500 µL of H₂O (containing 2% AcOH) to the solution, and then freeze-dried.

Fig. 4 shows results of analyzing the compound composition in the solution by HPLC in the process from 6.

"Load" indicates the composition of the solution added to the beads in the step of 6., and "Flowthrought" indicates the solution composition of the supernatant removed after the target substance was retained in the beads in the step of 6. "Wash" indicates the solution composition of each supernatant that was washed three times in the step of 7. "Elute" indicates the composition of the solution liquated in the step of 8.

### [Synthesis Example 4]

### Synthesis Example of Aminopeptidase and Amidase Probes

An aminopeptidase probe was synthesized by the following procedures according to the following reaction scheme using the compound (Compound 2) in which phosphonic acid of NH₂ type fluorescent mother nucleus was protected. 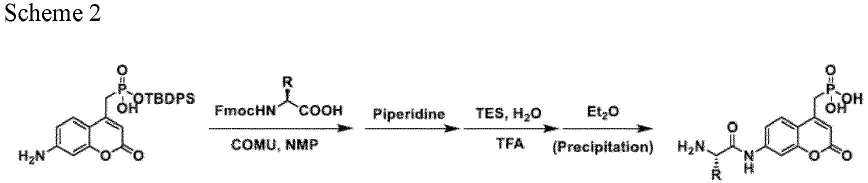 R: Side chain of any amino acid

1. PMAC-TBDPS (5 µmol), COMU (30 µmol), and Fmoc-protected amino acid (30 µmol) were mixed in a 1.5 mL test tube and dissolved in 30 µL of NMP.
2. The reaction mixture was stirred at 60°C.
3. 20 µL of piperidine was added and the reaction mixture was stirred.
4. 300 µL of TFA, 5 µL of triethylsilane, and 5 µL of H₂O were added and the reaction mixture was stirred.
5. 1 mL of Et₂O was added and the mixture was centrifuged (15,000 rpm × 10 min, 4°C) to remove the supernatant.
6. The residue was dissolved in a 1 mL load buffer solution (80% Bis Tris buffer solution (1 M, pH 6.8) and 20% MeCN) and supported on 500 µL phos-tag beads.
7. The beads were washed five times with 1 mL of wash buffer solution (80% H₂O and 20% MeCN, pH 6.8, containing 100 mM Bis Tris-AcOH).
8. The beads were washed three times with 1 mL of washing solution (80% H₂O and 20% MeCN).
9. The compound was eluted three times with 300 µL of an eluent (80% H₂O and 20% MeCN, containing 1% ethylenediamine), neutralized by adding 500 µL of H₂O (containing 2% AcOH) to the solution, and then freeze-dried.

Hereinafter, NMR data of representative compounds and HPLC charts (320 nm absorption) of compounds synthesized by a similar method are shown.

### Arg-PMAC (Compound 5)

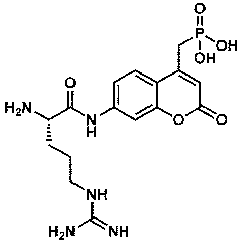

Synthesis was performed using Fmoc-Arg(Pbf)-OH as Fmoc-protected amino acid.

¹H-NMR (400 MHz, D₂O) δ 7.58 (d, 1H, J = 8.0 Hz), 7.41 (s, 1H), 7.21 (d, 1H, J = 7.8 Hz), 6.18 (d, 1H, J = 2.0 Hz), 3.1-2.9 (m, 5H), 1.9 (m, 2H), 1.5 (m, 2H).

HRMS (ESI⁺): calcd. for [M+Na]⁺, 434.1243, Found, 434.1205 (-3.8 mmu).

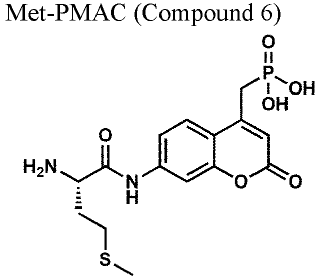

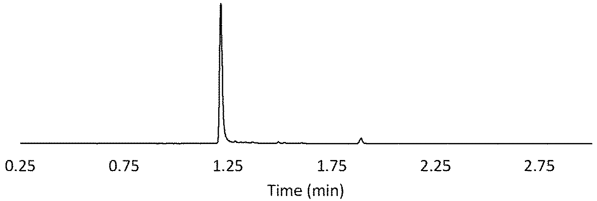

Synthesis was performed using Fmoc-Met-OH as Fmoc-protected amino acid.

Met-PMAC was prepared using Fmoc-Met-OH as the building block.

¹H-NMR (400 MHz, D₂O) δ 7.69 (d, 1H, J = 7.8 Hz), 7.48 (s, 1H), 7.18 (d, 1H, J = 8.0 Hz), 6.1 (d, 1H, J = 2.0 Hz), 3.1-2.9 (m, 3H), 2.51 (d, 2H, J = 7.8 Hz), 2.12 (m, 2H), 1.93 (s, 3H).

HRMS (ESI⁺): calcd. for [M+Na]⁺, 409.0599, Found, 409.0578 (-2.1 mmu).

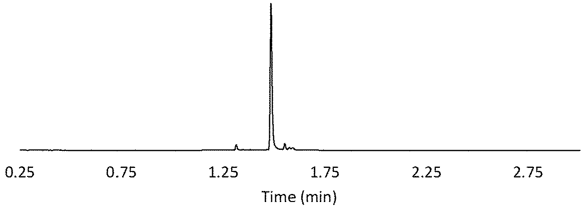

The following compounds were synthesized and purified in the same manner as described above.

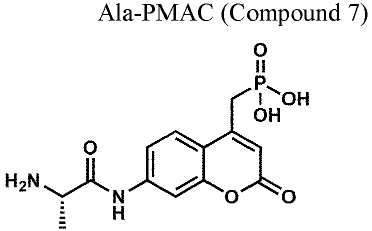

Synthesis was performed using Fmoc-Ala-OH as Fmoc-protected amino acid.

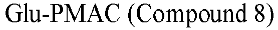

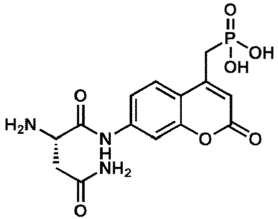

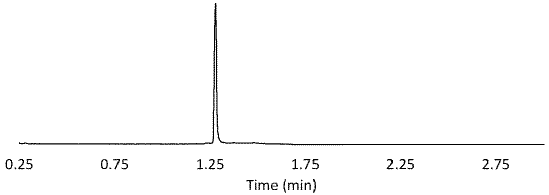

Synthesis was performed using Fmoc-Glu(tBu)-OH as Fmoc-protected amino acid.

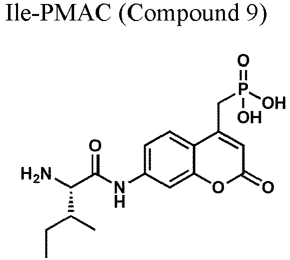

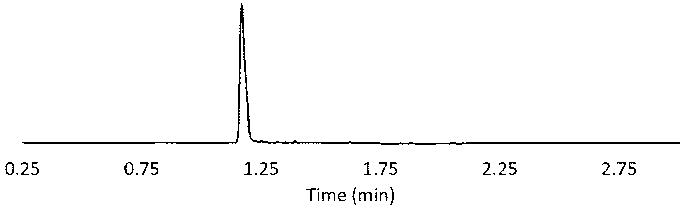

Synthesis was performed using Fmoc-Ile-OH as Fmoc-protected amino acid.

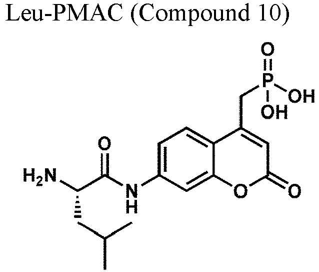

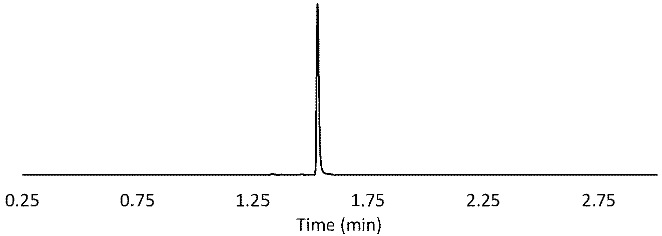

Synthesis was performed using Fmoc-Leu-OH as Fmoc-protected amino acid.

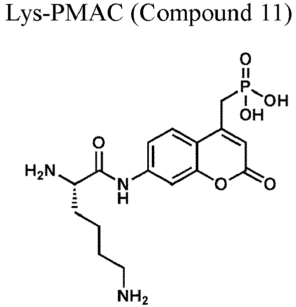

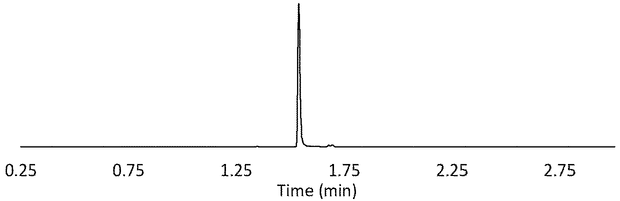

Synthesis was performed using Fmoc-Lys(Boc)-OH as Fmoc-protected amino acid.

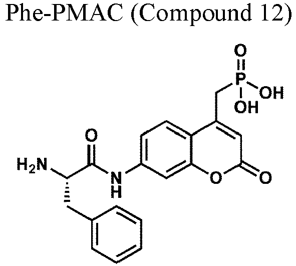

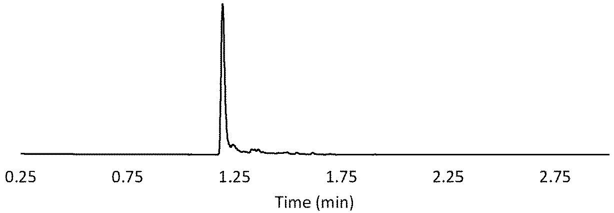

Synthesis was performed using Fmoc-Phe-OH as Fmoc-protected amino acid.

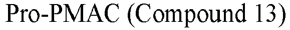

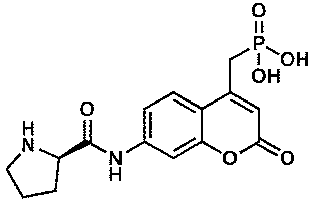

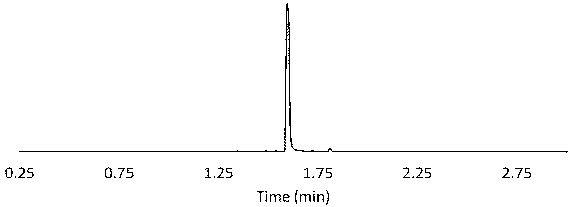

Synthesis was performed using Fmoc-Pro-OH as Fmoc-protected amino acid.

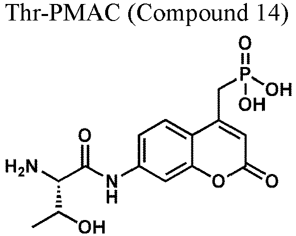

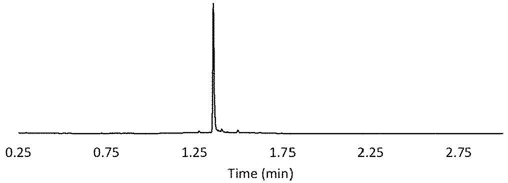

Synthesis was performed using Fmoc-Thr(tBu)-OH as Fmoc-protected amino acid.

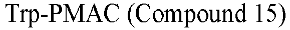

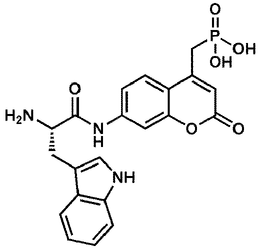

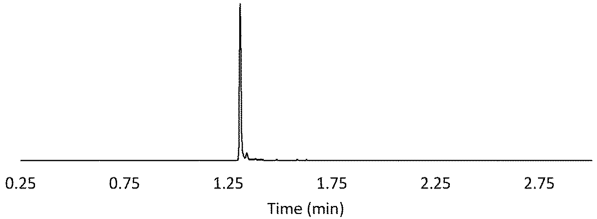

Synthesis was performed using Fmoc-Trp(Boc)-OH as Fmoc-protected amino acid.

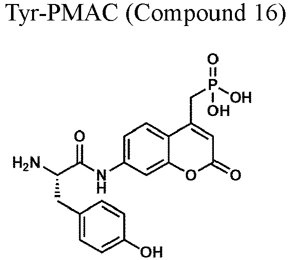

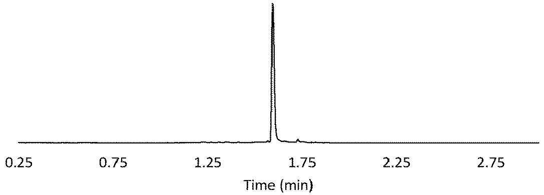

Synthesis was performed using Fmoc-Tyr(tBu)-OH as Fmoc-protected amino acid.

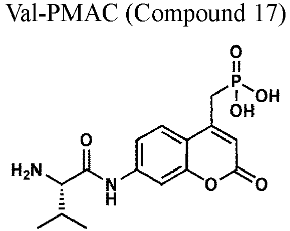

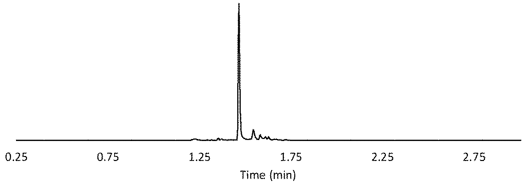

Synthesis was performed using Fmoc-Val-OH as Fmoc-protected amino acid.

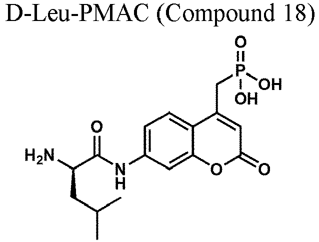

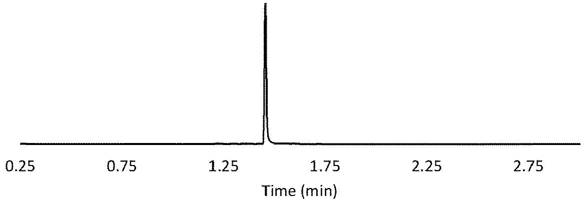

Synthesis was performed using Fmoc-D-Leu-OH as Fmoc-protected amino acid.

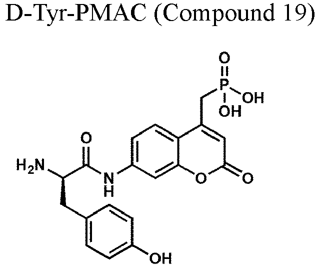

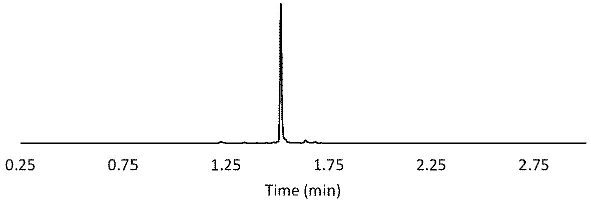

Synthesis was performed using Fmoc-D-Tyr-OH as Fmoc-protected amino acid.

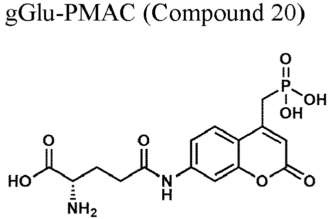

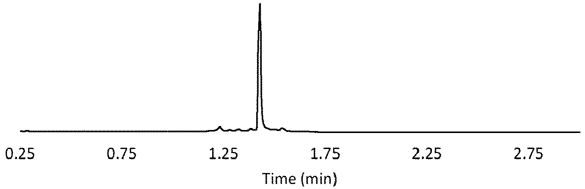

Synthesis was performed using Boc-Glu(OH)-OtBu as Fmoc-protected amino acid.

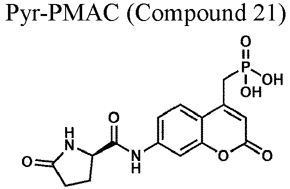

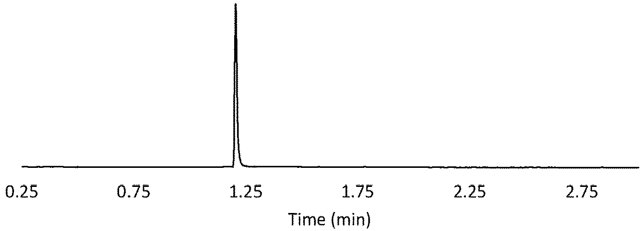

Synthesis was performed using Fmoc-Pyr-OH as Fmoc-protected amino acid.

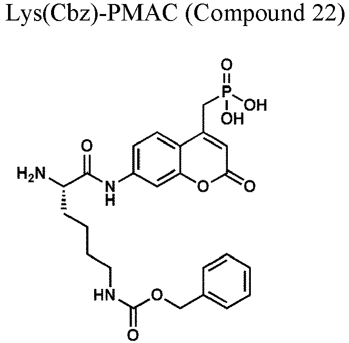

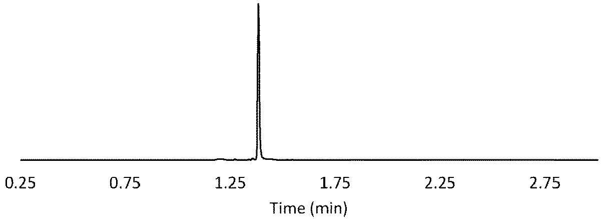

Synthesis was performed using Fmoc-Lys(Cbz)-OH as Fmoc-protected amino acid.

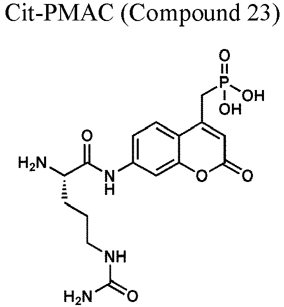

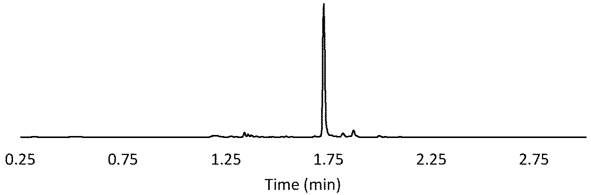

Synthesis was performed using Fmoc-Cit-OH as Fmoc-protected amino acid.

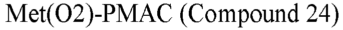

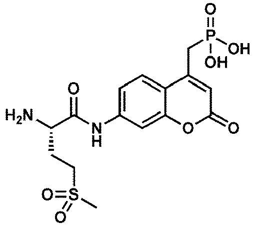

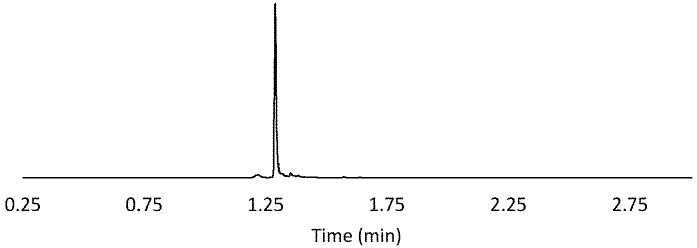

Synthesis was performed using Fmoc-Met(O2)-OH as Fmoc-protected amino acid.

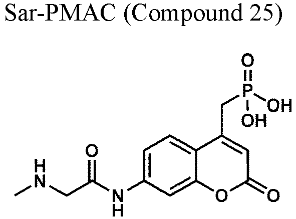

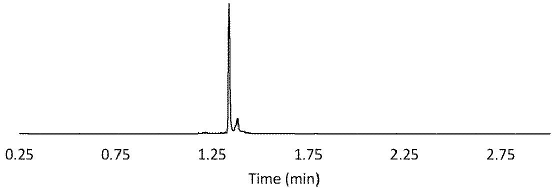

Synthesis was performed using Fmoc-Sar-OH as Fmoc-protected amino acid.

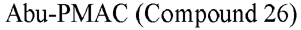

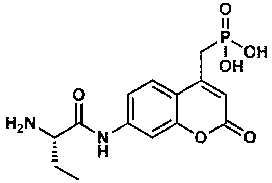

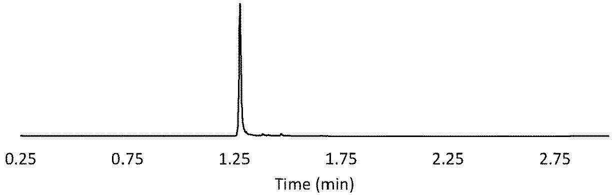

Synthesis was performed using Fmoc-Abu-OH as Fmoc-protected amino acid.

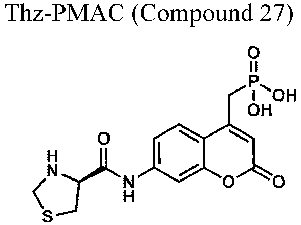

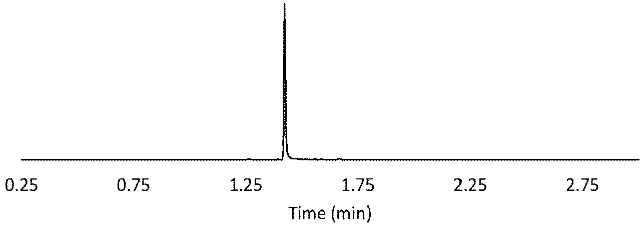

Synthesis was performed using Fmoc-Thz-OH as Fmoc-protected amino acid.

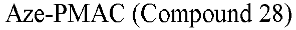

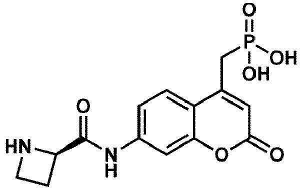

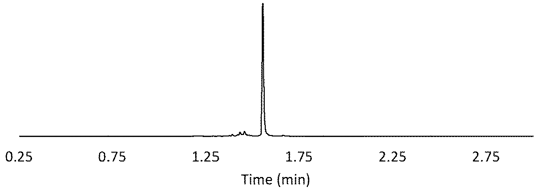

Synthesis was performed using Fmoc-Aze(2)-OH as Fmoc-protected amino acid.

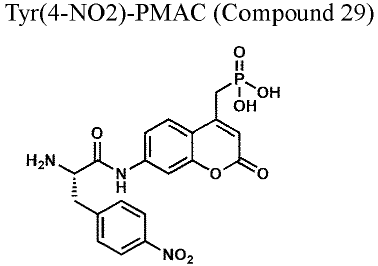

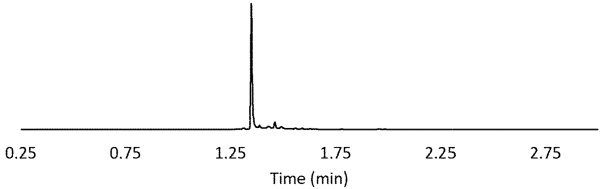

Synthesis was performed using Fmoc-Tyr(4-NO2)-OH as Fmoc-protected amino acid.

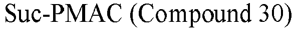

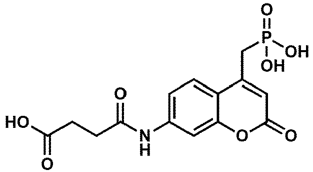

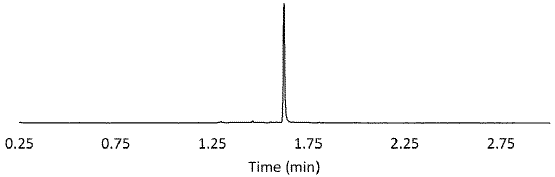

Synthesis was performed using tBuO-Suc-OH as tBuO-protected amino acid.

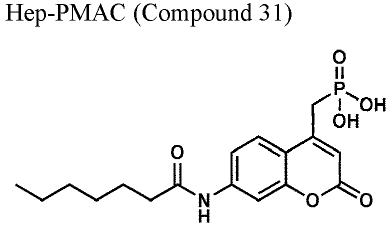

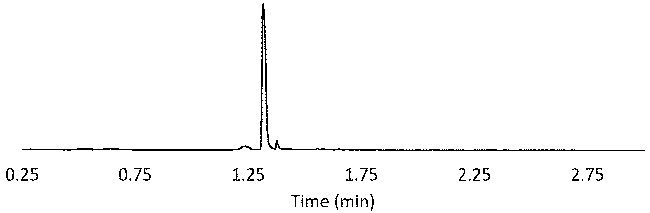

Synthesis was performed using heptanoic acid.

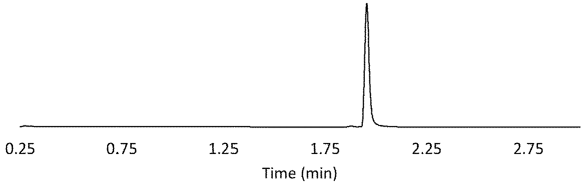

### [Synthesis Example 5]

### Synthesis Example of peptidase and Protease Probes

Peptidase and protease probes were synthesized by the following procedures according to the following reaction scheme using the compound (Compound 2) in which phosphonic acid of NH₂ type fluorescent mother nucleus was protected. 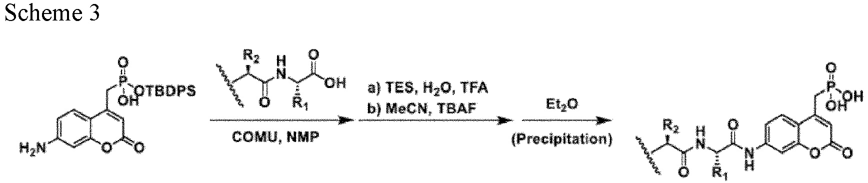 R₁ and R₂: Side chain of any amino acid

1. PMAC-TBDPS (5 µmol), COMU (30 µmol), and a peptide (30 µmol) were mixed in a 1.5 mL test tube and dissolved in 30 µL of NMP.
2. The reaction mixture was stirred at 60°C.
3. a) 300 µL of TFA, 5 µL of triethylsilane, and 5 µL of H₂O were added and the reaction mixture was stirred.
   b) (when an acid-weak protective group was not desired to be deprotected) 300 µL of MeCN and 5 µL of 1 M TBAF in THF were added and the reaction mixture was stirred.
4. 1 mL of Et₂O was added and the mixture was centrifuged (15,000 rpm × 10 min, 4°C) to remove the supernatant.
5. The residue was dissolved in a 1 mL load buffer solution (80% Bis Tris buffer solution (1 M, pH 6.8) and 20% MeCN) and supported on 500 µL phos-tag beads.
6. The beads were washed five times with 1 mL of wash buffer solution (80% H₂O and 20% MeCN, pH 6.8, containing 100 mM Bis Tris-AcOH).
7. The beads were washed three times with 1 mL of washing solution (80% H₂O and 20% MeCN).
8. The compound was eluted three times with 300 µL of an eluent (80% H₂O and 20% MeCN, containing 1% ethylenediamine), neutralized by adding 500 µL of H₂O (containing 2% AcOH) to the solution, and then freeze-dried.

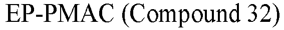

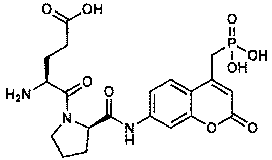

Synthesis was performed using Fmoc-Glu(tBu)-Pro-OH as the peptide.

¹H-NMR (400 MHz, D₂O) δ 7.60 (d, 1H, J = 7.2 Hz), 7.44 (s, 1H), 7.18 (d, 1H, J = 7.2 Hz), 6.15 (d, 1H, J = 2.1 Hz), 4.29 (m, 1H), 3.6-3.5 (m, 2H), 3.06 (d, 2H, J = 20.4 Hz), 2.96 (m, 1H), 2.43 (t, 2H, J = 8.0 Hz), 2.24 (m, 1H), 2.1-1.7 (m, 5H).

HRMS (ESI⁺): calcd. for [M+H]⁺, 482.1328, Found, 482.1336 (+0.8 mmu).

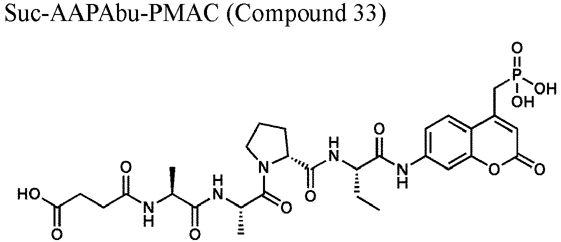

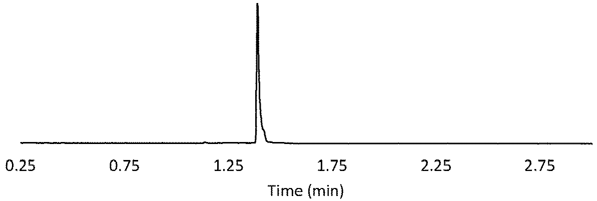

Synthesis was performed using tBuOSuc-AAPAbu-OH as the peptide.

¹H-NMR (400 MHz, D₂O) δ 7.68 (d, 1H, J = 8.4 Hz), 7.49 (s, 1H), 7.24 (d, 1H, J = 8.4 Hz), 6.20 (d, 1H, J = 2.0 Hz), 4.39 (m, 1H), 4.27 (m, 1H), 4.11 (m, 2H), 3.6-3.4 (m, 2H), 3.02 (d, 2H, J = 20.8 Hz), 2.4-2.3 (m, 5H), 2.1 (m, 1H), 1.8 (m, 2H), 1.7 (m, 2H), 1.2 (m, 6H), 0.8 (t, 3H, J = 8.4 Hz).

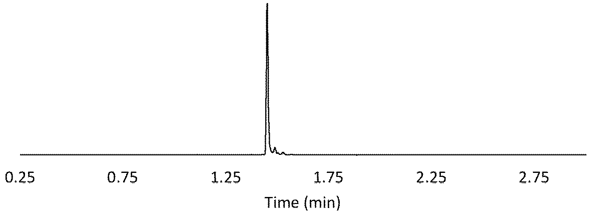

The following compounds were synthesized and purified in the same manner as described above.

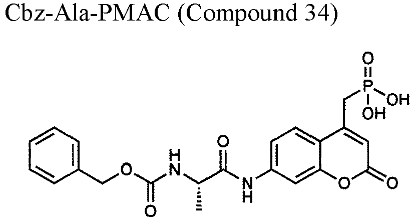

Synthesis was performed using Cbz-Ala-OH.

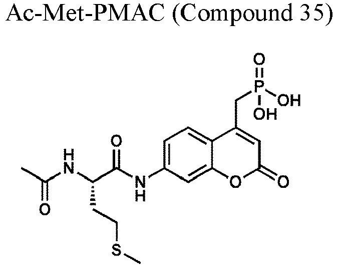

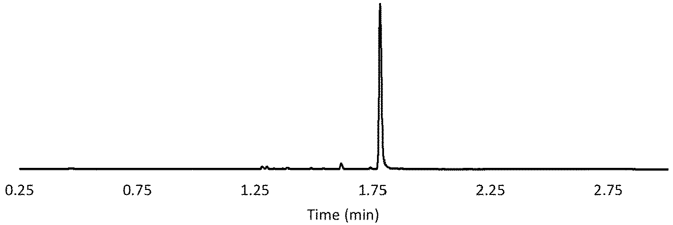

Synthesis was performed using Ac-Met-OH.

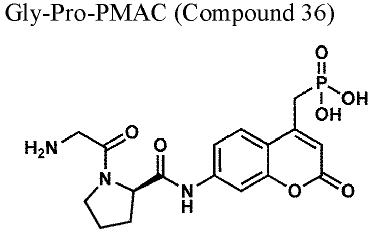

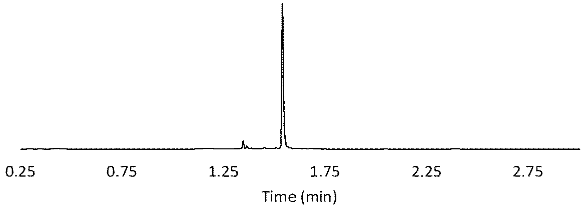

Synthesis was performed using Fmoc-Gly-Pro-OH as the peptide.

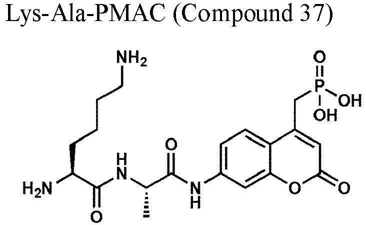

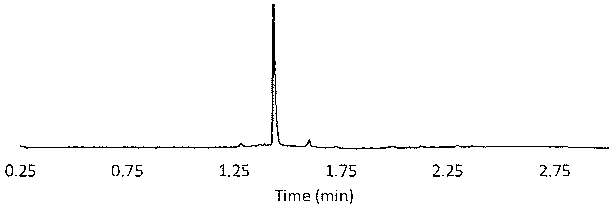

Synthesis was performed using Fmoc-Lys(Boc)-Ala-OH as the peptide.

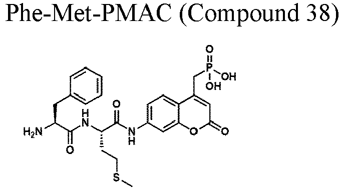

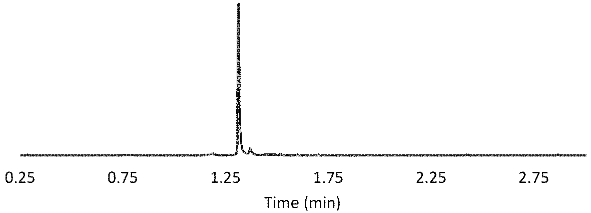

Synthesis was performed using Fmoc-Phe-Met-OH as the peptide.

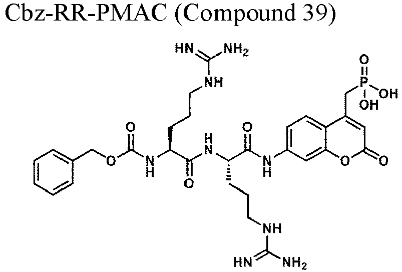

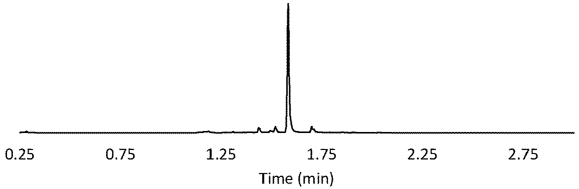

Synthesis was performed using Cbz-Arg(Pbf)-Arg(Pbf)-OH as the peptide.

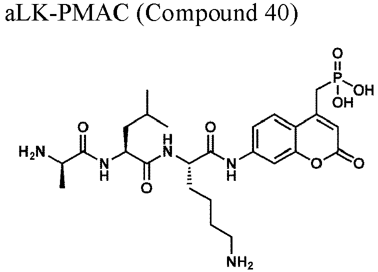

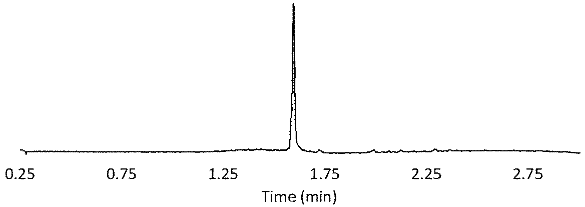

Synthesis was performed using Cbz-D-Ala-Leu-Lys(Boc)-OH as the peptide.

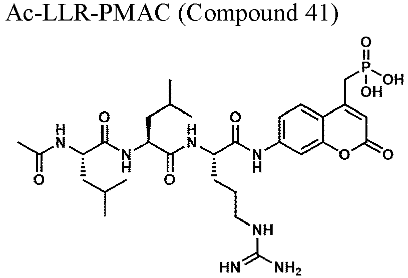

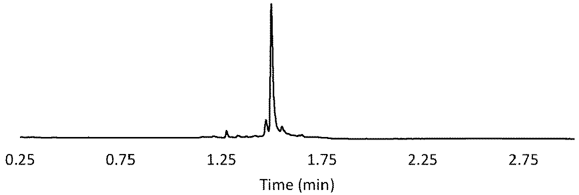

Synthesis was performed using Ac-Leu-Leu-Arg-OH as the peptide.

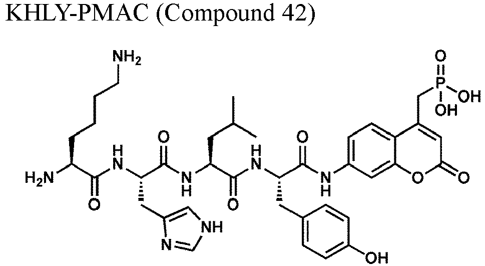

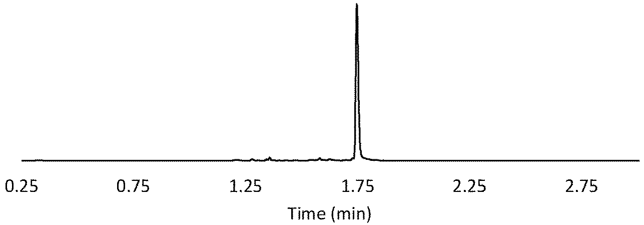

Synthesis was performed using Fmoc-Lys(Boc)-His(Trt)-Leu-Tyr(tBu)-OH as the peptide.

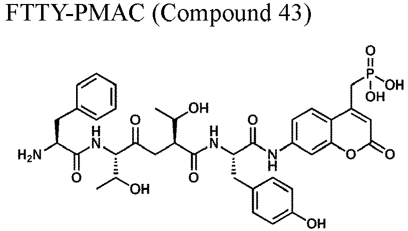

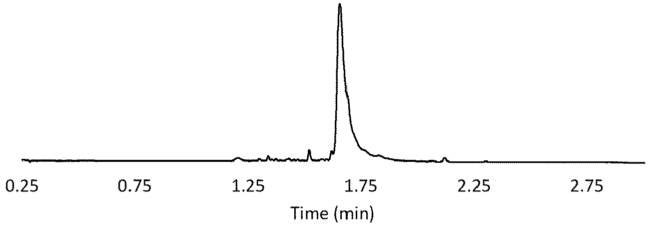

Synthesis was performed using Fmoc-Phe-Thr(tBu)-Thr(tBu)-Tyr(tBu)-OH as the peptide.

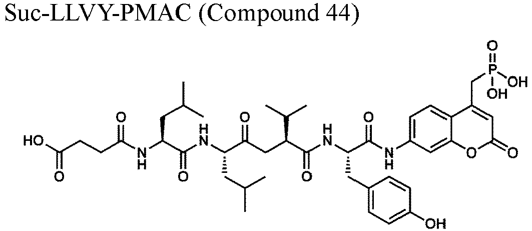

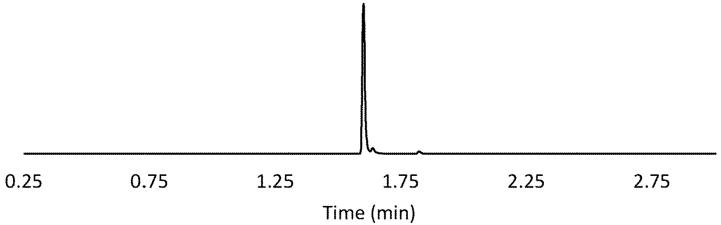

Synthesis was performed using tBuO-Suc-Leu-Leu-Val-Tyr(tBu)-OH as the peptide.

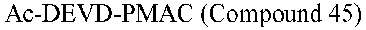

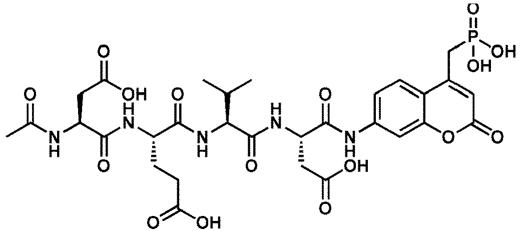

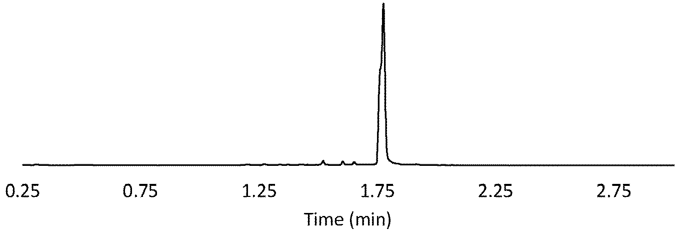

Synthesis was performed using Ac-Asp(tBu)-Glu(tBu)-Val-Asp(tBu)-OH as the peptide.

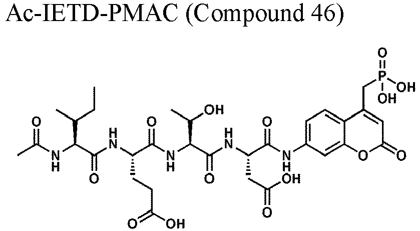

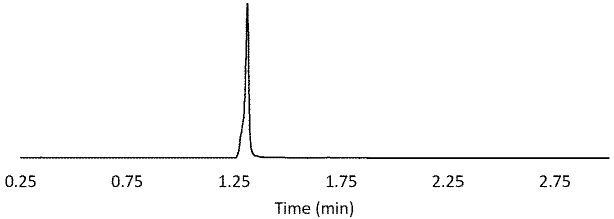

Synthesis was performed using Ac-Ile-Glu(tBu)-Thr(tBu)-Asp(tBu)-OH as the peptide.

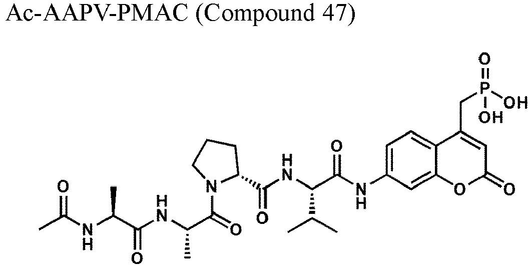

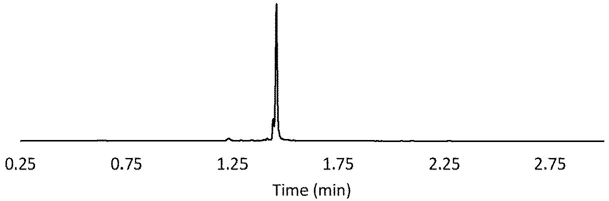

Synthesis was performed using Ac-Ala-Ala-Pro-Val-OH as the peptide.

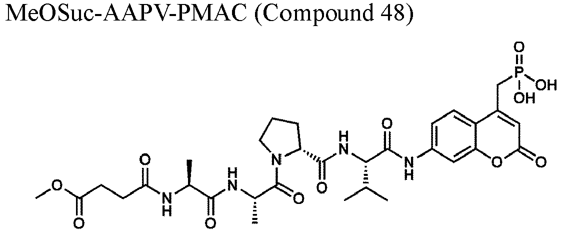

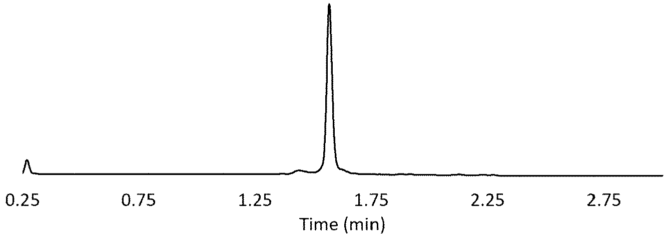

Synthesis was performed using MeOSuc-Ala-Ala-Pro-Val-OH as the peptide.

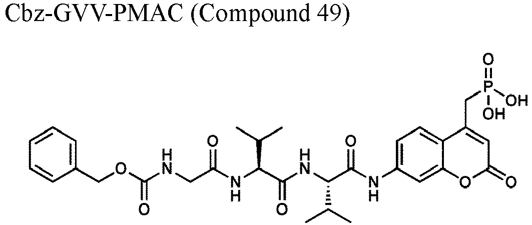

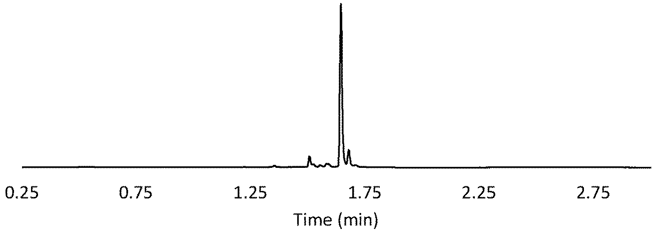

Synthesis was performed using Cbz-Gly-Val-Val-OH as the peptide.

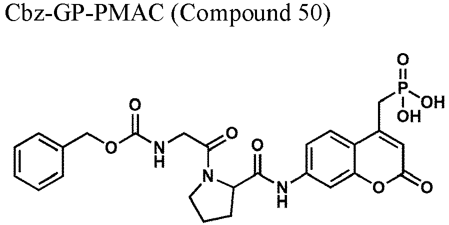

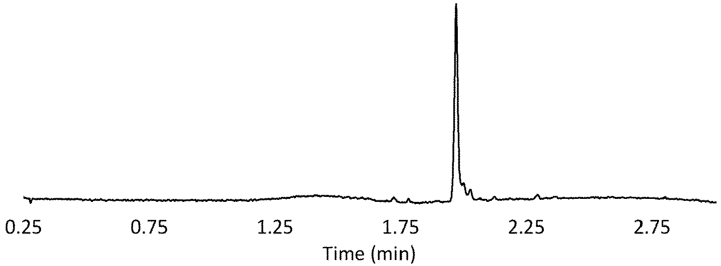

Synthesis was performed using Cbz-Gly-Pro-OH as the peptide.

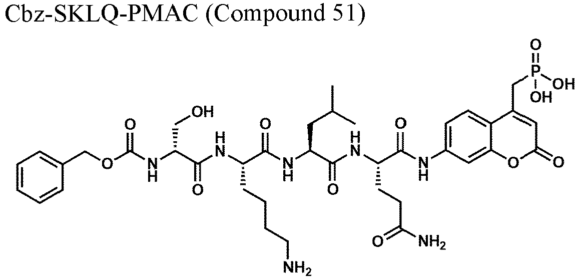

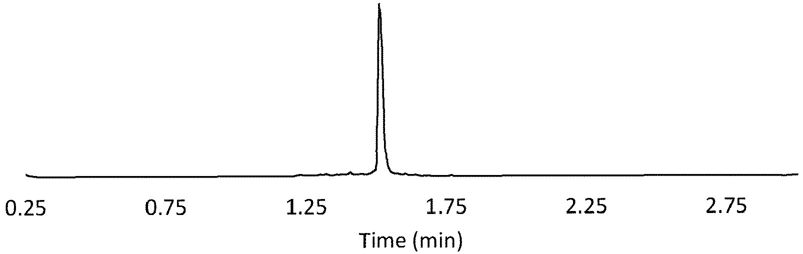

Synthesis was performed using Cbz-Ser(tBu)-Lys(Boc)-Leu-Gln(Trt)-OH as the peptide.

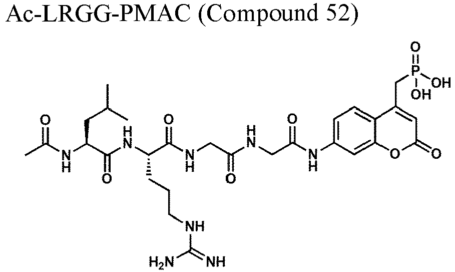

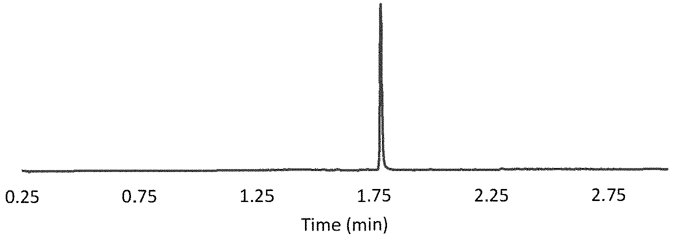

Synthesis was performed using Ac-Leu-Arg(Pbf)-Gly-Gly-OH as the peptide.

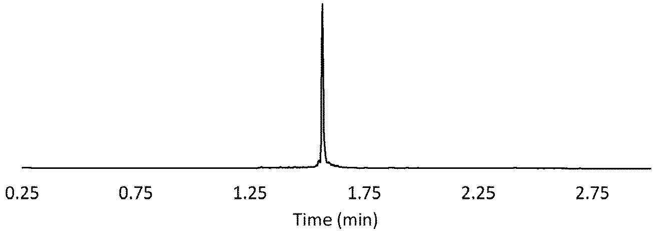

### [Synthesis Example 6]

### Synthesis Example of Glycosidase Probe

A glycosidase probe was synthesized by the following procedures according to the following reaction scheme using the compound (Compound 4) in which phosphonic acid of OH type fluorescent mother nucleus was protected. 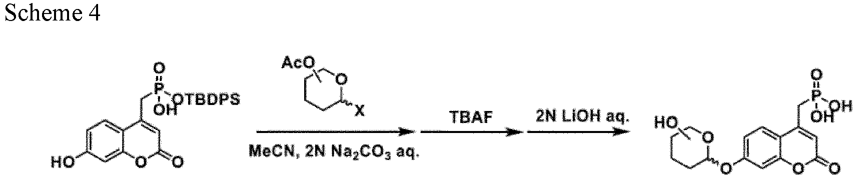
1. In a test tube, PMAC-TBDPS (5 µmol) and acetylglucopyranosyl chloride (25 µmol) were dissolved in 250 µL of MeCN and 500 µL of a 2 N Na₂CO₃ aqueous solution.
2. The reaction mixture was stirred at room temperature.
3. The MeCN phase was collected.
4. 150 µL of 1 M TBAF in THF was added and stirred.
5. 100 µL of a 2 N LiOH aqueous solution was added and stirred for 10 minutes.
6. A 400 µL load buffer solution (80% H₂O and 20% MeCN, pH 6.8, containing 100 mM Bis Tris-AcOH) and 11.5 µL of AcOH were added for neutralization and the mixture was washed with 100 µL of AcOEt.
7. The residue was dissolved in a 1 mL load buffer solution (80 % Bis Tris buffer solution (1 M, pH 6.8) and 20% MeCN) and supported on 500 µL phos-tag beads.
8. The beads were washed five times with 1 mL of wash buffer solution (80% H₂O and 20% MeCN, pH 6.8, containing 100 mM Bis Tris-AcOH).
9. The beads were washed three times with 1 mL of washing solution (80% H₂O and 20% MeCN).
10. The compound was eluted three times with 300 µL of an eluent (80% H₂O and 20% MeCN, containing 1% ethylenediamine), neutralized by adding 500 µL of H₂O (containing 2% AcOH) to the solution, and then freeze-dried.

### 3. Evaluation of Fluorescent Probe

The results of an assay of enzyme activity in blood using a microdevice for the fluorescent probe of the compound synthesized using the procedures shown in Synthesis Examples 3 to 6 are shown below.

### [Example 1]

Results of Enzyme Activity Detection in Blood Using Compounds 5 to 51

### Assay Protocol

1. Compounds 5 to 51, which are fluorescent probes for activity detection of an aminopeptidase, an amidase, and a protease were diluted with an assay buffer so that the concentration of each compound was 100 µM. The composition of the assay buffer is 100 mM HEPES-NaOH (pH 7.4), 1 mM MgCl₂, 1 mM DTT, 3 mM TritonX-100.
2. Next, a human plasma sample (derived from a healthy subject or a patient with pancreatic cancer) was diluted 500 times with the above assay buffer, and mixed in an equal amount with the diluted solution of the probe. At this time, the final concentrations are 50 µM for the fluorescent probe and 1000 fold dilution for the human plasma sample.
3. The mixed solution was then added to a microdevice, followed by the addition of sealing oil to enclose the solution in each well.
4. The microdevice in which the solution was enclosed was incubated at 37°C for 4 hours, and then the fluorescence intensity of each well was measured with a fluorescence microscope. In the wells in which the enzyme reacting with the fluorescent probe was enclosed, an increase in fluorescence intensity was observed.

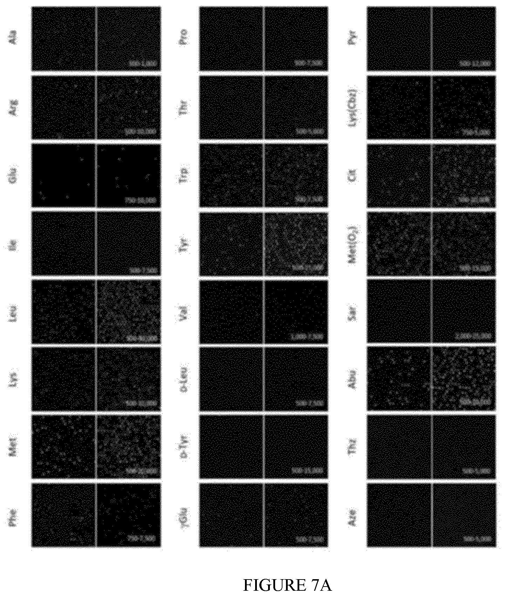

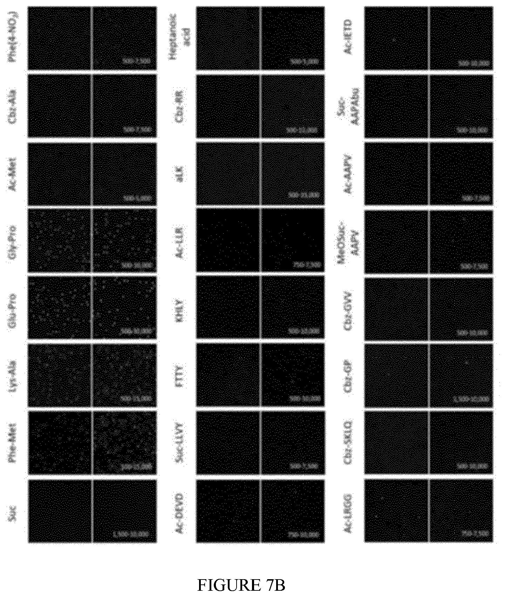

Fluorescence microscopic images obtained by performing the above assay for the fluorescent probes of Compounds 5 to 51 are shown in Figs. 7A and 7B.

In Figs. 7A and 7B, the left image (labeled as "Healthy") of each fluorescence microscopic image shows the result obtained when a human plasma sample derived from a healthy subject is used, and the right image (labeled as "Tumor") shows the result obtained when a human plasma sample derived from a patient with pancreatic cancer is used. The types of amino acid residues or peptides of each fluorescent probe were described on the left side of each fluorescence microscopic image.

As described above, the presence of phosphonic acid did not negatively affect the fluorescence intensity of the fluorescent probe. From this point, the synthesis scheme of the present invention may be useful in various fluorescent probes.

### [Example 2]

### Measurement of Human Plasma Sample Using CD13 Probe in Microdevice

In this example, an experiment for verifying the applicability of Arg-PMAC and Met-PMAC, which are CD13 activity detection probes, to biomarker detection was performed by the following procedures.

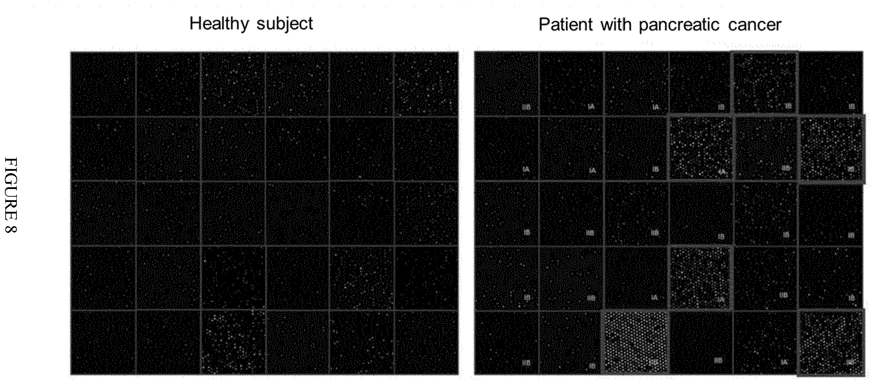

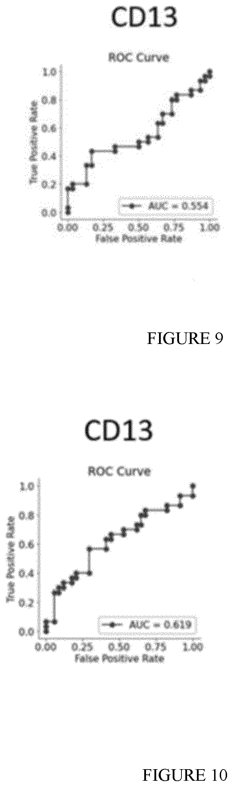

1. Arg-PMAC as a CD13 probe was diluted with an assay buffer to 100 µM. The composition of the buffer is 100 mM HEPES-NaOH (pH 7.4), 1 mM MgCl₂, 1 mM DTT, 3 mM TritonX-100.
2. Next, human plasma samples collected from 30 patients with pancreatic cancer and 30 healthy subjects were diluted 500 times with the above assay buffer, and mixed in an equal amount with the diluted solution of the CD 13 probe. At this time, the final concentrations are 50 µM for the CD13 probe and 1000 fold dilution for the human plasma sample.
3. The mixed solution was then added to a microdevice, followed by the addition of sealing oil to enclose the solution in each well.
4. The microdevice in which the solution was enclosed was incubated at 37°C for 4 hours, and then the fluorescence intensity of each well was measured with a fluorescence microscope. Fig. 8 shows a fluorescence microscopic image obtained using Arg-PMAC.
5. An ROC curve was created for the number of wells in which the detected fluorescence intensity is equal to or higher than Average + 3SD of background fluorescence separately measured, that is, the number of wells in which CD13 is considered to be enclosed, and as a result, the number of AUC = 0.554 was obtained as shown in Fig. 9.
6. An ROC curve was created by performing the processes of 1 to 5 using Met-PMAC, and as a result, the number of AUC = 0.619 was obtained as shown in Fig. 10.

As described above, by measuring the fluorescence intensity of human plasma samples using Arg-PMAC and Met-PMAC, which are CD13 probes, with a microdevice, a significant difference was observed in a patient with pancreatic cancer as compared with a healthy subject. From this result, it is possible to find CD13 specifically found in pancreatic cancer by comparing the enzyme activity of CD 13 between biological samples derived from a healthy subject and a subject having a disease using these fluorescent probes.

### [Example 3]

### Measurement of Human Plasma Sample Using DPP4 Probe in Microdevice

In this example, an experiment for verifying the applicability of Glu-Pro-PMAC, which is a DPP4 activity detection probes, to biomarker detection was performed by the following procedures.

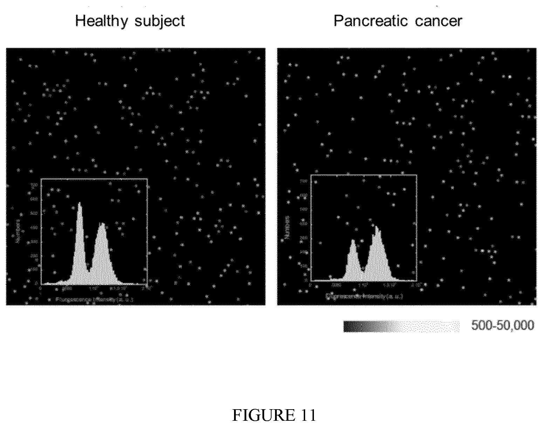

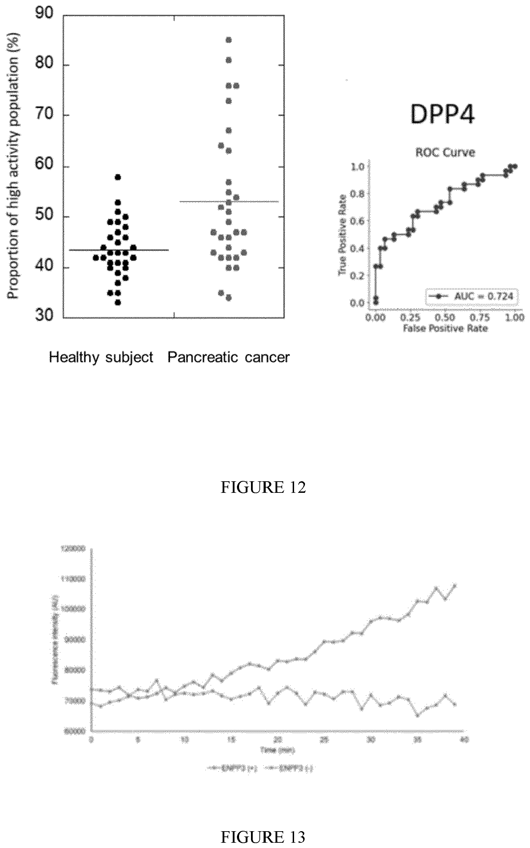

1. Glu-Pro-PMAC as a DPP4 probe was diluted with an assay buffer to 100 µM. The composition of the buffer is 100 mM HEPES-NaOH (pH 7.4), 1 mM MgCl₂, 1 mM DTT, 3 mM TritonX-100.
2. Next, human plasma samples collected from 30 patients with pancreatic cancer and 30 healthy subjects were diluted 500 times with the above assay buffer, and mixed in an equal amount with the diluted solution of the DPP4 probe. At this time, the final concentrations are 50 µM for the DPP4 probe and 1000 fold dilution for the human plasma sample.
3. The mixed solution was then added to a microdevice, followed by the addition of sealing oil to enclose the solution in each well.
4. The microdevice in which the solution was enclosed was incubated at 25°C for 2 hours, and then the fluorescence intensity of each well was measured with a fluorescence microscope. Fig. 11 shows a representative fluorescence microscopic image.
5. As for the number of wells in which the detected fluorescence intensity is equal to or higher than Average + 3SD of background fluorescence separately measured, that is, DPP4 is considered to be enclosed, two peaks were observed, and the ratio of the number of wells belonging to these peaks was obtained to create an ROC curve, and as a result, the number of AUC = 0.724 was obtained as shown in Fig. 12.

As described above, by measuring the fluorescence intensity of human plasma samples using Glu-Pro-PMAC, which is a DPP4 probe, with a microdevice, a significant difference was observed in a patient with pancreatic cancer as compared with a healthy subject. From this result, it is possible to find activity abnormality of DPP4 specifically found in pancreatic cancer by comparing the enzyme activity of DPP4 between biological samples derived from a healthy subject and a subject having a disease using these fluorescent probes.

### [Synthesis Example 6]

### Synthesis of PMUM-dCMP

PMUM-dCMP (Compound 53) was synthesized by the following procedures using the compound (Compound 4) in which phosphonic acid of OH type fluorescent mother nucleus was protected.

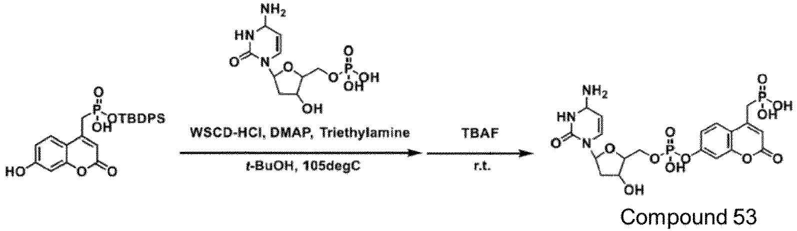

1. PMUM-TBDPS (5.1 µmol), 2'-deoxythidine-5'-monophosphate (10 µmol), WSCDHC1 (20 µmol), DMAP (0.6 µmol), and triethylamine (2.5 µmol) were mixed in a 1.5 mL test tube and dissolved in 30 µL of t-BuOH.
2. The reaction solution was stirred at 105°C.
3. 3 mL of TBAF (1 M, THF solution) was added and stirred at room temperature.
4. The solution was subjected to crude purification by MPLC (eluent: A/B = 100/0 > 0/100, A: 0.1% TFA-containing 100% H₂O, B: 0.1% TFA-containing 100% MeCN).
5. The eluent was evaporated to distill off the solvent and the residue was dissolved in a 1 mL load buffer solution (80% H₂O and 20% MeCN, pH 6.8, containing 100 mM bis tris-AcOH) and supported on 500 µL phos-tag beads.
6. The beads were washed five times with 1 mL of wash buffer solution (80% H₂O and 20% MeCN, pH 6.8, containing 100 mM bis tris-AcOH), and washed twice with a 1 mL rinse solution (80% H₂O and 20% MeCN).
7. The compound was eluted three times with 300 µL of an eluent (80% H₂O and 20% MeCN, containing 1% ethylenediamine) and then the solution was freeze-dried.

### [Example 4]

### Evaluation of Activity of PMLTM-dCMP with respect to ENPP

The activity measurement of PMUM-dCMP obtained in Synthesis Example 7 was performed using a purified enzyme of ENPP3 on a plate reader.

The assay was performed with a Tris-HCl buffer (pH 9.3) containing 1 mM MgCl₂, 0.5% w/w CHAPS, 10 µM PMUM-dCMP, and ENPP3 (recombinant, 9.9 µg/mL). The results are shown in Fig. 13.

As shown in Fig. 13, in the case of adding ENPP3, a remarkable increase in fluorescence intensity (FI) was observed as compared with the case of adding no ENPP3.

### [Example 5]

### Study of Biomarker Activity Detection Fluorescent Probe in Pancreatic Cancer

Next, in order to search for a biomarker activity detection fluorescent probe of pancreatic cancer, plasma samples derived from three healthy subjects and three patients with pancreatic cancer were used, and enzyme assays in a microdevice were performed using a plurality of compounds similar to Formula (III) including PMUM-dCMP.

The protocol of the enzyme assay is as follows.
1. A compound analogous to Formula (III) was diluted with an assay buffer. The composition of the assay buffer is 100 mM HEPES-NaOH (pH 5 to 10), 1 mM MgCl₂, 3 mM TritonX-100.
2. Next, a human plasma sample was diluted with the above assay buffer, and mixed in an equal amount with the diluted solution of the probe. At this time, the final concentrations are 10 to 100 µM for the probe and 500 to 1000 fold dilution for the human plasma sample.
3. The mixed solution was then added to a microdevice, followed by the addition of sealing oil to enclose the solution in each well.
4. The microdevice in which the solution was enclosed was incubated at 37°C for 1 to 12 hours, and then the fluorescence intensity of each well was measured with a fluorescence microscope.
5. Image analysis was performed to found that there were compounds having different enzyme activities depending on the presence or absence of a disease.

### [Example 6]

### Measurement of Human Plasma Sample Using ENPP Probe in Microdevice

In this example, an experiment for verifying the applicability of PMUM-dCMP to biomarker detection was performed by the following procedures.

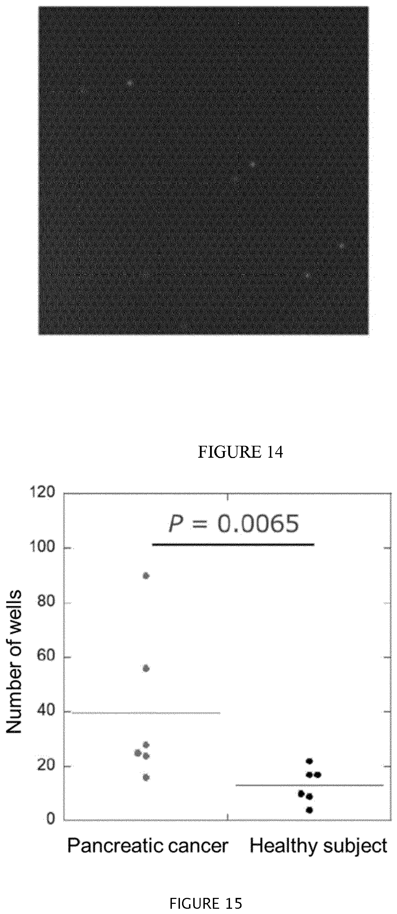

1. PMLTM-dCMP as an ENPP probe was diluted with an assay buffer to 200 µM. The composition of the assay buffer is 100 mM Tris-HCl (pH 9.3), 1 mM MgCl₂, 0.5% (w/v) CHAPS.
2. Next, human plasma samples collected from six patients with pancreatic cancer and six healthy subjects were diluted 250 times with the above assay buffer, and mixed in an equal amount with the diluted solution of the ENPP probe. At this time, the final concentrations are 100 µM for the ENPP probe and 500 fold dilution for the human plasma sample.
3. The mixed solution was then added to a microdevice, followed by the addition of sealing oil to enclose the solution in each well.
4. The microdevice in which the solution was enclosed was incubated at 25°C for 40 minutes, and then the fluorescence intensity of each well was measured with a fluorescence microscope. Fig. 14 shows a fluorescence microscopic image.
5. As for the number of wells in which the detected fluorescence intensity is equal to or higher than 2500 AU, that is, ENPP3 is considered to be enclosed, a significant increase in the number of wells was observed in the patients with pancreatic cancer as compared with the healthy subjects. The results thereof are shown in Fig. 15.

As described above, by measuring the fluorescence intensity of human plasma samples using PMUM-dCMP, which is an ENPP probe, with a microdevice, a significant difference was observed in a patient with pancreatic cancer as compared with a healthy subject. From this result, it is possible to find ENPP3 specifically found in pancreatic cancer by comparing the enzyme activity of ENPP between biological samples derived from a healthy subject and a subject having a disease using PMUM-dCMP.

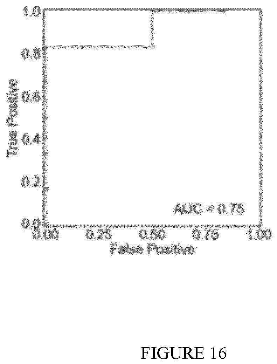

Specifically, in this example, an ROC curve for determination based on the enzyme activity of ENPP determined based on the number of wells with 2500 AU or more is created, and when a threshold is subtracted at the time when the number of wells with 2500 AU or more is 23, the sensitivity is 83% and the specificity is 100% (Fig. 16). It is possible to determine pancreatic cancer by such an ROC curve.

## Claims

1. A compound represented by General Formula (I) below: 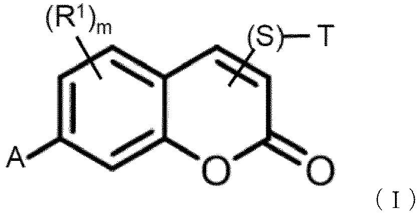 wherein
A is an amino group (-NR²H) or a hydroxyl group (-OH),
where R² is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO);
R¹, when present, is the same or different monovalent substituent present on a benzene ring;
S, when present, is a linker;
T is selected from a phosphonic acid group (-P(=O)(OH)₂), a phosphoric acid ester group (-O-P(=O)(OH)₂), or a phosphoric amide group (-NH-P(=O)(OH)₂); and
m is an integer of 0 to 3.

2. The compound according to claim 1, wherein A is -NR²H.

3. The compound according to claim 1, wherein A is -OH.

4. A method for preparing a compound represented by General Formula (III) below: 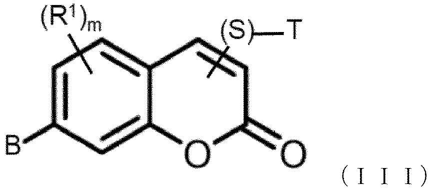 wherein
B is selected from an amide group (-NR²C(=O)R, R is a hydrogen atom or a substituted or unsubstituted alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), an ester group, a phosphoric acid ester group, a sulfuric acid ester group, an ether group, or -O-L' (L' represents a saccharide or a partial structure of a saccharide); and
S, T, R¹, and m are as defined in Formula (I),
the method comprising the steps of:
(1) protecting a T group of a compound represented by General Formula (I) below: 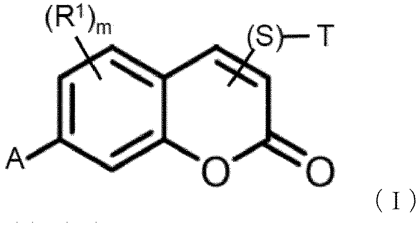 wherein,
A is an amino group (-NR²H) or a hydroxyl group (-OH),
where, R² is a hydrogen atom or a substituted or unsubstituted alkyl group having 1 to 8 carbon atoms;
R¹, when present, is the same or different monovalent substituent present on a benzene ring;
S, when present, is a linker;
T is selected from a phosphonic acid group (-P(=O)(OH)₂), a phosphoric acid ester group (-O-P(=O)(OH)₂), or a phosphoric amide group (-NH-P(=O)(OH)₂); and
m is an integer of 0 to 3;
(2) (i) when A is an amino group (-NR²H), converting the amino group of a product obtained in the step (1) into an amide group (-NR²C(=O)R, R is a hydrogen atom or an alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or
(ii) when A is a hydroxyl group, converting the hydroxyl group of a product obtained in the step (1) into an ester group, a phosphoric acid ester group, a sulfuric acid ester group, an ether group, or -O-L' (L' represents a saccharide or a partial structure of a saccharide);
(3) removing a protective group of T of a product obtained in the step (2), which may optionally include a crude purification step after the removal of the protective group;
(4) adding a compound represented by Formula (II) below to a product obtained in the step (3): 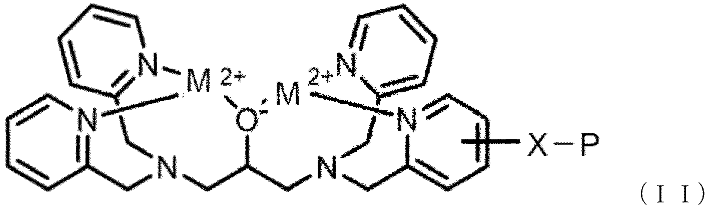 wherein,
M is Zn or Cu;
X is a linker group; and
P is a carrier; and
(5) purifying a product obtained in the step (4) and then liquating or eluting a compound of Formula (III).

5. A method for preparing one kind of compound represented by General Formula (III) below for each vessel of a plurality of reaction vessels by performing the following steps (1) to (5) in parallel in the plurality of reaction vessels:
(1) protecting a T group of a compound represented by Formula (I) below: 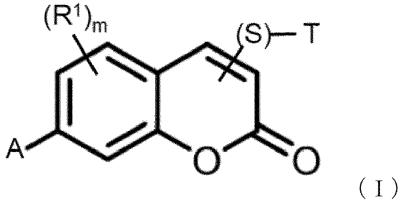 wherein,
A is an amino group (-NR²H) or a hydroxyl group (-OH),
where, R² is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO);
R¹, when present, is the same or different monovalent substituent present on a benzene ring;
S, when present, is a linker;
T is selected from a phosphonic acid group (-P(=O)(OH)₂), a phosphoric acid ester group (-O-P(=O)(OH)₂), or a phosphoric amide group (-NH-P(=O)(OH)₂); and
m is an integer of 0 to 3;
(2) (i) when A is an amino group (-NR²H), converting the amino group of a product obtained in the step (1) into an amide group (-NR²C(=O)R, R is a hydrogen atom or an alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or
(ii) when A is a hydroxyl group, converting the hydroxyl group of a product obtained in the step (1) into an ester group, a phosphoric acid ester group, a sulfuric acid ester group, an ether group, or -O-L' (L' represents a saccharide or a partial structure of a saccharide);
(3) removing a protective group of T of a product obtained in the step (2), which may optionally include a crude purification step after the removal of the protective group;
(4) adding a compound represented by Formula (II) below to a product obtained in the step (3): 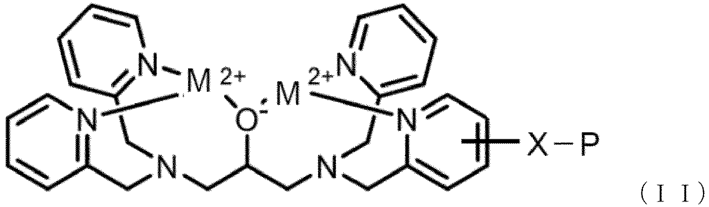 wherein,
M is Zn or Cu;
X is a linker group; and
P is a carrier; and
(5) purifying a product obtained in the step (4) and then liquating or eluting a compound of Formula (III): 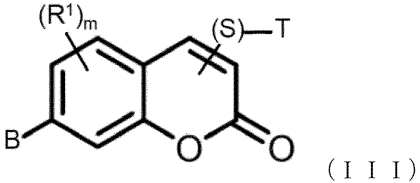 wherein,
B is selected from an amide group (-NR²C(=O)R, R is a hydrogen atom or a substituted or unsubstituted alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), or a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), an ester group, a phosphoric acid ester group, a sulfuric acid ester group, an ether group, or -O-L' (L' represents a saccharide or a partial structure of a saccharide); and
S, T, R¹, and m are as defined in Formula (I).

6. A compound represented by General Formula (III) below or a salt thereof: 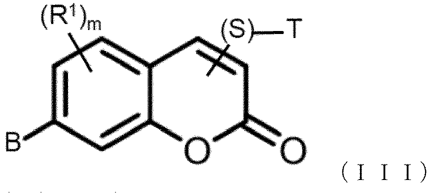 wherein,
B is selected from an amide group (-NR²C(=O)R, R is a hydrogen atom or a substituted or unsubstituted alkyl group having 1 to 8 carbon atoms), -NR²-CO-L (L represents a partial structure of an amino acid), a phosphoric amide group (-NR²-PO(ORₐ)(OR_{b}), Rₐ and R_{b} are each independently selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), a sulfonamide group (-NR²-SO₂-R_{c}, R_{c} is selected from the group consisting of a hydrogen atom and a branched, linear or cyclic, substituted or unsubstituted alkyl group having 1 to 8 carbon atoms (one or more non-adjacent and non-terminal C atoms of the alkyl group may be replaced with O, S, CO, or COO)), an ester group, a phosphoric acid ester group, a sulfuric acid ester group, an ether group, or -O-L'(L' represents a saccharide or a partial structure of a saccharide);
R¹, when present, is the same or different monovalent substituent present on a benzene ring;
S, when present, is a linker;
T is selected from a phosphonic acid group (-P(=O)(OH)₂), a phosphoric acid ester group (-O-P(=O)(OH)₂), or a phosphoric amide group (-NH-P(=O)(OH)₂); and
m is an integer of 0 to 3.

7. A fluorescent probe for detecting enzyme activity, comprising the compound represented by General Formula (III) or the salt thereof according to claim 6.

8. A method for detecting activity of a plurality of enzymes in a biological sample, the method comprising bringing the biological sample into contact with the compound of General Formula (III) according to claim 6.

9. The method according to claim 8, wherein a library of the compound of General Formula (III) is used.

10. The method according to claim 8 or 9, wherein a microdevice is used.

11. A method for testing an enzyme assay of a composition containing the compound of General Formula (III) according to claim 6, the method comprising bringing the composition into contact with a biological sample containing or suspected of containing an enzyme which cleaves the compound.

12. A method for screening a fluorescent probe capable of detecting a biomarker of a specific disease, the method comprising the steps of:
(1) adding a library of the compound of Formula (III) according to claim 6 to a microdevice so that at least one well of the microdevice includes one kind of compound of Formula (III);
(2) adding a solution containing a biological sample to the microdevice so that at least one well containing one molecule of enzyme is generated in the microdevice, the biological sample being a biological sample obtained from a patient with a specific disease or a biological sample obtained from a healthy subject;
(3) bringing the compound of Formula (III) into contact with an enzyme to detect fluorescence in a well of the microdevice, the step including
bringing the compound of Formula (III) into contact with the biological sample obtained from a patient with a specific disease to measure a fluorescence intensity (first fluorescence intensity) from the compound of Formula (III), and
bringing the compound of Formula (III) into contact with the biological sample obtained from a healthy subject to measure a fluorescence intensity (second fluorescence intensity) from the compound of Formula (III); and
(4) determining that the compound is a biomarker activity detection fluorescent probe by showing that the compound is a candidate for the fluorescent probe when there is a difference between the first fluorescence intensity and the second fluorescence intensity.

13. A method for detecting activity of ENPP in a biological sample, the method comprising bringing a compound of Formula (IV) or a salt thereof into contact with a biological sample in an aqueous solution, an increase in fluorescence intensity in the aqueous solution indicating the presence of activity of ENPP: 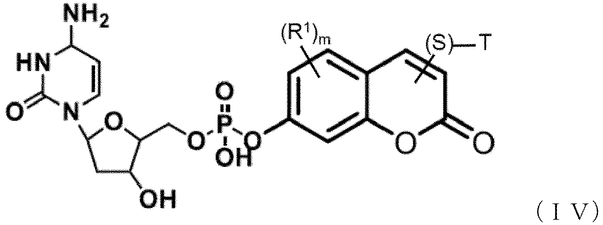 wherein R¹, S, T, and m are as described in detail in General Formula (I).

14. The method according to claim 13, wherein the biological sample is a biological sample of a patient with pancreatic cancer, a patient suspected of having pancreatic cancer, or a healthy subject.

15. A method for diagnosing pancreatic cancer or a method for predicting a possibility that a subject from which a biological sample is derived has pancreatic cancer, the method comprising the steps of: (a) applying a fluorescent probe containing a compound of General Formula (IV) or a salt thereof to a clinical specimen of a test subject; and (b) measuring a fluorescent image of the clinical specimen to which the fluorescent probe is applied.

16. A method for diagnosing pancreatic cancer or a method for predicting a possibility that a subject from which a biological sample is derived has pancreatic cancer, the method comprising the steps of: (a) bringing a biological sample obtained from a subject into contact with a fluorescent probe containing a compound of General Formula (IV) or a salt thereof; and (b) measuring a fluorescence intensity of the biological sample brought into contact with the fluorescent probe.

17. The diagnostic method or prediction method according to claim 15 or 16, wherein the compound of General Formula (VI) is a compound below:

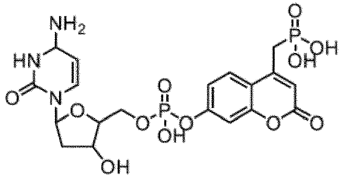

18. A fluorescent probe for detection of pancreatic cancer for use in the method according to claim 15 or 16, comprising a compound of General Formula (IV) or a salt thereof.

19. A kit for detection of pancreatic cancer cells or tissues, comprising a compound of General Formula (IV) or a salt thereof.

20. The fluorescent probe according to claim 18, wherein the compound of General Formula (VI) is a compound below:

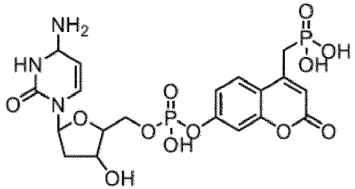

21. A method for diagnosing pancreatic cancer or a method for predicting a possibility that a subject from which a biological sample is derived has pancreatic cancer, the method comprising detecting, by a microdevice, single-molecule enzyme activity of CD13 in a biological sample obtained from a subject, using a fluorescent probe containing the compound or the salt thereof according to claim 6, in which B is - NR²-CO-L, and a moiety of -CO-L is an alanine, lysine, arginine, or methionine residue in General Formula (III).

22. A method for diagnosing pancreatic cancer or a method for predicting a possibility that a subject from which a biological sample is derived has pancreatic cancer, the method comprising detecting, by a microdevice, single-molecule enzyme activity of DPP4 in a biological sample obtained from a subject, using a fluorescent probe containing the compound or the salt thereof according to claim 6, in which B is - NR²-CO-L, and a moiety of -CO-L is -Pro-Xaa (Pro represents a proline residue and Xaa represents an amino acid residue such as glycine, serine, or glutamic acid) peptide in General Formula (III).

23. A method for diagnosing pancreatic cancer or a method for predicting a possibility that a subject from which a biological sample is derived has pancreatic cancer, the method comprising:
detecting, by a microdevice, single-molecule enzyme activity of CD13 in a biological sample obtained from a subject, using a fluorescent probe containing the compound or the salt thereof according to claim 6, in which B is -NR²-CO-L, and a moiety of -CO-L is an alanine, lysine, arginine, or methionine residue in General Formula (III); and
detecting, by a microdevice, single-molecule enzyme activity of DPP4 in a biological sample obtained from a subject, using a fluorescent probe containing the compound or the salt thereof according to claim 6, in which B is -NR²-CO-L, and a moiety of -CO-L is -Pro-Xaa (Pro represents a proline residue and Xaa represents an amino acid residue such as glycine, serine, or glutamic acid) peptide in General Formula (III).

24. The method according to any one of claims 21 to 23, wherein the biological sample is a biological sample of a patient with pancreatic cancer, a patient suspected of having pancreatic cancer, or a healthy subject.
